# EUROPEAN PATENT APPLICATION

(11) **EP 4 813 986 A1**
(43) Date of publication of application: **30.09.2026**
(21) Application number: 24893574.4
(22) Date of filing: 22.11.2024
(51) Int. Cl.: C07D 471/04, C07D 221/00, A61K 31/41, A61K 31/44, A61P 27/02

(54) **COMPOUND FOR REDUCING SERUM CONCENTRATION OF RBP4, AND USE**

(30) Priority: 24.11.2023 WO PCT/CN2023/133891; 29.11.2023 WO PCT/CN2023/134874; 22.12.2023 WO PCT/CN2023/141113; 29.12.2023 WO PCT/CN2023/143407; 03.01.2024 WO PCT/CN2024/070370; 08.03.2024 WO PCT/CN2024/080774; 13.06.2024 WO PCT/CN2024/098922; 15.10.2024 WO PCT/CN2024/124934
(71) Applicant: Heronova Pharmaceuticals, LLC, Beijing 100102 (CN)
(72) Inventor: DENG, Xiaobing, Beijing 100102 (CN); SU, Yaning, Beijing 100102 (CN); TIAN, Wenjia, Beijing 100102 (CN); XIAO, Donghuai, Beijing 100102 (CN); FU, Yuqing, Beijing 100102 (CN); YU, Xiaoyu, Beijing 100102 (CN); ZHAO, Yan, Beijing 100102 (CN); LI, Bo, Beijing 100102 (CN); CHEN, Huiling, Beijing 100102 (CN); WANG, Yanhong, Beijing 100102 (CN); CAO, Shuo, Beijing 100102 (CN); ZHOU, Yisui, Beijing 100102 (CN)
(74) Representative: Graf von Stosch Patentanwaltsgesellschaft mbH
(86) International application number: PCT/CN2024/133816
(87) International publication number: WO 2025/108420

(57) **Abstract**

This application relates to a compound for reducing the serum concentration of RBP4, stereoisomers thereof, deuterated derivatives thereof, pharmaceutically acceptable salts thereof, pharmaceutically acceptable salts of the stereoisomers thereof, or pharmaceutically acceptable salts of the deuterated derivatives thereof, as well as methods for preparing the compound, compositions comprising the same, and uses thereof.

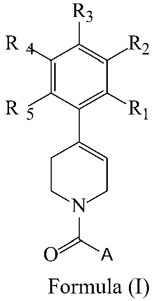

## Description

This application claims the priority of the prior applications filed on November 24, 2023 with International Application No. PCT/CN2023/133891 and title of invention "Compound for Reducing Serum Concentration of RBP4, and Use", filed on November 29, 2023 with International Application No. PCT/CN2023/134874 and title of invention "Compound for Reducing Serum Concentration of RBP4, and Use", filed on December 22, 2023 with International Application No. PCT/CN2023/141113 and title of invention "Compound for Reducing Serum Concentration of RBP4, and Use", filed on December 29, 2023 with International Application No. PCT/CN2023/143407 and title of invention "Compound for Reducing Serum Concentration of RBP4, and Use", filed on January 03, 2024 with International Bureau Application No. PCT/CN2024/070370 and title of invention "Compound for Reducing Serum Concentration of RBP4, and Use", filed on March 08, 2024 with International Application No. PCT/CN2024/080774 and title of invention "Compound for Reducing Serum Concentration of RBP4, and Use", filed on June 13, 2024 with International Application No. PCT/CN2024/098922 and title of invention "Compound for Reducing Serum Concentration of RBP4 and, Use", and filed on October 15, 2024 with International Application No. PCT/CN2024/124934 and title of invention "Compound for Reducing Serum Concentration of RBP4, and Use", the entire contents of which are incorporated herein by reference.

### Technical Field

This application belongs to the field of medicinal chemistry, and relates to a compound for reducing the serum concentration of RBP4, and the use of said compound.

### Background Art

Retinol-binding protein 4 (RBP4) is the only plasma transport protein that delivers vitamin A from the liver to peripheral tissues. Since retinol is involved in a variety of biological functions, including cell growth, immune function, fetal development and vision, research on RBP4 has attracted increasing interest in recent years (see Clin. Immunol. Immunopathol., 80 (1996), pp. S52-S62; Faseb. J., 10 (1996), pp. 961-968; Endocr. Rev., 10 (1989), pp. 308-316; Cell Metabol., 5 (2007), pp. 164-166; Harvey Lect., 90 (1994), pp. 105-117; Annu. Rev. Pharmacol. Toxicol., 46 (2006), pp. 451-480).

Several studies have elucidated the role of RBP4 in the pathogenesis of cardiovascular disease (CVD), obesity, non-alcoholic fatty liver disease (NAFLD), non-alcoholic steatohepatitis (NASH) and insulin resistance in type 2 diabetes mellitus (T2DM) (see N. Engl. J. Med., 354 (2006), pp. 2552-2563; Adv. Nutr., 6 (2015), pp. 748-762; Nature, 436 (2005), pp. 356-362; Circulation, 127 (2013), pp. 1938-1947; Lipids Health Dis., 16 (2017), p. 180).

RBP4 is also thought to be associated with cancer and the development of age-related macular degeneration (AMD) and Stargardt disease (STGD) (see Oncotarget, 8 (2017), pp. 92254-92264; Medicine, 99 (2020), article e21254; J. Ovarian Res., 11 (2018), p. 29; Cancers, 12 (2020), p. 623; Endocr. Relat. Canc., 22 (2015), pp. L1-L4; BioMed Res. Int., 2014 (2014), p. 179040; Health Technol. Assess., 22 (2018), pp. 1-168). In the treatment of the above diseases, studies have been conducted to reduce the expression of RBP4 by directly antagonizing or inhibiting the level of RBP4. Although RBP4 is mainly synthesized in the liver, with about 20% synthesized in adipose tissue (see J. Lipid Res., 30 (1989), pp. 171-180), it has been regarded as a biomarker of insulin resistance and metabolism. RBP4 is known as an adipokine involved in energy metabolism, insulin signal transduction and insulin resistance (see Proc. Natl. Acad. Sci. United States Am., 108 (2011), pp. 4340-4345; Am. J. Physiol. Endocrinol. Metab., 297 (2009), pp. E1420-E1429; Diabetes, 64 (2015), pp. 1603-1614; Diabetologia, 59 (2016), pp. 354-362). In addition to acting as a selective retinol transporter, a recent study showed that RBP4 is also involved in fatty acid transport (see Biochim. Biophys. Acta Mol. Cell Biol. Lipids, 1863 (2018), pp. 458-466). Furthermore, many studies have reported elevated RBP4 levels in patients with diabetes and insulin resistance (see J. Clin. Endocrinol., 92 (2007), pp. 1971-1974; Diabetes Care, 29 (2006), pp. 2457-2461; Diabetes Care, 30 (2007), pp. 1173-1178).

Studies are also exploring the role of RBP4 in atrophic (dry) AMD and STGD. AMD and STGD are slowly progressive neurodegenerative diseases that can lead to blindness. Excessive accumulation of lipofuscin in the retinal pigment epithelium (RPE) is considered the etiology of these diseases. Elevated lipofuscin levels inhibit the normal function of the RPE, thereby promoting its degeneration. Lack of RPE metabolic support subsequently leads to photoreceptor death (see Exp. Eye Res., 80 (2005), pp. 595-606; Faseb. J., 18 (2004), pp. 562-564). Therefore, to prolong RPE function and photoreceptor lifespan, the concentration of lipofuscin should be reduced. Lipofuscin toxicity is mainly mediated by A2E, one of its bisretinoid components.

Accordingly, reduction of RBP4 levels will affect the expression of specific genes and subsequent biological functions. A recent paper discussed the biological function of RBP4 and its pathological relevance in human diseases (see Front. Physiol., 12 (2021), p. 659977). There is also a need to develop compounds with excellent RBP4-lowering effects.

### Summary of the Invention

In one aspect, the present application provides a compound for reducing the serum concentration of RBP4. Details are as follows:
A first aspect of the present application provides a compound of formula (I), a stereoisomer thereof, a deuterated derivative thereof, a pharmaceutically acceptable salt thereof, a pharmaceutically acceptable salt of a stereoisomer thereof, or an acceptable salt of a deuterated derivative thereof, 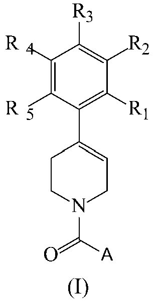 wherein,
R₁, R₂, R₃, R₄ and R₅ are each independently -H, -halogen, -CF₃ or -C₁₄ alkyl; wherein at least two of R₁, R₂, R₃, R₄ or R₅ are not H;
A is 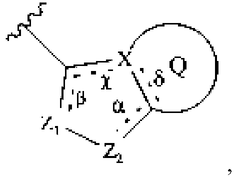
α, β, χ and δ are each independently absent or present, and each is a bond when present;
X is C or N;
Z₁ is N;
Z₂ is O, S, N or NR₇,
R₇ is -H, -C₁₋₄ alkyl or -(oxetanyl) group;
Q is a substituted or unsubstituted heterobicyclic ring; preferably, Q is a substituted or unsubstituted 5, 6 or 7-membered ring structure.

In some embodiments, R₁, R₂, R₃, R₄ and R₅ are each independently -H, -F, -Cl, -Br or -CF₃.

In some embodiments of the present application, in the compound of the first aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₁ is -CF₃, R₂ is -F, R₃ is -F, R₄ is -H and R₅ is -H, or
R₁ is -CF₃, R₂ is -F, R₃ is -H, R₄ is -H and R₅ is -H, or
R₁ is -CF₃, R₂ is -F, R₃ is -H, R₄ is -F and R₅ is -H, or
R₁ is -CF₃, R₂ is -H, R₃ is -F, R₄ is -F and R₅ is -H, or
R₁ is -CF₃, R₂ is -H, R₃ is -H, R₄ is -H and R₅ is -F, or
R₁ is -CF₃, R₂ is -H, R₃ is -F, R₄ is -H and R₅ is -H, or
R₁ is -CF₃, R₂ is -H, R₃ is -H, R₄ is -Cl and R₅ is -H, or
R₁ is -CF₃, R₂ is -Cl, R₃ is -H, R₄ is -H and R₅ is -H, or
R₁ is -H, R₂ is -CF₃, R₃ is -H, R₄ is -CF₃ and R₅ is -H, or
R₁ is -Cl, R₂ is -H, R₃ is -H, R₄ is -F and R₅ is -H, or
R₁ is -Cl, R₂ is -F, R₃ is -H, R₄ is -H and R₅ is -H.

In some embodiments of the present application, in the compound of the first aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₁ is -CF₃, R₂ is -F, R₃ is -F, R₄ is -H and R₅ is -H, or
R₁ is -CF₃, R₂ is -F, R₃ is -H, R₄ is -H and R₅ is -H.

In some embodiments of the present application, in the compound of the first aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,

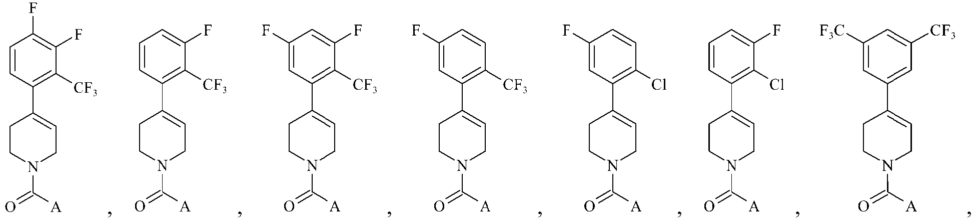

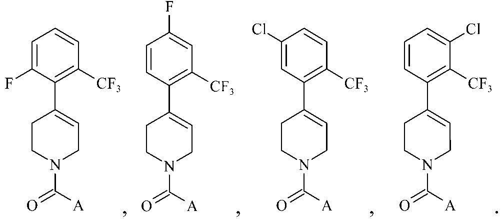

In some embodiments of the present application, in the compound of the first aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is 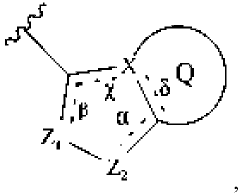
wherein when α is present, then Z₁ and Z₂ are N, X is N, β is present, and χ and δ are absent;
and when α is absent, then Z₁ is N, Z₂ is NR₇, X is C, β and δ are present, and χ is absent.

In some embodiments of the present application, in the compound of the first aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is 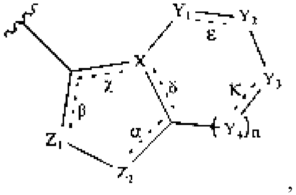
n is an integer of 0-2;
R₇ is -H, -C₁₋₄ alkyl or -(oxetanyl) group;
α, β, χ, δ, ε, and κ are each independently absent or present, and each is a bond when present;
Z₁ is N;
Z₂ is O, S, N or NR₇,
X is C or N;
and Y₁, Y₂, Y₃ and each occurrence of Y₄ are each independently CR₈, CH₂ or NR₉;
wherein,
R₈ is -H, halogen, -OCH₃, -CN or -CF₃;
and R₉ is -H, -CN, -C₁₋₆ alkyl, -C₁₋₆ haloalkyl, -C₃₋₆ cycloalkyl, -(C₁₋₄ alkylene)-(C₃₋₆ cycloalkyl), -(C₃₋₆ heterocyclyl), -(C₁₋₄ alkylene)-(C₃₋₆ heterocyclyl), -(C₁₋₆ alkylene)-O(C₁₋₃ alkyl), -(C₁₋₆ alkylene)-CF₃, -C(O)-(C₁₋₆ alkyl), -C(O)₂-(C₁₋₆ alkyl), -C(O)-NH₂, -C(O)-NH(C₁₋₃ alkyl), -C(O)-N(C₁₋₃ alkyl)₂, -C(O)NH-(C₁₋₆ alkyl), -C(O)-(C₁₋₆ haloalkyl), -C(O)-(C₁₋₆ alkoxy), -C(O)-(C₁₋₆ alkylene)-(OC₁₋₆ alkyl), -C(O)-(C₆ aryl), -C(O)-(C₆ heteroaryl), -C(O)-(C₆ heterocyclyl), -(C₁₋₆ alkylene)-CO₂H, -(C₁₋₆ alkylene)-CO2(C₁₋₆ alkyl) or -SO₂-(C₁₋₆ alkyl).

A second aspect of the present application provides a compound of formula (I), a stereoisomer thereof, a deuterated derivative thereof, a pharmaceutically acceptable salt thereof, a pharmaceutically acceptable salt of a stereoisomer thereof, or an acceptable salt of a deuterated derivative thereof, 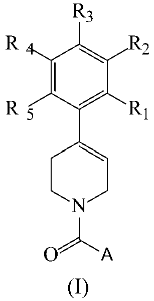 wherein,
R₁, R₂, R₃, R₄ and R₅ are each independently -H, -halogen, -CF₃ or -C₁₄ alkyl; wherein at least two of R₁, R₂, R₃, R₄ or R₅ are not H;
A is selected from 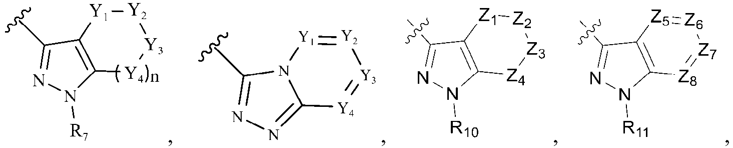 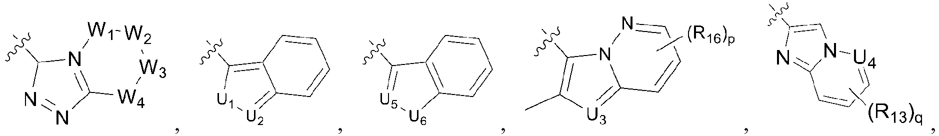 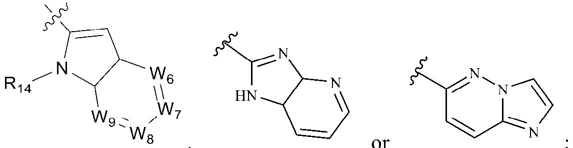
wherein R₇ is selected from -H, -C₁₋₄ alkyl or -(oxetanyl) group;
when A is 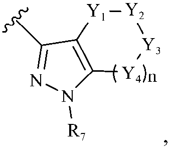
wherein n is 0, 1 or 2;
Y₁, Y₂ and Y₄ are each -CH₂-, and Y₃ is -NR₉-; or Y₁, Y₃ and Y₄ are each -CH₂-, and Y₂ is -NR₉-; or Y₁, Y₂ and Y₄ are each -CH₂-, and Y₃ is -O- or -S(=O)₂-; or Y₁, Y₃ and Y₄ are each -CH₂-, and Y₂ is -O- or -S(=O)₂-;
R₉ is selected from hydrogen, -C₁₋₆ alkyl, -C(=O)(C₁₋₆ alkyl), -S(=O)(C₁₋₆ alkyl), -S(=O)₂(C₁₋₆ alkyl), 3-7 membered carbocyclyl, 3-7 membered heterocyclyl, phenyl, 5-10 membered heteroaryl; said R₉ is optionally substituted with 1, 2, 3, 4, 5 or 6 substituents selected from halogen, -C₁₋₆ alkyl, -C₁₋₆ alkenyl, -C₁₋₆ alkynyl, -C₁₋₆ haloalkyl, -C₁₋₆ alkoxy, -CN, -NH₂, -COOH, -NH(C₁₋₆ alkyl), -N(C₁₋₆ alkyl)₂, -OH, -O(C₁₋₆ alkyl), -O(C₁₋₆ deuterated alkyl), -SH, -S(C₁₋₆ alkyl), =O, -C(=O)(C₁₋₆ alkyl), -C(=O)(C₁₋₆ alkoxy), -S(=O)(C₁₋₆ alkyl), -S(=O)₂(C₁₋₆ alkyl), -C(=O)NH₂, -C(=O)-C(=O)NH₂, -C(=O)NH(C₁₋₆ alkyl), -C(=O)N(C₁₋₆ alkyl)₂, -NHC(=O)(C₁₋₆ alkyl), -C(=O)O(C₁₋₆ alkyl), -OC(=O)(C₁₋₆ alkyl), -S(=O)NH₂, -S(=O)NH(C₁₋₆ alkyl), -S(=O)N(C₁₋₆ alkyl)₂, -NHS(=O)(C₁₋₆ alkyl), -S(=O)₂NH₂, -S(=O)₂NH(C₁₋₆ alkyl), -S(=O)₂N(C₁₋₆ alkyl)₂, -NHS(=O)₂(C₁₋₆ alkyl), -C(=O)-phenyl, -C(=O)-5-10 membered heteroaryl, -C(=O)-3-7 membered heterocyclyl, 3-7 membered carbocyclyl, 3-7 membered heterocyclyl, phenyl or 5-10 membered heteroaryl; said heterocyclyl or heteroaryl each independently includes one or more heteroatoms selected from N, O or S at each occurrence;
when A is 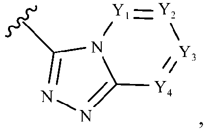
wherein Y₁, Y₂, Y₃ and Y₄ are each independently CR₈ or N, and each R₈ is independently -H, halogen, -C₁₋₆ alkyl, -C(=O)NH₂, -C(=O)NH(C₁₋₆ alkyl), -C(=O)N(C₁₋₆ alkyl)₂, -COOH, -NHC(=O)N(C₁₋₆ alkyl)₂, -S(=O)₂NH(C₁₋₆ alkyl), -O-(C₁₋₆ alkyl), -CN or -C₁₋₆ haloalkyl;
when A is 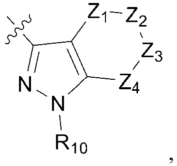
wherein Z₂ is selected from -O-, Z₃ is selected from -C(CH₃)₂-, and Z₁ and Z₄ are selected from -CH₂-; alternatively, Z₂ and Z₃ are each independently selected from -C(O)-, -N(CH₃)- or -NH-, and Z₁ and Z₄ are selected from -CH₂-;
R₁₀ is selected from hydrogen or -CH₃;
when A is 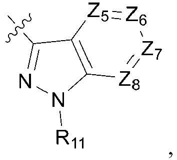
wherein Z₅, Z₆, Z₇ and Z₈ are each independently selected from CR₁₂ or N, and R₁₂ is each independently selected from hydrogen, F, Cl, -CN or -OCH₃;
R₁₁ is selected from H, -CH₃, -CH₂CH₃, 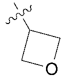 or -CH(CH₃)_{2;}
when A is 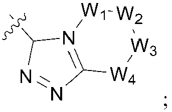
wherein W₁, W₂, W₃ and W ₄ are each independently selected from -CH₂-, -O-, -NH-, 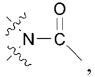 -N (CH₃) - or 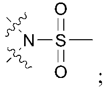
when A is 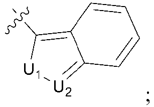
wherein U₁ is selected from O or S, and U₂ is selected from N;
when A is 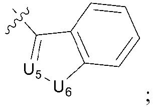
wherein U₅ is selected from N, and U₆ is selected from O or S; alternatively, U₅ is selected from CH, and U₆ is selected from NH;
when A is 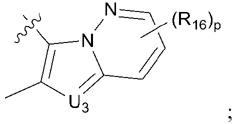
wherein U₃ is selected from N or CH;
R₁6 is selected from Cl;
p is selected from 0 or 1;
when A is 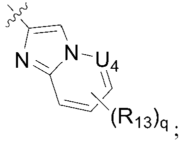
wherein U₄ is selected from N or CH;
R₁₃ is selected from-CH₃, -OCH₃, 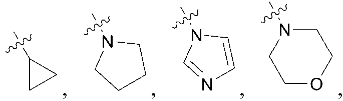 F, Cl or -CF₃;
q is selected from 0 or 1;
when A is 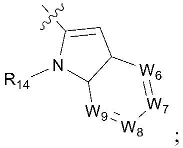
wherein W₆, W₇, W₈ and W₉ are each independently selected from Nor CR₁₅;
R₁₄ is selected from hydrogen or -CH₃;
R₁₅ is selected from H, F, Cl, -CH₃, -OCH₃,

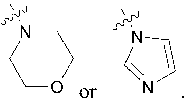

A third aspect of the present application provides a compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof, 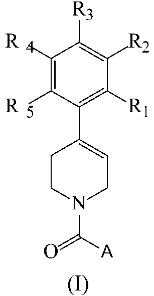
wherein R₁, R₂, R₃, R₄ and R₅ are each independently -H, -halogen, -CF₃ or -C₁₄ alkyl; wherein at least two of R₁, R₂, R₃, R₄ or R₅ are not H;
when A is 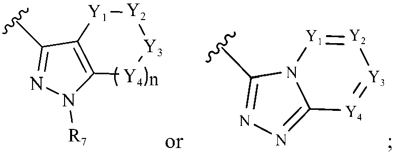
wherein R₇ is -H, -C₁₋₄ alkyl or -(oxetanyl) group;
when A is 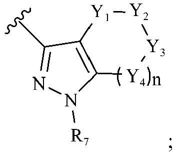
n is 0, 1 or 2, preferably 0 or 1; Y₁, Y₂ and Y₄ are each -CH₂-, and Y₃ is -NR₉-; or
Y₁, Y₃ and Y₄ are each -CH₂-, and Y₂ is -NR₉-;
R₉ is selected from hydrogen, -C₁₋₆ alkyl, -C(=O)(C₁₋₆ alkyl), -S(=O)(C₁₋₆ alkyl), -S(=O)₂(C₁₋₆ alkyl), 3-7 membered carbocyclyl, 3-7 membered heterocyclyl, phenyl, 5-10 membered heteroaryl; said R₉ is optionally substituted with 1, 2, 3, 4, 5 or 6 substituents selected from -C₁₋₆ alkyl, -C₁₋₆ alkenyl, -C₁₋₆ alkynyl, -C₁₋₆ haloalkyl, -C₁₋₆ alkoxy, -CN, -NH₂, -COOH, -NH(C₁₋₆ alkyl), -N(C₁₋₆ alkyl)₂, -OH, -O(C₁₋₆ alkyl), -SH, -S(C₁₋₆ alkyl), -C(=O)(C₁₋₆ alkyl), -S(=O)(C₁₋₆ alkyl), -S(=O)₂(C₁₋₆ alkyl), -C(=O)NH₂, -C(=O)NH(C₁₋₆ alkyl), -C(=O)N(C₁₋₆ alkyl)₂, -NHC(=O)(C₁₋₆ alkyl), -C(=O)O(C₁₋₆ alkyl), -OC(=O)(C₁₋₆ alkyl), -S(=O)NH₂, -S(=O)NH(C₁₋₆ alkyl), -S(=O)N(C₁₋₆ alkyl)₂, -NHS(=O)(C₁₋₆ alkyl), -S(=O)₂NH₂, -S(=O)₂NH(C₁₋₆ alkyl), -S(=O)₂N(C₁₋₆ alkyl)₂, -NHS(=O)₂(C₁₋₆ alkyl), 3-7 membered carbocyclyl, 3-7 membered heterocyclyl, phenyl or 5-10 membered heteroaryl; said heterocyclyl or heteroaryl each independently includes one or more heteroatoms selected from N, O or S at each occurrence;
when A is 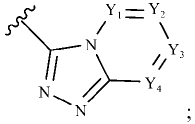
wherein, Y₁, Y₂, Y₃ and Y₄ are each independently CR₈ or N,
and each R₈ is independently -H, halogen, -O-(C₁₋₃ alkyl), -CN or -C₁₋₃ haloalkyl.

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₁, R₂, R₃, R₄ and R₅ are each independently -H, -F, -Cl, -Br or -CF₃; other groups are as defined in the second or third aspect of the present application.

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₁ is -CF₃, R₂ is -F, R₃ is -F, R₄ is -H and R₅ is -H, or
R₁ is -CF₃, R₂ is -F, R₃ is -H, R₄ is -H and R₅ is -H, or
R₁ is -CF₃, R₂ is -F, R₃ is -H, R₄ is -F and R₅ is -H, or
R₁ is -CF₃, R₂ is -H, R₃ is -F, R₄ is -F and R₅ is -H, or
R₁ is -CF₃, R₂ is -H, R₃ is -H, R₄ is -H and R₅ is -F, or
R₁ is -CF₃, R₂ is -H, R₃ is -F, R₄ is -H and R₅ is -H, or
R₁ is -CF₃, R₂ is -H, R₃ is -H, R₄ is -Cl and R₅ is -H, or
R₁ is -CF₃, R₂ is -Cl, R₃ is -H, R₄ is -H and R₅ is -H, or
R₁ is -H, R₂ is -CF₃, R₃ is -H, R₄ is -CF₃ and R₅ is -H, or
R₁ is -Cl, R₂ is -H, R₃ is -H, R₄ is -F and R₅ is -H, or
R₁ is -Cl, R₂ is -F, R₃ is -H, R₄ is -H and R₅ is -H, or
R₁ is -CF₃, R₂ is -H, R₃ is -H, R₄ is -F and R₅ is -H; other groups are as defined in the second or third aspect of the present application.

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₁ is -CF₃, R₂ is -F, R₃ is -F, R₄ is -H and R₅ is -H,
or R₁ is -CF₃, R₂ is -F, R₃ is -H, R₄ is -H and R₅ is -H; other groups are as defined in the second or third aspect of the present application.

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof, said compound is any one of the following formulae: 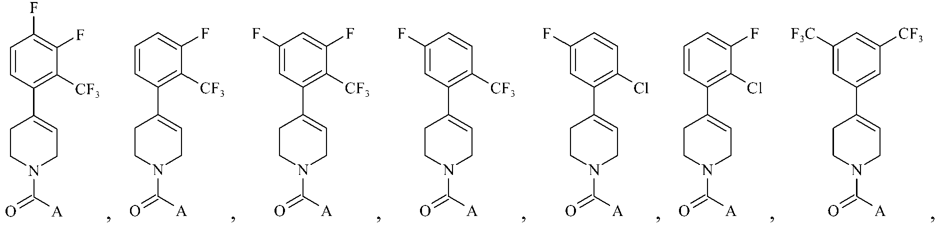 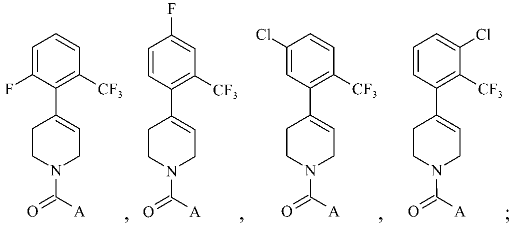 other groups are as defined in the second or third aspect of the present application.

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is 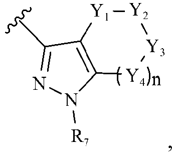 n is 0 or 1, and
when n is 0, Y₁ and Y₃ are each -CH₂-, and Y₂ is -NR₉-;
when n is 1, Y₁, Y₂ and Y₄ are each -CH₂-, and Y₃ is -NR₉-; or Y₁, Y₃ and Y₄ are each -CH₂-, and Y₂ is -NR₉-; R₉ is selected from hydrogen, -C₁₋₆ alkyl, -C(=O)(C₁₋₆ alkyl), -S(=O)(C₁₋₆ alkyl), -S(=O)₂(C₁₋₆ alkyl), 3-7 membered carbocyclyl, 3-7 membered heterocyclyl, phenyl, 5-10 membered heteroaryl; said R₉ is optionally substituted with 1, 2, 3, 4, 5 or 6 substituents selected from -C₁₋₃ alkyl, -C₁₋₃ alkenyl, -C₁₋₃ alkynyl, -C₁₋₃ haloalkyl, -C₁₋₃ alkoxy, -CN, -NH₂, -COOH, -NH(C₁₋₃ alkyl), -N(C₁₋₃ alkyl)₂, -OH, -O(C₁₋₃ alkyl), -SH, -S(C₁₋₃ alkyl), -C(=O)(C₁₋₃ alkyl), -S(=O)(C₁₋₃ alkyl), -S(=O)₂(C₁₋₃ alkyl), -C(=O)NH₂, -C(=O)NH(C₁₋₃ alkyl), -C(=O)N(C₁₋₃ alkyl)₂, -NHC(=O)(C₁₋₃ alkyl), -C(=O)O(C₁₋₃ alkyl), -C(=O)-(C₁₋₃ alkylene)-(OC₁₋₃ alkyl), -OC(=O)(C₁₋₃ alkyl), -S(=O)NH₂, -S(=O)NH(C₁₋₃ alkyl), -S(=O)N(C₁₋₃ alkyl)₂, -NHS(=O)(C₁₋₃ alkyl), -S(=O)₂NH₂, -S(=O)₂NH(C₁₋₃ alkyl), -S(=O)₂N(C₁₋₃ alkyl)₂, -NHS(=O)₂(C₁₋₃ alkyl), 3-7 membered carbocyclyl, 3-7 membered heterocyclyl, phenyl or 5-10 membered heteroaryl; said heterocyclyl or heteroaryl each independently includes one or more heteroatoms selected from N, O or S at each occurrence; said R₉ is preferably -H, -CN, -C₁₋₆ alkyl, -C₁₋₆ haloalkyl, -C₃₋₆ cycloalkyl, -(C₁₋₄ alkylene)-(C₃₋₆ cycloalkyl), -C₃₋₆ heterocyclyl, -(C₁₋₄ alkylene)-(C₃₋₆ heterocyclyl), -(C₁₋₆ alkylene)-O(C₁₋₃ alkyl), -(C₁₋₆ alkylene)-CF₃, -C(O)-(C₁₋₆ alkyl), -C(O)₂-(C₁₋₆ alkyl), -C(O)-NH₂, -CH₂C(O)-NH₂, -C(O)-C(O)-NH₂, -C(O)-CH(C₁₋₆ alkylene)-C(O)-NH₂, -CH₂C(O)-NH(C₁₋₃ alkyl), -CH₂C(O)-N(C₁₋₃ alkyl)₂, -C(O)-NH(C₁₋₃ alkyl), -C(O)-N(C₁₋₃ alkyl)₂, -C(O)NH-(C₁₋₆ alkyl), -C(O)-(C₁₋₆ haloalkyl), -C(O)-(C₁₋₆ alkoxy), -C(O)-(C₁₋₆ alkylene)-(OC₁₋₆ alkyl), -C(O)-(C₆ aryl), -C(O)-(C₆ heteroaryl), -C(O)-(C₆ heterocyclyl), -(C₁₋₆ alkylene)-CO₂H, -(C₁₋₆ alkylene)-CO₂(C₁₋₆ alkyl), -SO₂-(C₁₋₆ alkyl), oxetanyl or 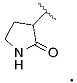
R₇ is -H, -C₁₋₄ alkyl or -(oxetanyl) group.

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is 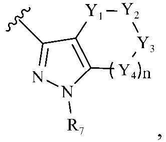
wherein, n is 0;
R₇ is -H, -C₁₋₄ alkyl or -(oxetanyl) group;
Y₁ and Y₃ are each -CH₂-, and Y₂ is -NR₉-;
said R₉ is -H, -CN, -C₁₋₆ alkyl, -C₁₋₆ haloalkyl, -C₃₋₆ cycloalkyl, -(C₁₋₄ alkylene)-(C₃₋₆ cycloalkyl), -(C₁₋₄ alkylene)-(C₃₋₆ heterocyclyl), -(C₁₋₆ alkylene)-O(C₁₋₃ alkyl), -(C₁₋₆ alkylene)-CF₃, -C(O)-(C₁₋₆ alkyl), -C(O)₂-(C₁₋₆ alkyl), -C(O)-NH₂, -C(O)-NH(C₁₋₃ alkyl), -C(O)-N(C₁₋₃ alkyl)₂, -C(O)NH-(C₁₋₆ alkyl), -C(O)-(C₁₋₆ haloalkyl), -C(O)-(C₁₋₆ alkoxy), -C(=O)-(C₁₋₆ alkylene)-(OC₁₋₃ alkyl), -C(O)-(C₆ aryl), -C(O)-(C₆ heteroaryl), -C(O)-(C₆ heterocyclyl), -(C₁₋₆ alkylene)-CO₂H, -(C₁₋₆ alkylene)-CO₂(C₁₋₆ alkyl) or -SO₂-(C₁₋₆ alkyl).

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is 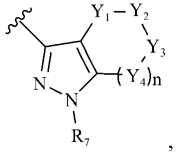
wherein, n is 1;
R₇ is -H, -C₁₋₄ alkyl or -(oxetanyl) group;
Y₁, Y₂ and Y₄ are each -CH₂-, and Y₃ is -NR₉-;
said R₉ is -H, -CN, -C₁₋₆ alkyl, -C₁₋₆ haloalkyl, -C₃₋₆ cycloalkyl, -(C₁₋₄ alkylene)-(C₃₋₆ cycloalkyl), -(C₃₋₆ heterocyclyl), -(C₁₋₄ alkylene)-(C₃₋₆ heterocyclyl), -(C₁₋₆ alkylene)-O(C₁₋₃ alkyl), -(C₁₋₆ alkylene)-CF₃, -C(O)-(C₁₋₆ alkyl), -C(O)₂-(C₁₋₆ alkyl), -C(O)-NH₂, -C(O)-NH(C₁₋₃ alkyl), -C(O)-N(C₁₋₃ alkyl)₂, -C(O)NH-(C₁₋₆ alkyl), -C(O)-(C₁₋₆ haloalkyl), -C(O)-(C₁₋₆ alkoxy), -C(=O)-(C₁₋₆ alkylene)-(OC₁₋₃ alkyl), -C(O)-(C₆ aryl), -C(O)-(C₆ heteroaryl), -C(O)-(C₆ heterocyclyl), -(C₁₋₆ alkylene)-CO₂H, -(C₁₋₆ alkylene)-CO₂(C₁₋₆ alkyl) or -SO₂-(C₁₋₆ alkyl).

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is 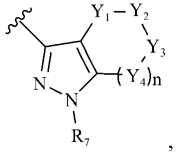
wherein, n is 1;
R₇ is -H, -C₁₋₄ alkyl or -(oxetanyl) group;
Y₁, Y₃ and Y₄ are each -CH₂-, and Y₂ is -NR₉-;
said R₉ is -H, -CN, -C₁₋₆ alkyl, -C₁₋₆ haloalkyl, -C₃₋₆ cycloalkyl, -(C₁₋₄ alkylene)-(C₃₋₆ cycloalkyl), -(C₃₋₆ heterocyclyl), -(C₁₋₄ alkylene)-(C₃₋₆ heterocyclyl), -(C₁₋₆ alkylene)-O(C₁₋₃ alkyl), -(C₁₋₆ alkylene)-CF₃, -C(O)-(C₁₋₆ alkyl), -C(O)₂-(C₁₋₆ alkyl), -C(O)-NH₂, -CH₂C(O)-NH₂, -C(O)-C(O)-NH₂, -C(O)-CH(C₁₋₆ alkylene)-C(O)-NH₂, -CH₂C(O)-NH(C₁₋₃ alkyl), -CH₂C(O)-N(C₁₋₃ alkyl)₂, -C(O)-NH(C₁₋₃ alkyl), -C(O)-N(C₁₋₃ alkyl)₂, -C(O)NH-(C₁₋₆ alkyl), -C(O)-(C₁₋₆ haloalkyl), -C(O)-(C₁₋₆ alkoxy), -C(=O)-(C₁₋₆ alkylene)-(OC₁₋₃ alkyl), -C(O)-(C₆ aryl), -C(O)-(C₆ heteroaryl), -C(O)-(C₆ heterocyclyl), -(C₁₋₆ alkylene)-CO₂H, -(C₁₋₆ alkylene)-CO₂(C₁₋₆ alkyl), -SO₂-(C₁₋₆ alkyl) or

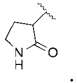

A fourth aspect of the present application provides a compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
wherein, R₁, R₂, R₃, R₄ and R₅ are each independently -H, -halogen, -CF₃ or -C₁₋₄ alkyl; wherein at least two of R₁, R₂, R₃, R₄ or R₅ are not H;
A is selected from 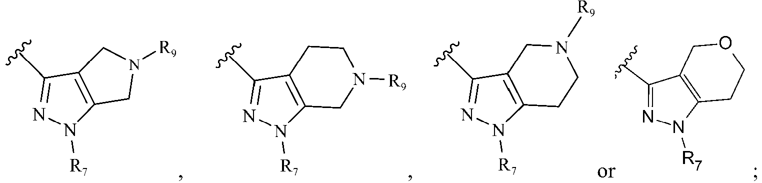
R₇ and R₉ are as defined in the second or third aspect of the present application.

In some embodiments, in the compound of the fourth aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₁, R₂, R₃, R₄ and R₅ are each independently -H, -F, -Cl, -Br or -CF₃; other groups are as defined in the second or third aspect of the present application.

In some embodiments, in the compound of the fourth aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₁ is -CF₃, R₂ is -F, R₃ is -F, R₄ is -H and R₅ is -H, or
R₁ is -CF₃, R₂ is -F, R₃ is -H, R₄ is -H and R₅ is -H, or
R₁ is -CF₃, R₂ is -F, R₃ is -H, R₄ is -F and R₅ is -H, or
R₁ is -CF₃, R₂ is -H, R₃ is -F, R₄ is -F and R₅ is -H, or
R₁ is -CF₃, R₂ is -H, R₃ is -H, R₄ is -H and R₅ is -F, or
R₁ is -CF₃, R₂ is -H, R₃ is -F, R₄ is -H and R₅ is -H, or
R₁ is -CF₃, R₂ is -H, R₃ is -H, R₄ is -Cl and R₅ is -H, or
R₁ is -CF₃, R₂ is -Cl, R₃ is -H, R₄ is -H and R₅ is -H, or
R₁ is -H, R₂ is -CF₃, R₃ is -H, R₄ is -CF₃ and R₅ is -H, or
R₁ is -Cl, R₂ is -H, R₃ is -H, R₄ is -F and R₅ is -H, or
R₁ is -Cl, R₂ is -F, R₃ is -H, R₄ is -H and R₅ is -H; other groups are as defined in the second or third aspect of the present application.

In some embodiments, in the compound of the second, third or fourth aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₉ is -H, -CN, -CH₃, -CH₂CH₃, -CH₂CH₂CH₃, -CH(CH₃)₂, -CH₂CH(CH₃)₂, -C(CH₃)₃, -CH₂CH(CH₃)₂, -CH₂C(CH₃)₃, -CH₂CF₃, -CH₂CH₂CF₃, -CH₂OCH₃, -CH₂CH₂OCH₃, 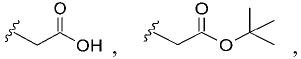 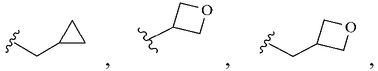 -SO₂-CH₃, -C(O)-CH₃, -C(O)-CH₂CH₃, -C(O)-CH₂CH₂CH₃, -C(O)-CH(CH₃)₂, -C(O)-CH₂CH(CH₃)₂, C(O)-C(CH₃)₃, -C(O)-OCH₃, -C(O)-NHCH₃, 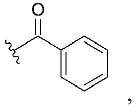 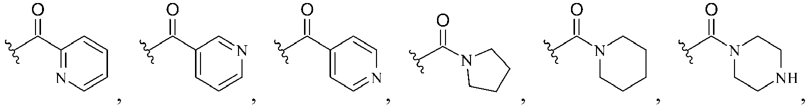 -C(O)-CH₂OCH₃, -C(O)-CH₂OCH₂CH₃, -C(O)-CH₂OCH(CH₃)₂, -C(O)-CH₂CH₂OCH₃, -C(O)-CH₂CH₂OCH₂CH₃, -C(O)-CH₂CH₂OCH(CH₃)₂, -C(O)-CH₂CF₃, -C(O)-CH₂Cl, -C(O)-CH₂F, -C(O)-CH₂CH₂OCH₃ , -C(O)-CH₂CH₂CF₃, -C(O)-CH₂CH₂Cl, -C(O)-CH₂CH₂F,

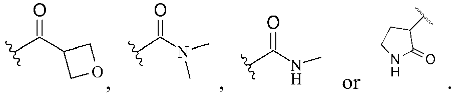

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof, R₉ is -H, -CN, -CH₃, -CH₂CH₃, -CH₂CH₂CH₃, -CH(CH₃)₂, -CH₂CH(CH₃)₂, -C(CH₃)₃, -CH₂CH(CH₃)₂, -CH₂C(CH₃)₃, -CH₂CF₃, -CH₂CH₂CF₃, -CH₂OCH₃, -CH₂CH₂OCH₃,

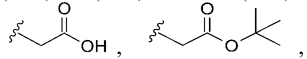

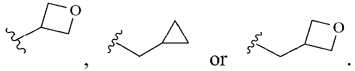

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof, R₉ is -SO₂-CH₃, -C(O)-CH₃, -C(O)-CH₂CH₃, -C(O)-CH₂CH₂CH₃, -C(O)-CH(CH₃)₂, -C(O)-CH₂CH(CH₃)₂, C(O)-C(CH₃)₃, -C(O)-OCH₃, -C(O)-NHCH₃,

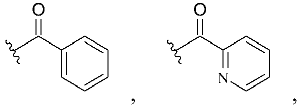

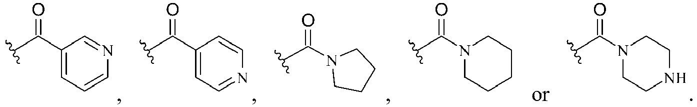

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof, R₉ is -C(O)-CH₃, -C(O)-CH₂CH₃, -C(O)-CH₂CH₂CH₃, -C(O)-CH(CH₃)₂, -C(O)-CH₂CH(CH₃)₂, -C(O)-C(CH₃)₃, -C(O)-CH₂OCH₃, -C(O)-CH₂OCH₂CH₃, -C(O)-CH₂OCH(CH₃)₂, -C(O)-CH₂CF₃, -C(O)-CH₂Cl, -C(O)-CH₂F, -C(O)-CH₂CH₂OCH₃, -C(O)-CH₂CH₂OCH₂CH₃, -C(O)-CH₂CH₂OCH(CH₃)₂, -C(O)-CH₂CH₂CF₃, -C(O)-CH₂CH₂Cl, -C(O)-CH₂CH₂F,

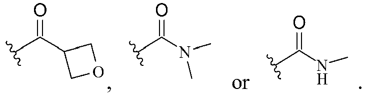

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
R₇ is -H, -CH₃, -CH₂CH₃, -CH(CH₃)₂ or

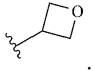

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is 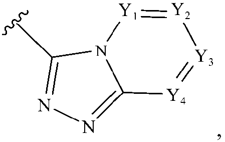
wherein, Y₁, Y₂, Y₃ and Y₄ are each independently CR₈ or N,
R₈ is each independently -H, halogen, -OCH₃, -CN or -CF₃ at each occurrence.

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is

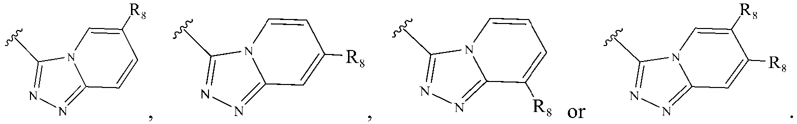

In some embodiments, in the compound of the second or third aspect of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof,
A is

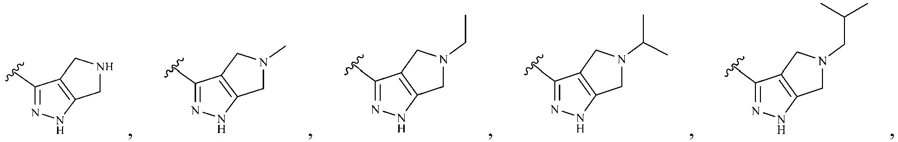

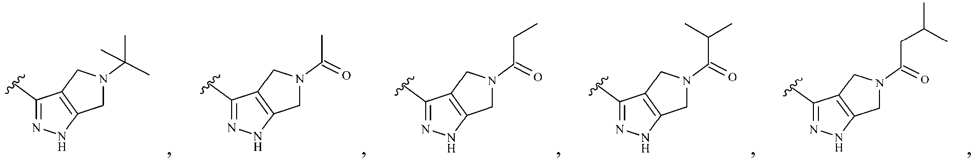

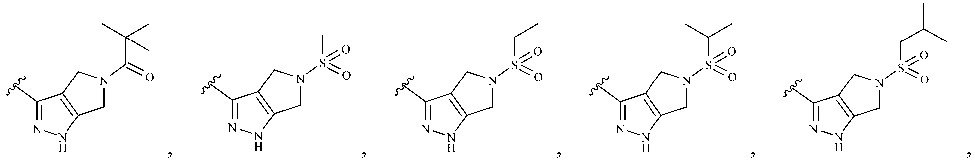

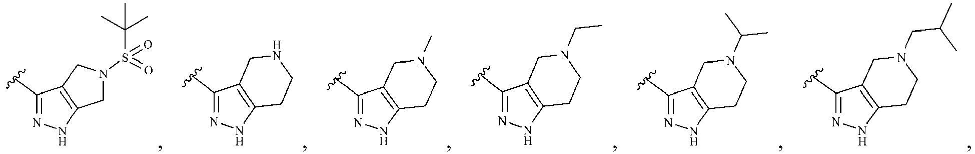

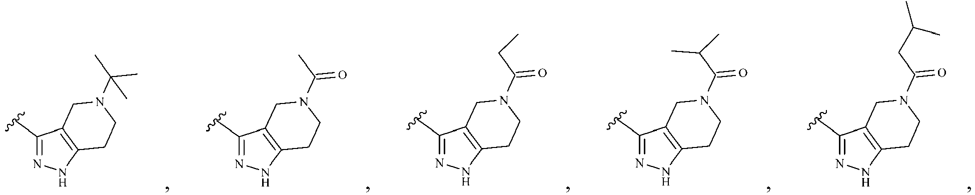

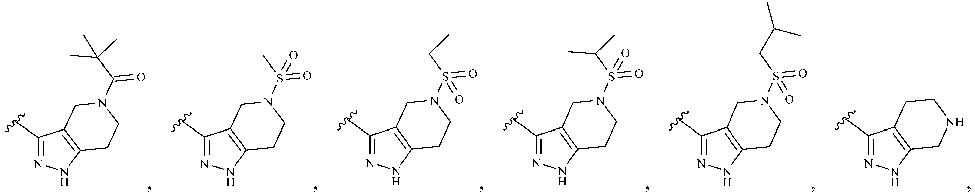

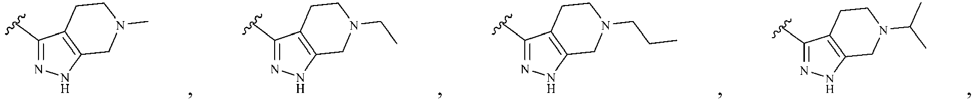

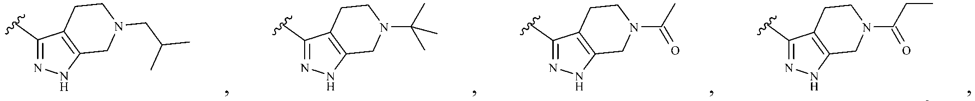

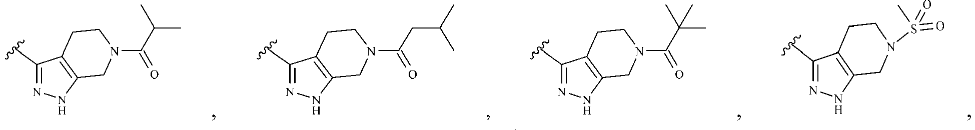

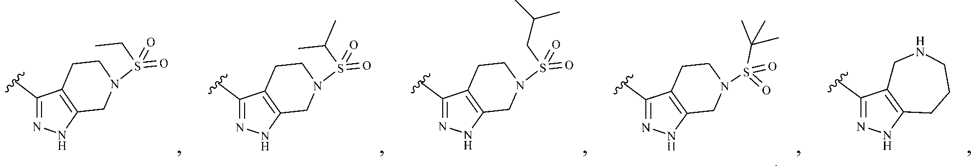

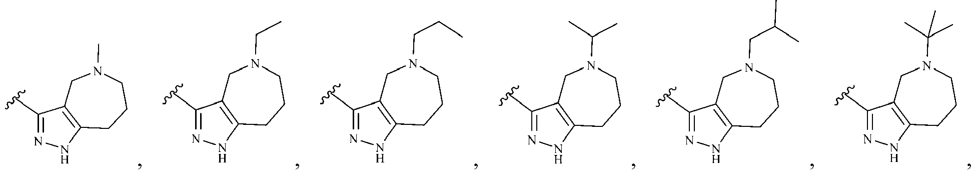

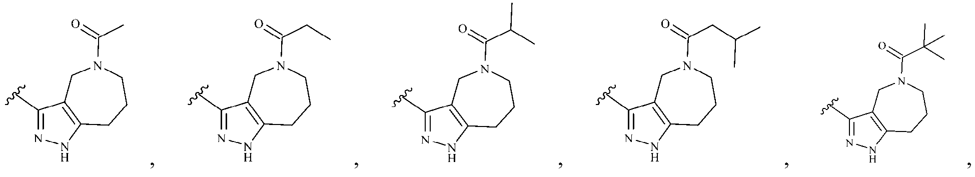

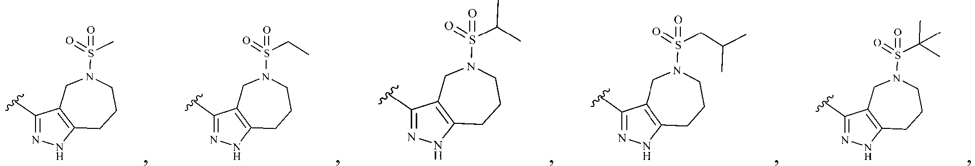

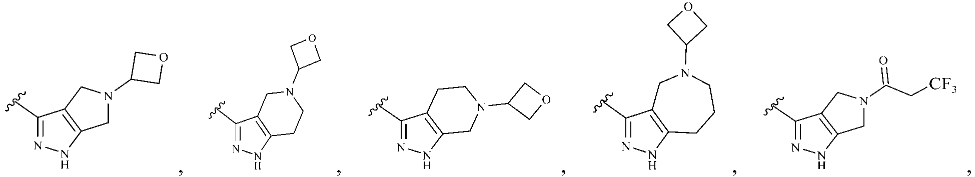

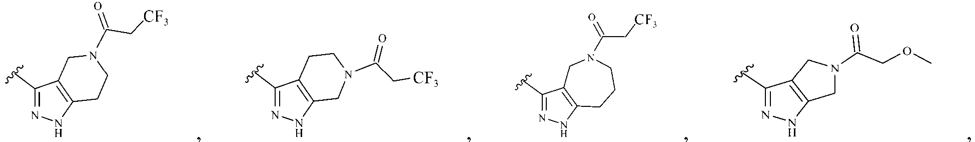

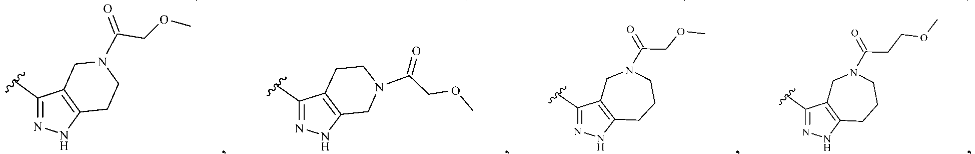

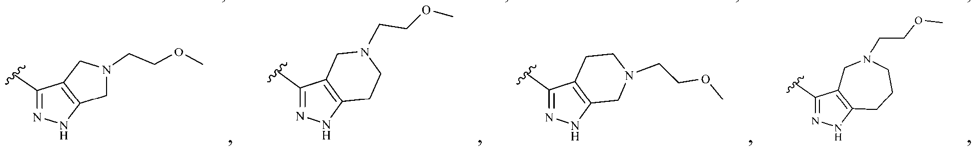

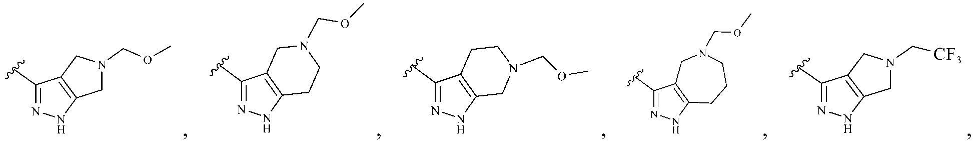

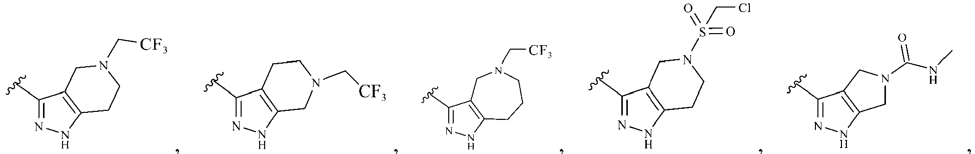

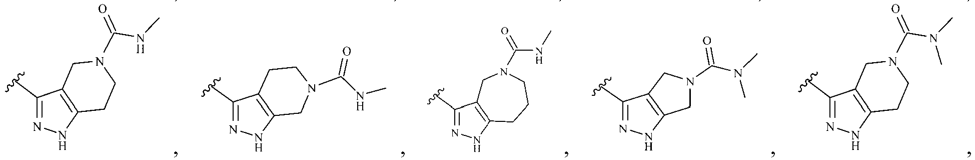

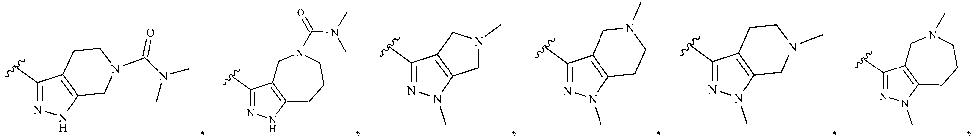

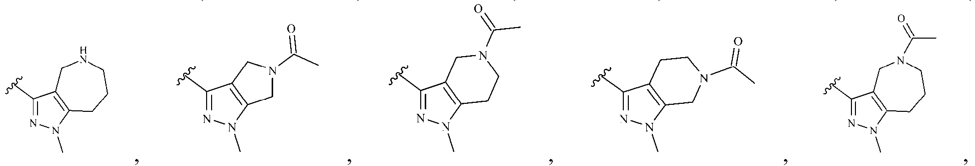

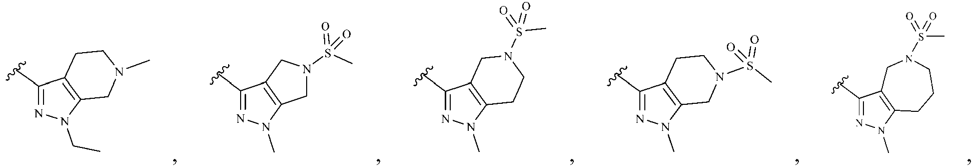

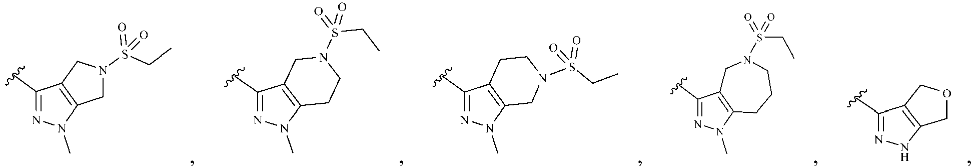

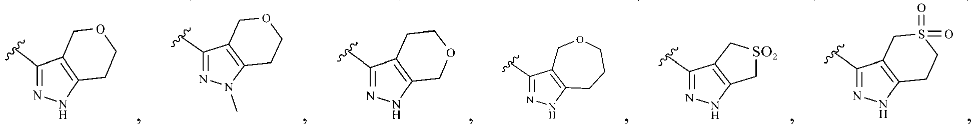

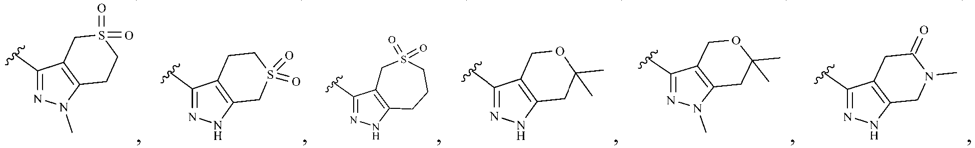

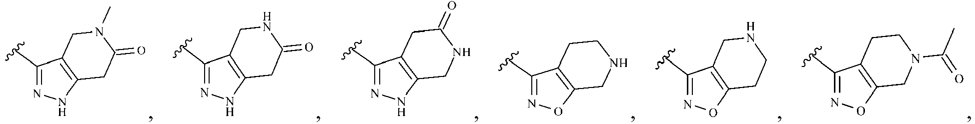

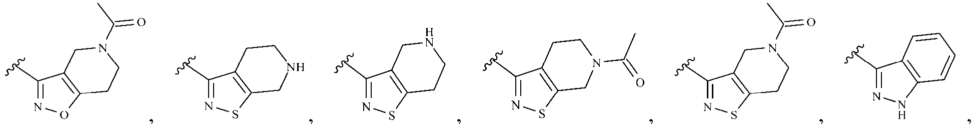

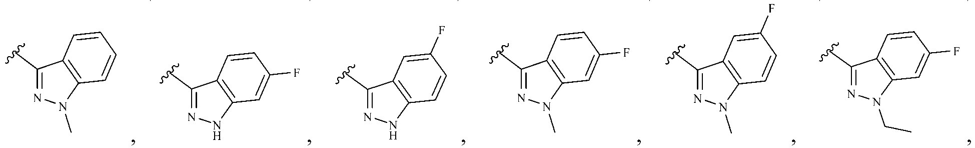

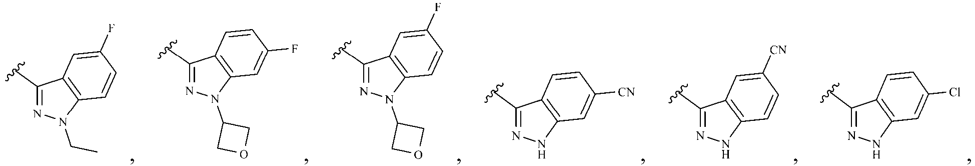

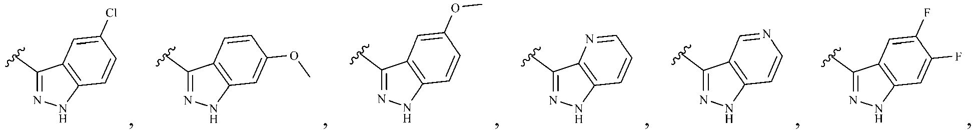

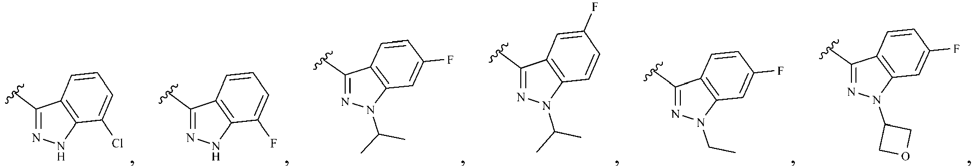

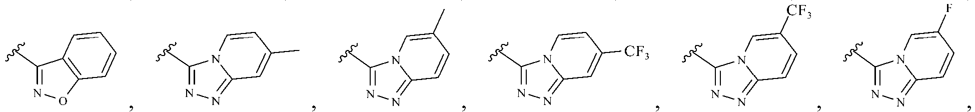

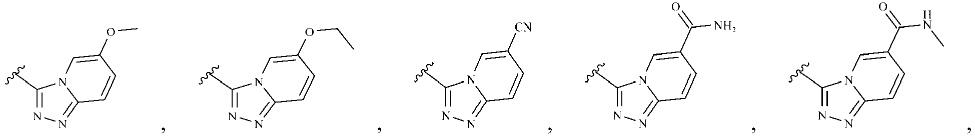

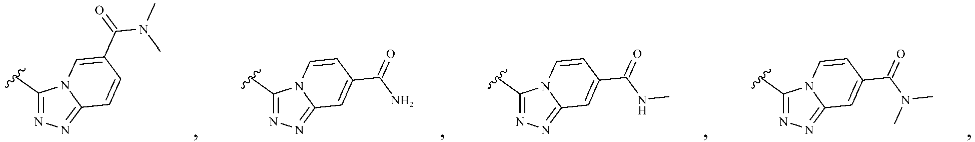

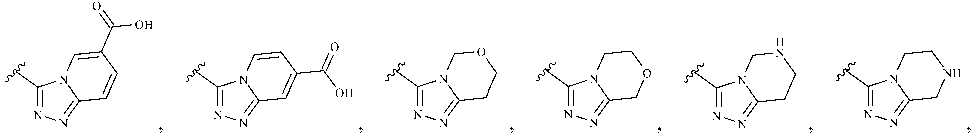

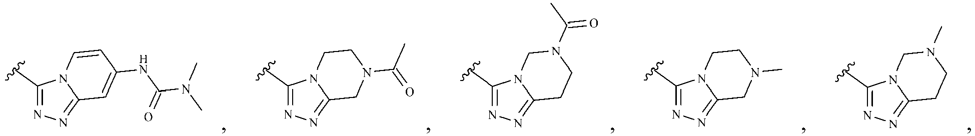

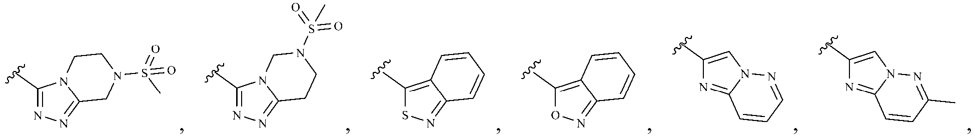

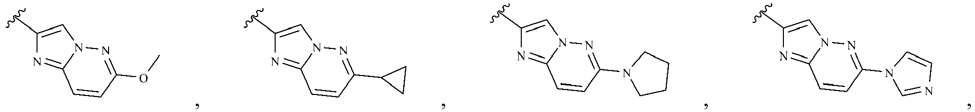

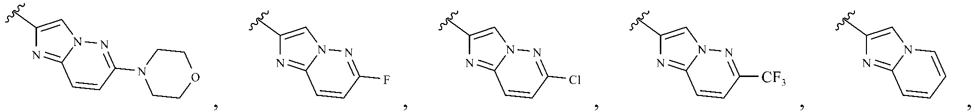

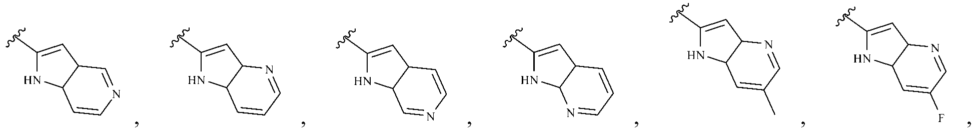

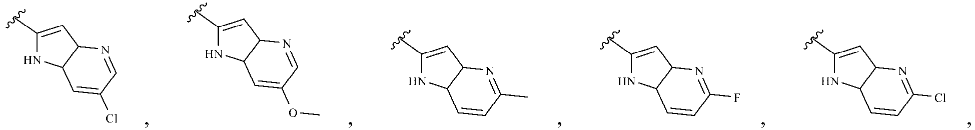

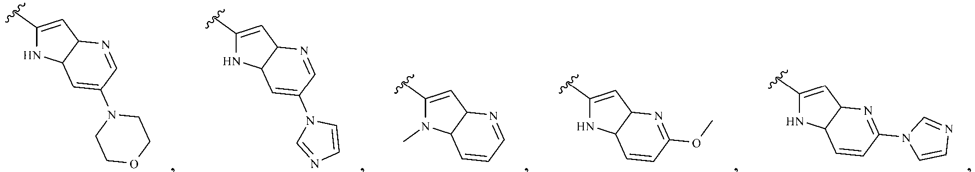

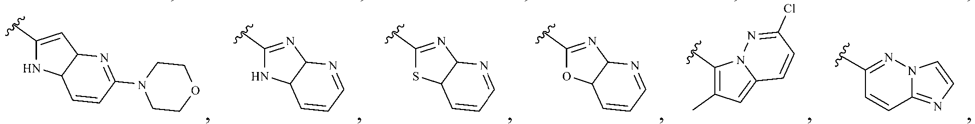

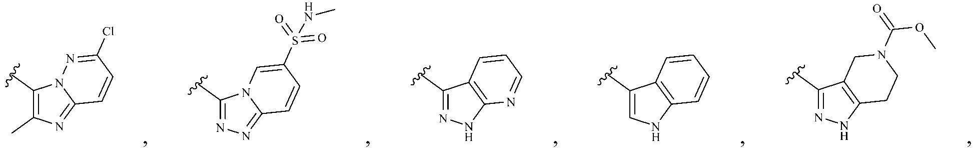

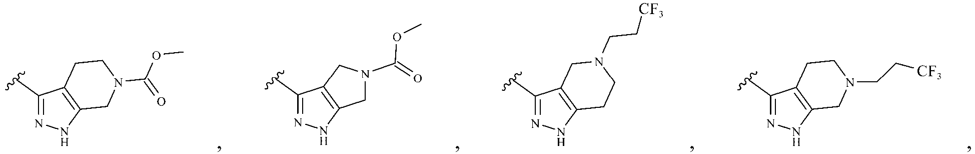

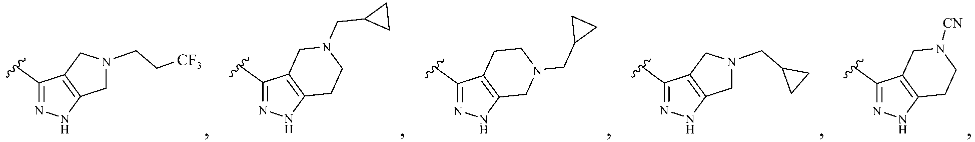

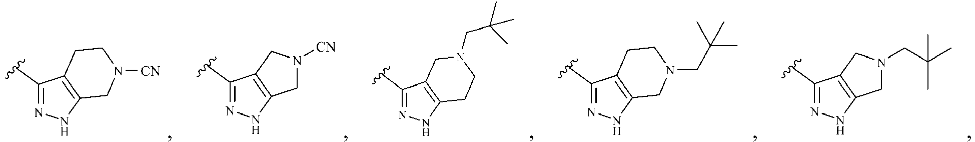

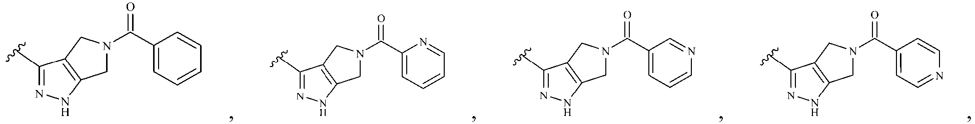

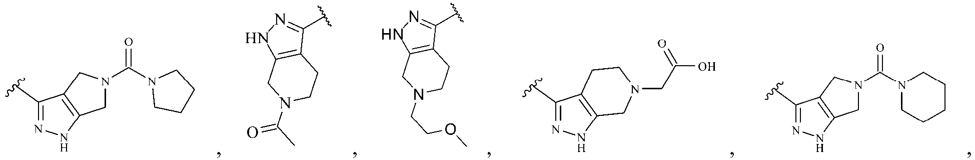

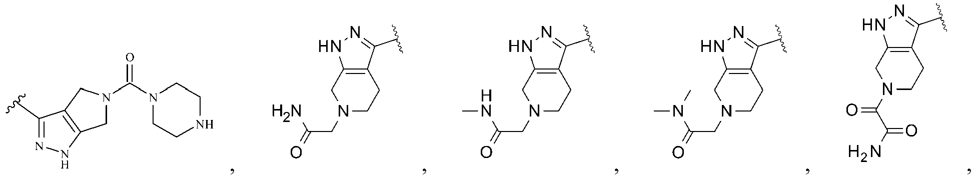

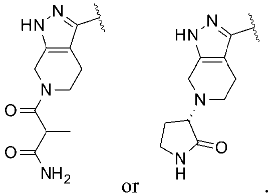

A fifth aspect of the present application provides a compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof, wherein said compound is any one of the following formulae:

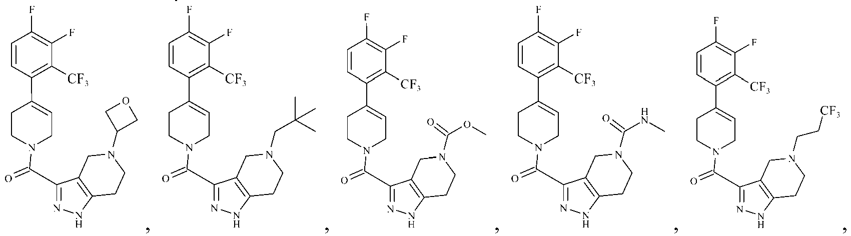

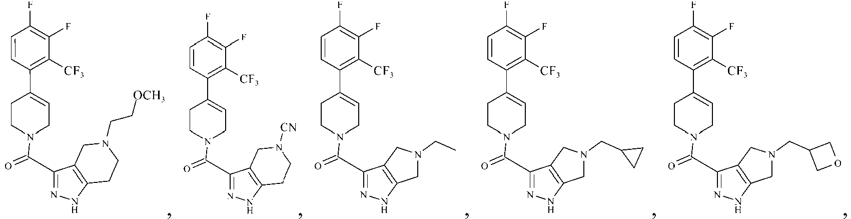

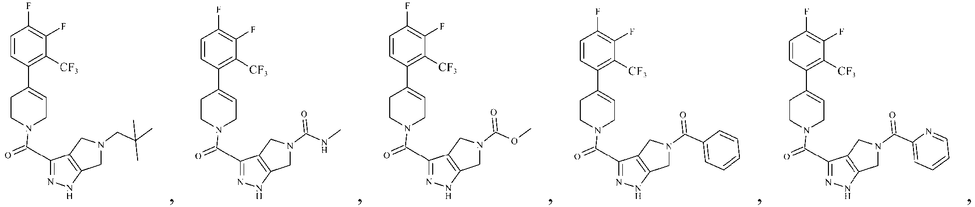

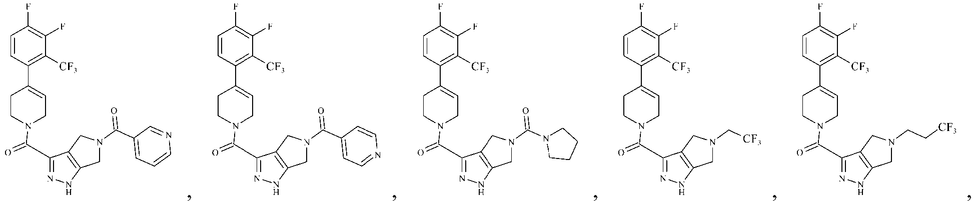

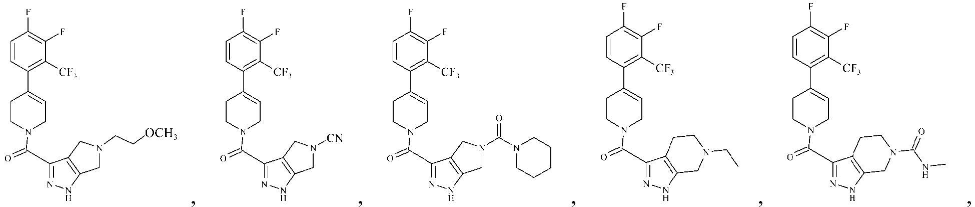

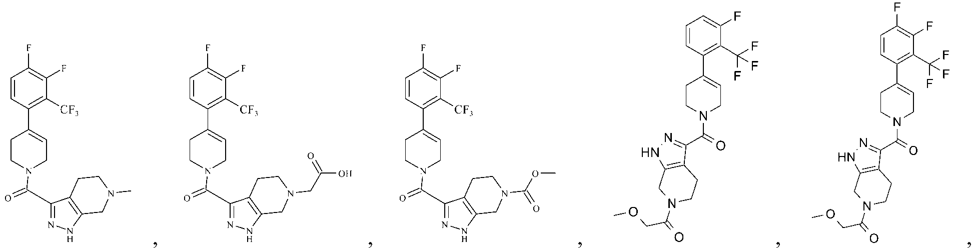

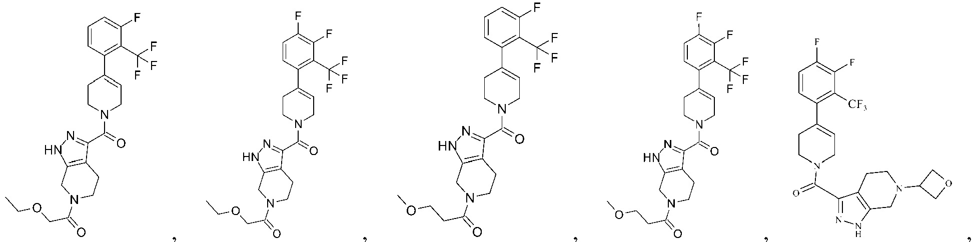

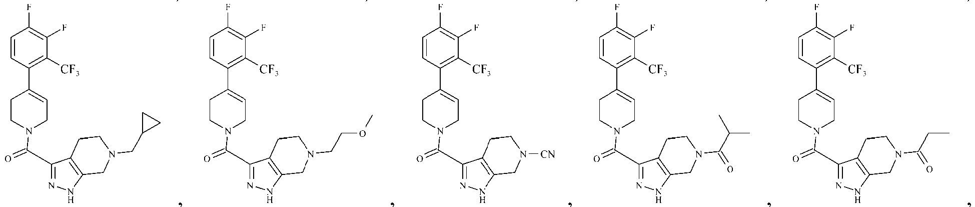

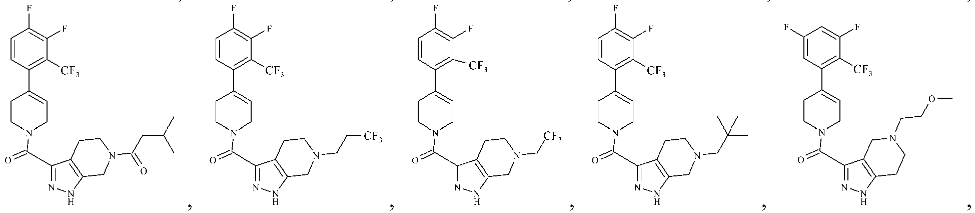

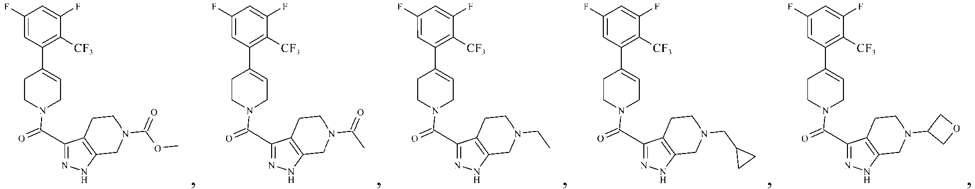

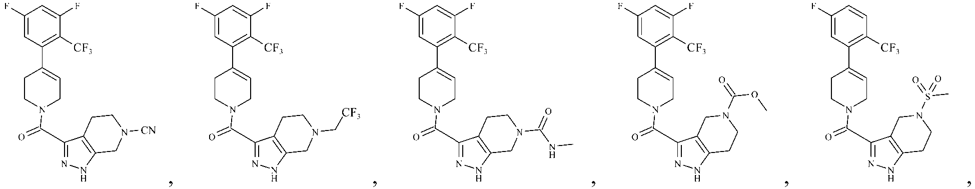

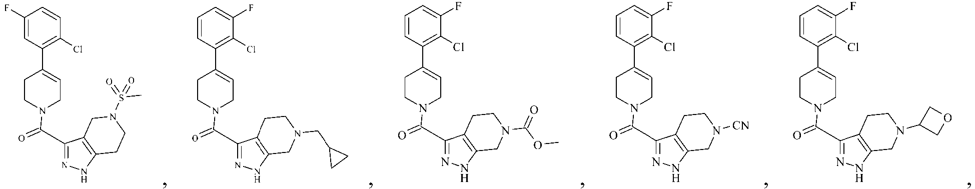

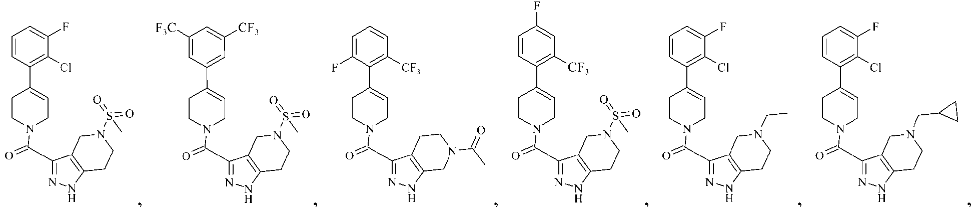

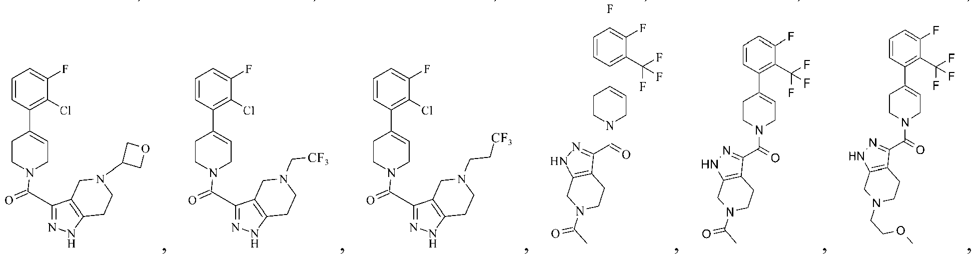

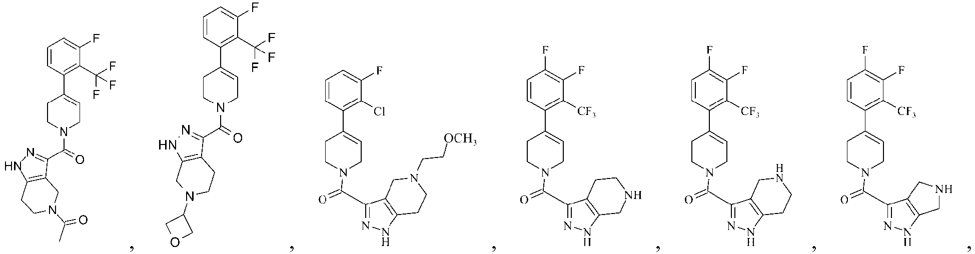

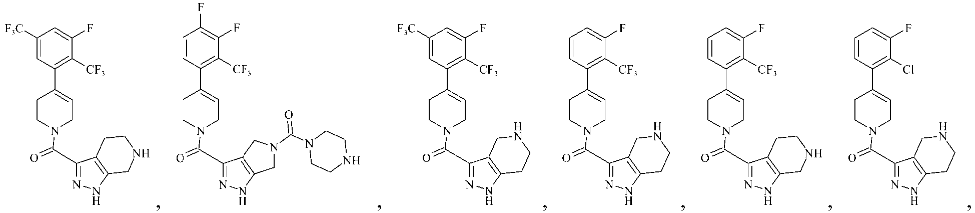

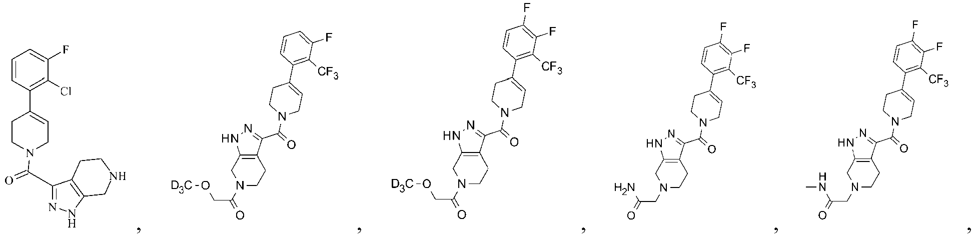

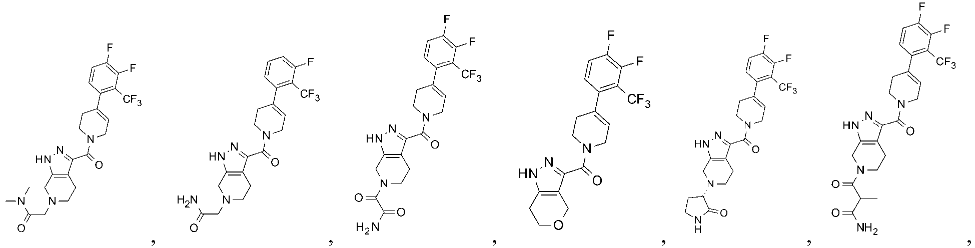

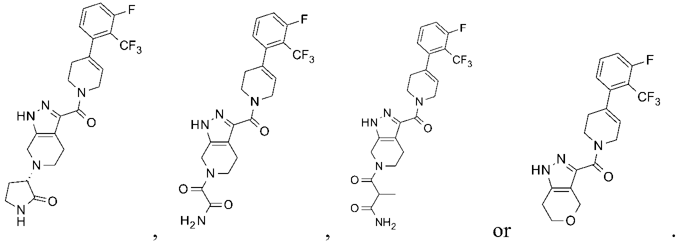

A sixth aspect of the present application provides a compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof, wherein said compound is any one of the following formulae:

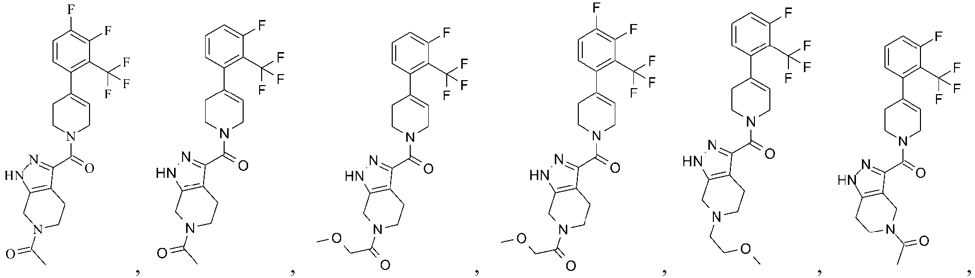

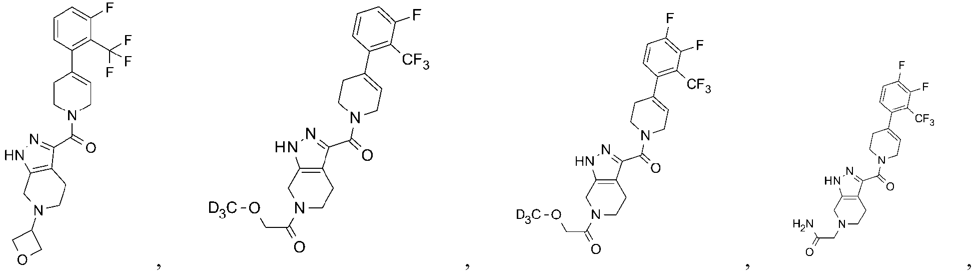

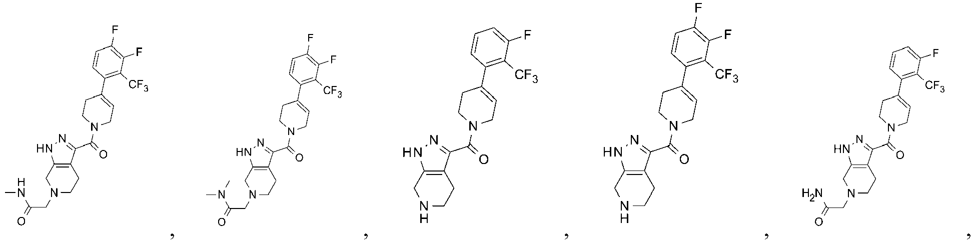

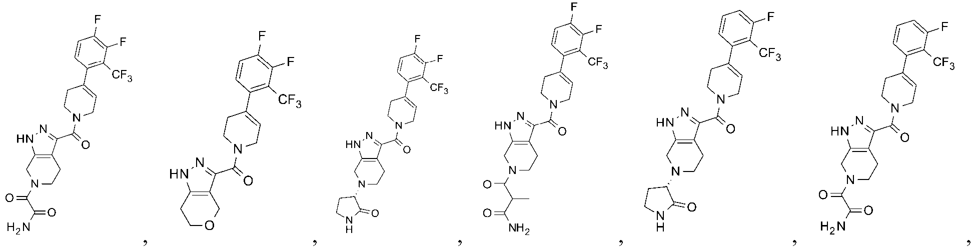

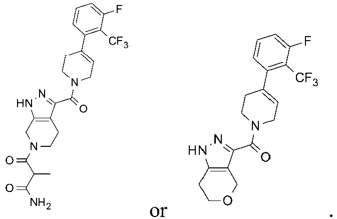

A seventh aspect of the present application provides a compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof, wherein said compound is any one of the following formulae:

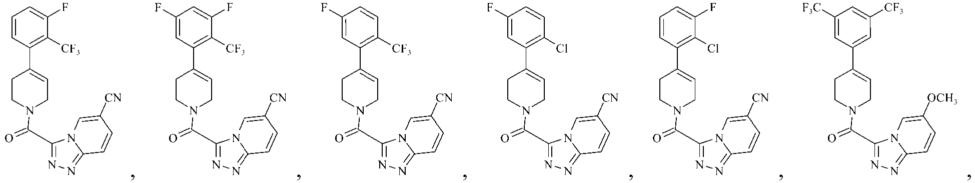

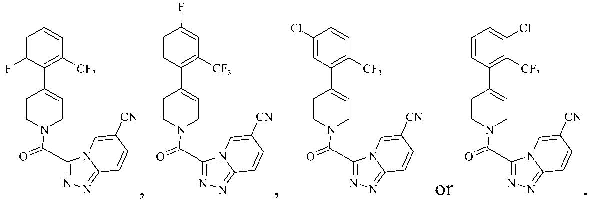

An eighth aspect of the present application provides a compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof, wherein said compound is any one of the following formulae:

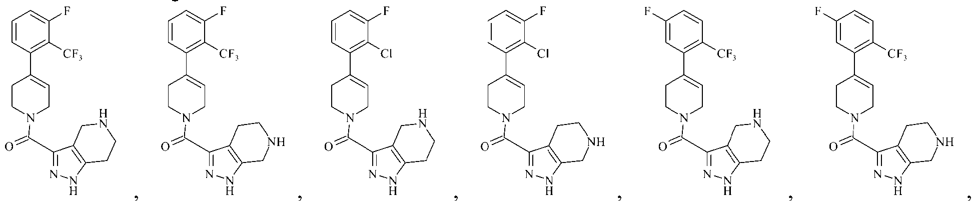

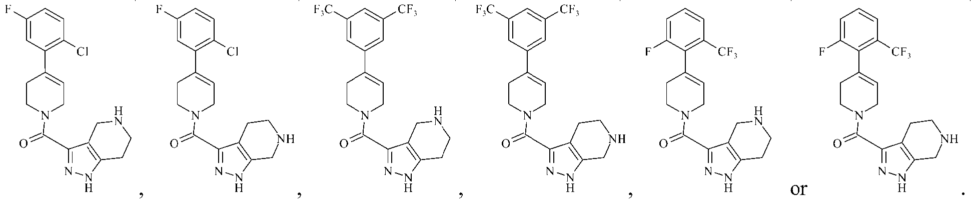

A ninth aspect of the present application provides a compound of the present application, a stereoisomer thereof, a deuterated derivative thereof, a pharmaceutically acceptable salt thereof, a pharmaceutically acceptable salt of a stereoisomer thereof, or an acceptable salt of a deuterated derivative thereof, wherein said compound is any one of the following formulae:

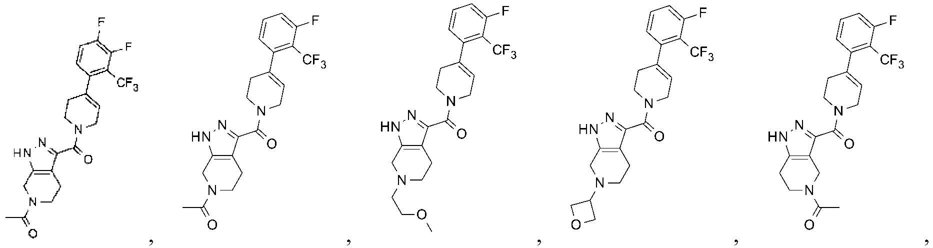

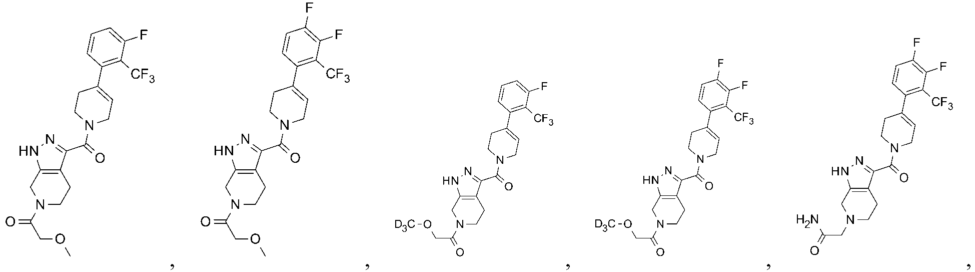

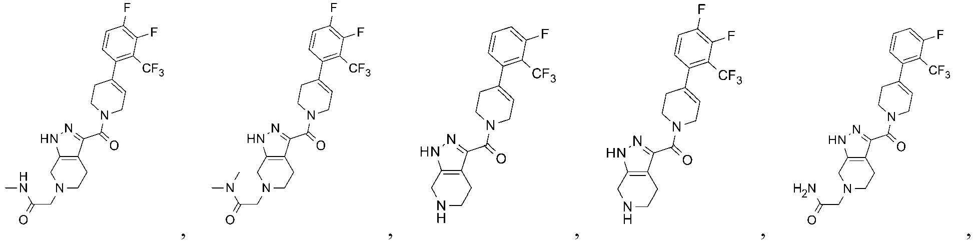

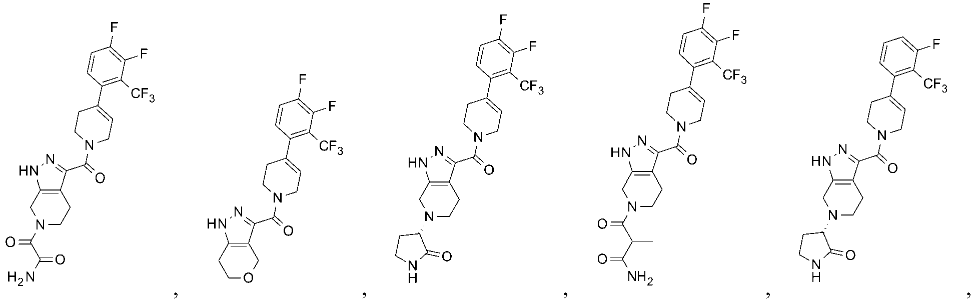

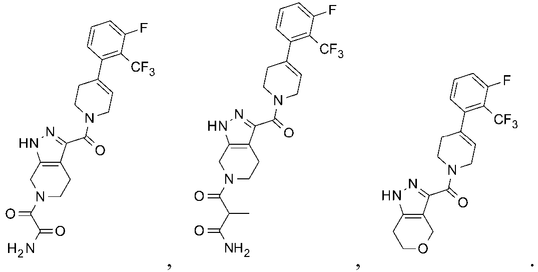

In another aspect, the present application provides a pharmaceutical composition comprising the compound of any one of the preceding aspects, a stereoisomer thereof, a deuterated derivative thereof, a pharmaceutically acceptable salt thereof, a pharmaceutically acceptable salt of a stereoisomer thereof, or an acceptable salt of a deuterated derivative thereof; and at least one pharmaceutically acceptable excipient.

In another aspect, the present application provides the use of the compound of any one of the preceding aspects, a stereoisomer thereof, a deuterated derivative thereof, a pharmaceutically acceptable salt thereof, a pharmaceutically acceptable salt of a stereoisomer thereof, or an acceptable salt of a deuterated derivative thereof; or the pharmaceutical composition of the present application in the manufacture of a medicament for the treatment of diseases associated with excessive accumulation of lipofuscin in the retina related to the serum concentration of RBP4; wherein said disease associated with excessive accumulation of lipofuscin in the retina is bisretinoid-mediated macular degeneration, including but not limited to age-related macular degeneration, dry (atrophic) age-related macular degeneration, Stargardt disease, Best disease, adult vitelliform macular dystrophy or Stargardt-like macular dystrophy, and said bisretinoid is A2E, isoA2E, A2-DHP-PE or atRALdi-PE.

In yet another aspect, the present application provides a method of treating a subject suffering from a disease associated with excessive accumulation of lipofuscin in the retina related to the serum concentration of RBP4, said method comprising administering to the subject a therapeutically effective amount of the compound of any one of the preceding aspects, a stereoisomer thereof, a deuterated derivative thereof, a pharmaceutically acceptable salt thereof, a pharmaceutically acceptable salt of a stereoisomer thereof, or an acceptable salt of a deuterated derivative thereof; or the pharmaceutical composition of the present application; wherein said disease associated with excessive accumulation of lipofuscin in the retina is bisretinoid-mediated macular degeneration, including but not limited to age-related macular degeneration, dry (atrophic) age-related macular degeneration, Stargardt disease, Best disease, adult vitelliform macular dystrophy or Stargardt-like macular dystrophy, and said bisretinoid is A2E, isoA2E, A2-DHP-PE or atRALdi-PE.

In yet another aspect, the present application provides the use of the compound of any one of the preceding aspects, a stereoisomer thereof, a deuterated derivative thereof, a pharmaceutically acceptable salt thereof, a pharmaceutically acceptable salt of a stereoisomer thereof, or an acceptable salt of a deuterated derivative thereof; or the pharmaceutical composition of the present application in the treatment of diseases associated with excessive accumulation of lipofuscin in the retina related to the serum concentration of RBP4; wherein said disease associated with excessive accumulation of lipofuscin in the retina is bisretinoid-mediated macular degeneration, including but not limited to age-related macular degeneration, dry (atrophic) age-related macular degeneration, Stargardt disease, Best disease, adult vitelliform macular dystrophy or Stargardt-like macular dystrophy, and said bisretinoid is A2E, isoA2E, A2-DHP-PE or atRALdi-PE.

### Definitions

Unless otherwise indicated, the term "halogen" as used interchangeably herein refers to fluorine, chlorine, bromine or iodine. Preferred halogen groups include -F, -Cl and -Br.

Unless otherwise indicated, the term "alkyl" as used herein includes saturated monovalent hydrocarbon groups having straight or branched chains. For example, alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl and 2-methylpentyl. Similarly, C₁₋₆ in C₁₋₆ alkyl is defined to identify groups having 1, 2, 3, 4, 5 or 6 carbon atoms in a straight or branched arrangement.

Unless otherwise indicated, the term "haloalkyl" as used herein refers to the above alkyl substituted with one or more (1, 2, 3, 4, 5 or 6) halogens (-F, -Cl or -Br). In some embodiments, the haloalkyl is interchangeably -C₁₋₆ haloalkyl or halo C₁₋₆ alkyl, wherein C₁₋₆ in -C₁₋₆ haloalkyl or halo C₁₋₆ alkyl means that the total number of carbon atoms of the alkyl is 1 to 6. In some embodiments, the -C₁₋₆ haloalkyl is -C₁₋₃ haloalkyl.

In some embodiments, the -C₁₋₃ haloalkyl is (methyl, ethyl, propyl or isopropyl) substituted with 1, 2, 3, 4, 5 or 6 -F; preferably, the -C₁₋₃ haloalkyl is -CF₃.

The term "alkylene" refers to a difunctional group obtained by removing an additional hydrogen atom from the alkyl as defined above. For example, methylene (i.e., -CH₂-), ethylene (i.e., -CH₂-CH₂- or -CH(CH₃)-) and propylene (i.e., -CH₂-CH₂-CH₂-, -CH(-CH₂-CH₃)- or -CH₂-CH(CH₃)-).

The term "alkenyl" refers to a straight or branched chain hydrocarbon group containing one or more double bonds, usually 2 to 20 carbon atoms in length. For example, "-C₂₋₆ alkenyl" contains 2 to 6 carbon atoms. Alkenyl includes, but is not limited to, vinyl, propenyl, butenyl, 2-methyl-2-buten-1-yl, heptenyl, octenyl and the like.

The term "alkynyl" refers to a straight or branched chain hydrocarbon group containing one or more triple bonds, usually 2 to 20 carbon atoms in length. For example, "-C2-6 alkynyl" contains 2 to 6 carbon atoms. Representative alkynyl groups include, but are not limited to, ethynyl, 1-propynyl, 1-butynyl, heptynyl, octynyl and the like.

The term "alkoxy" refers to an oxygen ether formed from the aforementioned alkyl groups, including but not limited to -OCH₃, -OCH₂CH₃, -OCH₂CH₂CH₃, -OCH(CH₃)₂, -CH₂OCH₃, -CH₂CH₂OCH₃.

Unless otherwise indicated, the term "haloalkoxy" as used herein refers to the above alkoxy substituted with one or more (1, 2, 3, 4, 5 or 6) halogens (-F, -Cl or -Br). In certain embodiments, haloalkoxy is interchangeably -C₁₋₆ haloalkoxy or halo C₁₋₆ alkoxy, wherein C₁₋₆ in -C₁₋₆ haloalkoxy or halo C₁₋₆ alkoxy indicates that the total number of carbon atoms of the alkoxy is 1 to 6. In certain embodiments, -C₁₋₆ haloalkoxy is -C₁₋₃ haloalkoxy. In certain embodiments, -C₁₋₆ haloalkoxy is (methoxy, ethoxy, propoxy or isopropoxy) substituted with 1, 2, 3, 4, 5 or 6 -F; preferred -C₁₋₃ haloalkoxy is -OCF₃.

Unless otherwise indicated, the term "aryl" or "aromatic ring" as used herein refers to an unsubstituted or substituted monocyclic or polycyclic aromatic ring system containing only carbon ring atoms. Preferred aryl groups are monocyclic or bicyclic 6-10 membered aromatic ring systems. Phenyl and naphthyl are preferred aryl groups.

Unless otherwise indicated, the term "heterocyclyl" or "heterocycle" as used herein refers to unsubstituted and substituted monocyclic or polycyclic non-aromatic ring systems containing one or more ring heteroatoms, including monocyclic heterocyclyl, bicyclic heterocyclyl, bridged heterocyclyl, fused heterocyclyl and spiro heterocyclyl. Preferred heteroatoms include N, O and S, including N-oxides, sulfur oxides and dioxides. Preferably, the heterocyclyl is a three to ten membered ring that is fully saturated or has one or more degrees of unsaturation. Multiple degrees of substitution (preferably one, two or three) are included within the present definition. Examples of such heterocyclyl groups include, but are not limited to, azetidinyl, pyrrolidinyl, piperidinyl, piperazinyl, oxopiperazinyl, oxopiperidinyl, oxazepanyl, azepanyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiomorpholinyl sulfoxide, thiomorpholinyl sulfone and oxadiazolyl.

Unless otherwise indicated, the term "heteroaryl" as used herein denotes an aromatic ring system containing carbon and at least one heteroatom. Heteroaryl or heteroaromatic ring can be monocyclic or polycyclic, substituted or unsubstituted. A monocyclic heteroaryl ring may have 1 to 4 heteroatoms in its ring, while a polycyclic heteroaryl ring may contain 1 to 10 heteroatoms. Polycyclic heteroaryl rings may contain fused, spiro or bridged ring linkages, for example, a bicyclic heteroaryl ring is a polycyclic heteroaryl ring. A bicyclic heteroaryl ring may contain 8 to 12 member atoms. A monocyclic heteroaryl ring may contain 5 to 8 member atoms (carbon atoms and heteroatoms). Examples of heteroaryl groups include, but are not limited to, thienyl, furyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuryl, benzothienyl, benzisoxazolyl, benzoxazolyl, benzopyrazolyl, benzothiazolyl, benzothiadiazolyl, benzotriazolyl, adeninyl, quinolinyl or isoquinolinyl.

The term "carbocyclyl" refers to a substituted or unsubstituted monocyclic, bicyclic, bridged, fused, spiro non-aromatic ring system containing only carbon atoms. The term "carbocycle" refers to a substituted or unsubstituted monocyclic, bicyclic, bridged, fused, spiro non-aromatic ring system containing only carbon atoms. Preferably, the ring is three to ten membered, and is either fully saturated or has one or more degrees of unsaturation. Multiple degrees of substitution, preferably one, two or three, are included within this definition. Carbocyclyl includes, but is not limited to, cycloalkyl, cycloalkenyl and cycloalkynyl. Exemplary "cycloalkyl" groups include, but are not limited to, cyclopropyl, cyclobutyl, cyclopentyl, cyclohexyl and the like.

The term "one or more" refers to one or more. In some embodiments, "one or more" refers to 1, 2, 3, 4, 5 or 6. In some embodiments, "one or more" refers to 1, 2, 3 or 4. In some embodiments, "one or more" refers to 1, 2 or 3. In some embodiments, "one or more" refers to 1 or 2. In some embodiments, "one or more" refers to 1. In some embodiments, "one or more" refers to 2. In some embodiments, "one or more" refers to 3. In some embodiments, "one or more" refers to 4. In some embodiments, "one or more" refers to 5. In some embodiments, "one or more" refers to 6.

In the present application, when a ring is substituted with one or more substituents, it means that each substituent may be independently substituted on each ring atom of the ring, including but not limited to ring carbon atoms or ring nitrogen atoms. Furthermore, when the ring is polycyclic, such as fused, bridged or spiro, each substituent may be independently substituted on each ring atom of the polycyclic ring.

The term "oxo" means that oxygen together with the carbon atom to which it is attached forms

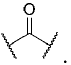

As used herein, the term "composition" is intended to encompass a product comprising a specified amount of a specified ingredient, and any product produced directly or indirectly from the combination of specified amounts of specified ingredients. Accordingly, a pharmaceutical composition comprising the compound of the present invention as an active ingredient and a method for preparing the compound of the present invention are also part of the present invention. Furthermore, some crystalline forms of the compound may exist as polymorphs and are therefore intended to be included herein. In addition, some compounds may form solvates with water (i.e., hydrates) or common organic solvents, and such solvates are also included within the scope of the present application.

The term "pharmaceutically acceptable salt" refers to a salt prepared from a pharmaceutically acceptable non-toxic base or acid. When the compound of the present application is acidic, its corresponding salt may be conveniently prepared from pharmaceutically acceptable non-toxic bases, including inorganic bases and organic bases. When the compound of the present application is basic, its corresponding salt may be conveniently prepared from pharmaceutically acceptable non-toxic acids, including inorganic acids and organic acids. Since the compounds of the present application are intended for pharmaceutical use, they are preferably provided in substantially pure form, for example at least 60% pure, more suitably at least 75% pure, especially at least 98% pure (% by weight).

The present application includes within its scope prodrugs of the compounds of the present application. In general, such prodrugs are functional derivatives of the compounds that are readily convertible in vivo to the desired compound. Accordingly, in the methods of treatment of the present application, the term "administering" shall include treating various conditions with the specifically disclosed compound or with a compound that may not be specifically disclosed but which is converted in vivo to the specific compound after administration to a subject. Conventional procedures for the selection and preparation of suitable prodrug derivatives are described, for example, in "Design of Prodrugs", ed. H. Bundgaard, Elsevier, 1985.

The definition of any substituent or variable at a particular position in a molecule is intended to be independent of the definition of any substituent or variable at other positions in the molecule. It is understood that those of ordinary skill in the art can select substituents and substitution patterns on the compounds of the present application to provide chemically stable compounds that can be readily synthesized by techniques known in the art and by the methods set forth herein.

The compounds described herein may contain one or more asymmetric centers and thus give rise to diastereomers and optical isomers. The present application includes all such possible diastereomers and their racemic mixtures, their substantially pure resolved enantiomers, all possible geometric isomers, and pharmaceutically acceptable salts thereof.

The present application includes all stereoisomers of the compounds and pharmaceutically acceptable salts thereof. Furthermore, mixtures of stereoisomers and isolated specific stereoisomers are also included. During the synthetic steps used to prepare these compounds, or during the use of racemization or epimerization methods known to those skilled in the art, the products of these steps may be mixtures of stereoisomers.

The term "stereoisomer" as used herein refers to isomers resulting from the same order of connection of atoms or groups of atoms in a molecule but different spatial arrangements, including configurational isomers and conformational isomers, wherein configurational isomers include geometric isomers and optical isomers, and optical isomers mainly include enantiomers and diastereomers. The present application includes all possible stereoisomers of the compound.

Certain compounds provided herein may exist as atropisomers, which are conformational stereoisomers that occur when rotation about a single bond in the molecule is prevented or greatly slowed due to steric interactions with other parts of the molecule. The compounds provided herein include all atropisomers, including pure individual atropisomers, respective enriched atropisomers or respective non-specific mixtures. Isolation of atropisomers may be permitted if the rotational barrier about the single bond is sufficiently high and interconversion between conformations is sufficiently slow.

The present application is intended to include all isotopes of atoms present in the compounds of the present application. Isotopes are atoms with the same atomic number but different mass numbers. By way of general example and not limitation, isotopes of hydrogen include deuterium and tritium. Isotopes of hydrogen may be represented as ¹H (hydrogen), ²H (deuterium) and ³H (tritium). They are also commonly denoted as D (deuterium) and T (tritium). In the present application, CD₃ represents a methyl group in which all hydrogen atoms are deuterium. Isotopes of carbon include ¹³C and ¹⁴C. The isotopically-labeled compounds of the present application can generally be prepared by conventional techniques known to those skilled in the art or by methods analogous to those described herein using an appropriately isotopically-labeled reagent in place of a non-labeled reagent.

Unless otherwise indicated, the term "deuterated derivative" as used herein refers to a compound having the same chemical structure as the reference compound, but with one or more hydrogen atoms replaced by deuterium atoms ("D"). It will be appreciated that some natural isotopic abundance variations will occur in the synthesized compounds depending on the source of the chemical materials used in the synthesis. The concentration of naturally abundant stable hydrogen isotopes, compared to the degree of stable isotopic substitution of the deuterated derivatives described herein, is small and immaterial, however. Thus, unless otherwise indicated, when referring to a "deuterated derivative" of a compound disclosed herein, at least one hydrogen is replaced by deuterium at a level well above its natural isotopic abundance (generally about 0.015%). In some embodiments, the deuterated derivatives disclosed herein have an isotopic enrichment factor of at least 3500 (containing 52.5% deuterium at each designated deuterium), at least 4500 (containing 67.5% deuterium), at least 5000 (containing 75% deuterium), at least 5500 (containing 82.5% deuterium), at least 6000 (containing 90% deuterium), at least 6333.3 (containing 95% deuterium), at least 6466.7 (containing 97% deuterium), or at least 6600 (containing 99% deuterium) for each deuterium atom.

When tautomers exist in the compounds of the present application, the present application includes any possible tautomers and pharmaceutically acceptable salts thereof and mixtures thereof, unless otherwise specified.

The pharmaceutical composition of the present application comprises the compound of the present application (or a pharmaceutically acceptable salt thereof) as an active ingredient, a pharmaceutically acceptable carrier and optionally other therapeutic ingredients or adjuvants. The compositions include those suitable for oral, rectal, topical and parenteral (including subcutaneous, intramuscular and intravenous) administration, although the most suitable route in any given case will depend on the particular host and the nature and severity of the condition for which the active ingredient is being administered. The pharmaceutical composition may conveniently be presented in unit dosage form and prepared by any method well known in the pharmaceutical arts.

In practice, the compound of the present application, or a prodrug or metabolite thereof, or a pharmaceutically acceptable salt thereof, may be intimately admixed as the active ingredient with a pharmaceutical carrier according to conventional pharmaceutical compounding techniques. The carrier may take a wide variety of forms depending on the form of preparation desired for the route of administration, e.g., oral or parenteral (including intravenous) administration. Thus, the pharmaceutical compositions of the present application may exist as discrete units suitable for oral administration, such as capsules, cachets or tablets, each containing a predetermined amount of the active ingredient. In addition, the compositions may exist as powders, granules, solutions, suspensions in aqueous liquids, non-aqueous liquids, oil-in-water emulsions or water-in-oil emulsions. In addition to the common dosage forms mentioned above, the compound represented by formula I or a pharmaceutically acceptable salt thereof may also be administered by controlled release means and/or delivery devices. The compositions may be prepared by any pharmaceutical method. Generally, such methods include the step of bringing the active ingredient into association with the carrier which constitutes one or more necessary ingredients. In general, the compositions are prepared by uniformly and intimately admixing the active ingredient with liquid carriers or finely divided solid carriers or both. The product may then be conveniently shaped into the desired form.

Thus, the pharmaceutical compositions of the present application may comprise a pharmaceutically acceptable carrier and a compound or a pharmaceutically acceptable salt thereof. The compound of formula I or a pharmaceutically acceptable salt thereof may also be included in a pharmaceutical composition in combination with one or more other therapeutically active compounds.

The pharmaceutical carrier employed may be, for example, a solid, liquid or gas. Examples of solid carriers include lactose, gypsum powder, sucrose, talc, gelatin, agar, pectin, acacia, magnesium stearate and stearic acid. Examples of liquid carriers are syrup, peanut oil, olive oil and water. Examples of gaseous carriers include carbon dioxide and nitrogen. In preparing the compositions for oral dosage forms, any convenient pharmaceutical medium may be used. For example, water, glycols, oils, alcohols, flavoring agents, preservatives, coloring agents and the like may be used to form oral liquid preparations such as suspensions, elixirs and solutions; while carriers such as starches, sugars, microcrystalline cellulose, diluents, granulating agents, lubricants, binders, disintegrating agents and the like may be used to form oral solid preparations such as powders, capsules and tablets. Because of their ease of administration, tablets and capsules are the preferred oral dosage units, wherein solid pharmaceutical carriers are used. Optionally, tablets may be coated by standard aqueous or non-aqueous techniques.

Tablets containing the composition of the present application may be prepared by compression or molding, optionally containing one or more accessory ingredients or adjuvants. Compressed tablets may be prepared by compressing, in a suitable machine, the active ingredient in a free-flowing form such as powder or granules, optionally mixed with a binder, lubricant, inert diluent, surface active agent or dispersing agent. Molded tablets may be made by molding in a suitable machine a mixture of the powdered compound moistened with an inert liquid diluent. Each tablet preferably contains from about 0.05 mg to about 5 g of the active ingredient, and each cachet or capsule preferably contains from about 0.05 mg to about 5 g of the active ingredient. For example, formulations for oral administration to humans may contain from about 0.5 mg to about 5 g of active agent admixed with an appropriate and convenient amount of carrier material which may vary from about 0.05 to about 95% of the total composition. Unit dosage forms will generally contain from about 0.01 mg to about 2 g of active ingredient, typically 0.01 mg, 0.02 mg, 1 mg, 2 mg, 3 mg, 4 mg, 5 mg, 6 mg, 7 mg, 8 mg, 9 mg, 10 mg, 25 mg, 50 mg, 100 mg, 200 mg, 300 mg, 400 mg, 500 mg, 600 mg, 800 mg, 1000 mg, 1500 mg or 2000 mg.

Pharmaceutical compositions of the present application suitable for parenteral administration may be prepared as solutions or suspensions of the active compound in water. Suitable surfactants, such as hydroxypropyl cellulose, may be included. Dispersions can also be prepared in glycerol, liquid polyethylene glycols and mixtures thereof in oils. In addition, preservatives may be included to prevent the harmful growth of microorganisms.

Pharmaceutical compositions suitable for injectable use include sterile aqueous solutions or dispersions. Furthermore, the compositions may be in the form of sterile powders for the extemporaneous preparation of such sterile injectable solutions or dispersions. In all cases, the final injectable form must be sterile and must be fluid to facilitate injection. The pharmaceutical compositions must be stable under the conditions of manufacture and storage; thus, preferably they should be preserved against the contaminating action of microorganisms such as bacteria and fungi. The carrier can be, for example, a solvent or dispersion medium containing water, ethanol, polyols (e.g., glycerol, propylene glycol and liquid polyethylene glycols), vegetable oils and suitable mixtures thereof.

The pharmaceutical compositions of the present application may be in a form suitable for topical use, such as aerosols, creams, ointments, lotions, dusting powders and the like. In addition, the compositions may be in a form suitable for transdermal devices. These preparations can be prepared by conventional processing methods utilizing the compound represented by formula (I) of the present application or a pharmaceutically acceptable salt thereof. For example, a cream or ointment is prepared by mixing hydrophilic materials and water with about 0.05 wt% to about 10 wt% of the compound to produce a cream or ointment having a desired consistency.

The pharmaceutical compositions of the present application may be in a form suitable for rectal administration wherein the carrier is a solid. Preferably, the mixture forms unit dose suppositories. Suitable carriers include cocoa butter and other materials commonly used in the art. Suppositories may be conveniently formed by first mixing the composition with a softened or melted carrier and then cooling and shaping in a mold.

In addition to the carrier components mentioned above, the pharmaceutical preparations mentioned above may suitably include one or more additional carrier components such as diluents, buffers, flavoring agents, binders, surfactants, thickeners, lubricants, preservatives (including antioxidants) and the like. In addition, other adjuvants may be included to render the preparation isotonic with the blood of the intended recipient. Compositions containing the compound or a pharmaceutically acceptable salt thereof may also be prepared in powder or liquid concentrate form.

Generally, dosage levels of about 0.001 mg/kg to about 150 mg/kg body weight per day may be used for the treatment of the above conditions, or about 0.05 mg to about 7 g per patient per day. For example, administration of about 0.001 to 50 mg of the compound per kg body weight per patient per day, or about 0.05 mg to about 3.5 g per patient per day, may be effective for the treatment of age-related macular degeneration, dry (atrophic) age-related macular degeneration, Stargardt disease, Best disease, adult vitelliform macular dystrophy or Stargardt-like macular dystrophy and the like. It should be understood, however, that the specific dosage level for any particular patient will depend upon a variety of factors including age, body weight, general health, sex, diet, time of administration, route of administration, rate of excretion, drug combination and the severity of the particular disease being treated.

Unless the context indicates otherwise, when a value is expressed as "about X" or "approximately X", the stated value of X will be understood to be accurate to +10%, preferably +5%, +2%.

These and other aspects of the present application will become apparent from the following written description of the present application.

### Brief description of the drawings

The accompanying drawings described herein are provided to further understand the present application and constitute a part of the present application. The exemplary embodiments of the present application and their descriptions are used to explain the present application and do not constitute an improper limitation to the present application.
Figure 1 is a ¹H NMR spectrum of Compound 1 synthesized in Example 1 of the present application;
Figure 2 is a graph showing the comparison of RBP4 reduction rates of Compound 17 of the present application and a reference compound at a dose of 1 mg/kg at different time points;
Figure 3 is a graph showing the comparison of RBP4 reduction rates of Compound 17 of the present application and a reference compound at a dose of 5 mg/kg at different time points.

### Detailed Description of the Embodiments

The compounds of the present application can be synthesized from commercially available reagents using the synthetic methods and reaction schemes described herein. The examples outlining specific synthetic routes are intended to guide synthetic chemists in the art, who will readily appreciate that solvents, concentrations, reagents, protecting groups, sequence of synthetic steps, time, temperature, and the like can be modified within the skill and judgment of those skilled in the art as needed.

The following examples are provided to better illustrate the present application. Unless expressly stated otherwise, all parts and percentages are by weight, and all temperatures are in degrees Celsius. The abbreviations in the table below are used in the examples:

| | |
|---|---|
| DMF | N, N-dimethylformamide |
| DMA | methylacetamide |
| EA, EtOAc | ethyl acetate |
| DCM | dichloromethane |
| Pd(PPh₃)₄ | tetrakis(triphenylphosphine)palladium |
| EDCI | 1-(3-dimethylaminopropyl)-3-ethylcarbodiimide hydrochloride |
| HOBt | 1-hydroxybenzotriazole |
| DIEA | N, N-diisopropylethylamine |
| TFA | trifluoroacetic acid |
| TEA, Et₃N | triethylamine |
| PE | petroleum ether |
| MeCN | acetonitrile |
| HOAc | acetic acid |
| MeOH | methanol |
| room temperature | 20°C-30°C |
| min(s) | minute(s) |
| h/hr(s) | hour(s) |

### Example 1

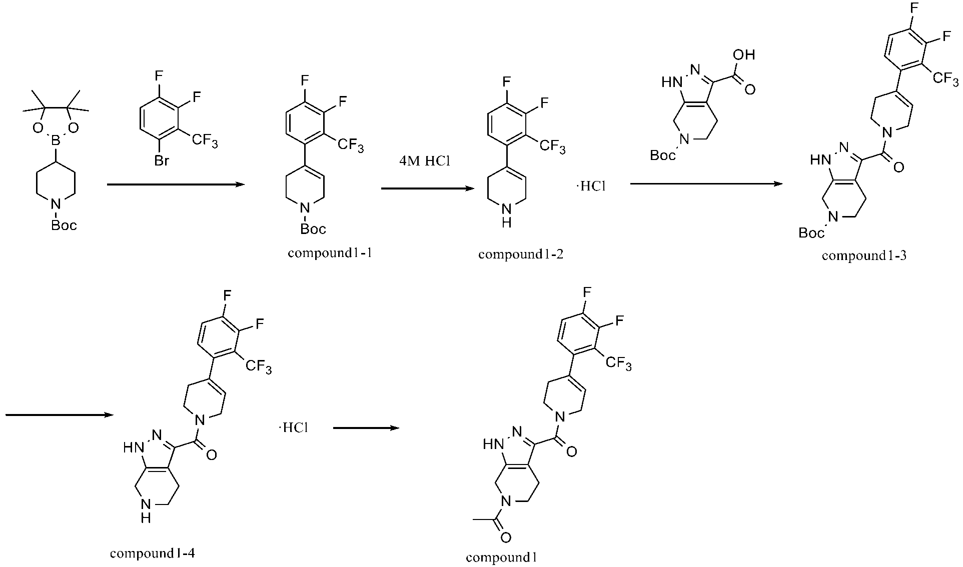

3,4-difluoro-2-(trifluoromethyl)bromobenzene (835 mg, 3.2 mmol), Pd(PPh₃)₄ (370 mg, 0.32 mmol), and aqueous K₂CO₃ solution (2.6 mL, 2 M, 5.2 mmol) were added to a solution of N-Boc-1,2,5,6-tetrahydropyridine-4-boronic acid pinacol ester (989 mg, 3.2 mmol) in 1,4-dioxane (dry solvent, 40 mL). The resulting reaction mixture was stirred at 80°C overnight under nitrogen atmosphere, quenched with water, and extracted with EtOAc. The obtained organic phase was washed with saturated brine, dried over anhydrous sodium sulfate, and concentrated to give a residue. The residue was purified by silica gel column chromatography (eluted with V_{petroleum ether}/V_{ethyl acetate} = 20/1) to obtain Compound 1-1 as a yellow liquid (834.8 mg, 71.5% yield). LCMS: m/z = 364.0 [M+H]⁺.

A solution of Compound 1-1 (832 mg, 2.29 mmol) in HCl/1,4-dioxane (32 mmol, 4 M, 8 mL) was stirred at room temperature for 2 hours, and then concentrated under reduced pressure to obtain the crude hydrochloride salt of Compound 1-2, which can be directly used in the next step without purification. LCMS: m/z = 264.4 [M+H]⁺.

EDCI (527 mg, 2.748 mmol), HOBt (371 mg, 2.748 mmol), and DIEA (1.2 mL, 6.87 mmol) were added to a solution of 6H-pyrazolo[3,4-c] 1,4,5,7-tetrahydropyridine-3,6-dicarboxylic acid 6-(1,1-dimethylethyl) ester (612 mg, 2.29 mmol) and the hydrochloride salt of Compound 1-2 (2.29 mmol) in DMF (dry solvent, 20 mL) at room temperature. The resulting reaction mixture was stirred at room temperature overnight, quenched with water, and extracted with EtOAc. The obtained organic phase was washed with saturated brine, dried over anhydrous sodium sulfate, and concentrated to give a residue. The residue was purified by silica gel column chromatography (eluted with V_{DCM}/V_{MeoH} = 30/1) to obtain Compound 1-3 as a pale yellow liquid (793 mg, 67.5% yield). LCMS: m/z = 513.3 [M+H]⁺.

A solution of Compound 1-3 (789 mg, 1.54 mmol) in HCl/1,4-dioxane (21.56 mmol, 4 M, 5.4 mL) was stirred at room temperature for 2 hours, and then concentrated under reduced pressure to obtain the crude hydrochloride salt of Compound 1-4, which can be directly used in the next step without purification. LCMS: m/z = 413.4 [M+H]⁺.

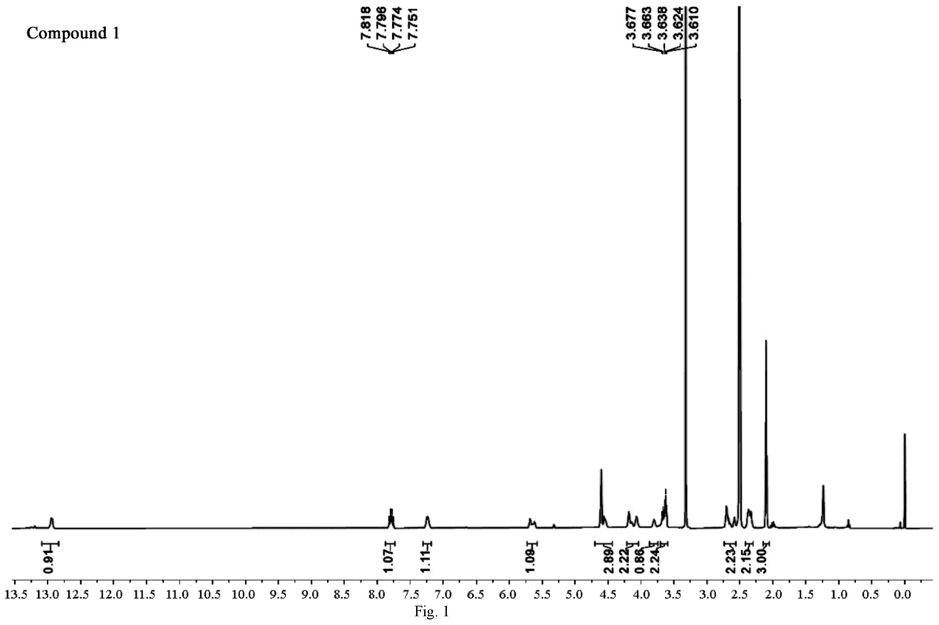

Acetic anhydride (30 µL, 0.315 mmol) diluted with DCM (dry, 1 mL) was slowly added to a solution of the hydrochloride salt of Compound 1-4 (0.35 mmol) and Et₃N (0.15 mL, 1.05 mmol) in DCM (5 mL) at 0°C. The resulting reaction mixture was stirred at 0°C for 30 minutes, quenched with water, and extracted with EtOAc. The obtained organic phase was washed with saturated brine, dried over anhydrous sodium sulfate, and concentrated to give a residue. The residue was purified by silica gel column chromatography (eluted with V_{DCM}/V_{MeOH} = 20/1) to obtain a crude product. The obtained crude product was purified by Prep-HPLC (C18 column, eluted with CH₃CN/H₂O) to obtain Compound 1 as a white solid (32.1 mg, 20.2% yield). LCMS: m/z = 455.3 [M+H]⁺. Figure 1 is a ¹H NMR spectrum of Compound 1 synthesized in Example 1 of the present application.

¹H NMR (400 MHz, DMSO-d6) δ 13.0-12.85 (m, 1H), 7.78 (q, J = 8.8 Hz, 1H), 7.30-7.17 (m, 1H), 5.73-5.58 (m, 1H), 4.69-4.51 (m, 3H), 4.21-4.03 (m, 2H), 3.86-3.74 (m, 1H), 3.72-3.59 (m, 2H), 2.73-2.56 (m, 2H), 2.41-2.29 (m, 2H), 2.14-2.05 (m, 3H).

### Example 2

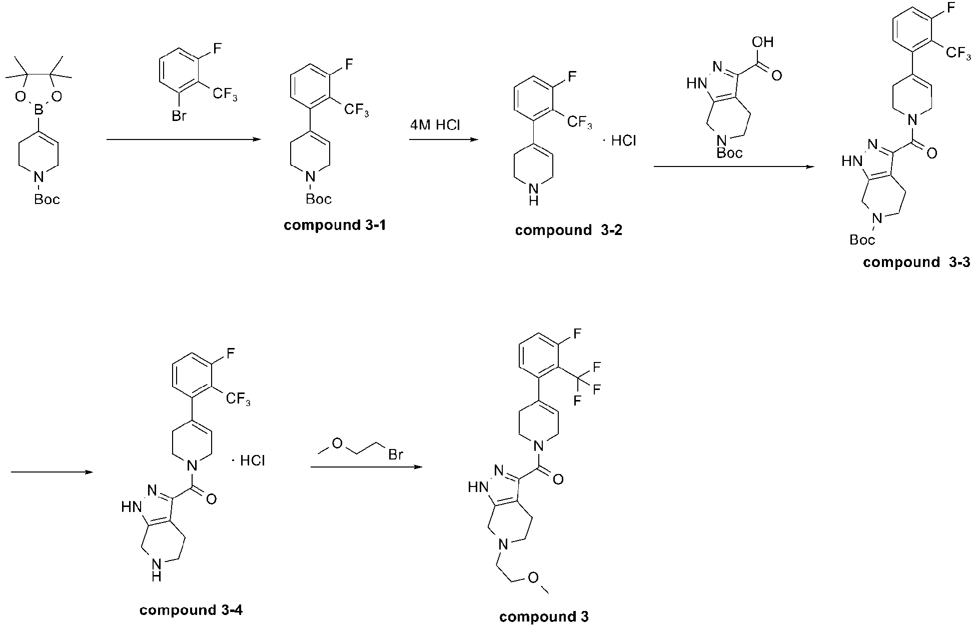

N-Boc-1,2,5,6-tetrahydropyridine-4-boronic acid pinacol ester (4.638 g, 15 mmol) was dissolved in anhydrous 1,4-dioxane (40 mL), and 3-fluoro-2-(trifluoromethyl)bromobenzene (3.645 g, 15 mmol), Pd(PPh₃)₄ (1.733 g, 1.5 mmol), and 12 mL K₂CO₃ (2 M aqueous solution, 24 mmol) were added. The resulting mixture was heated to 80°C and stirred overnight under nitrogen atmosphere. The resulting mixture was quenched with water and extracted with EtOAc. The organic solution was dried over anhydrous Na₂SO₄ and concentrated. The crude product was purified by silica gel column chromatography (PE/EtOAc = 20/1, volume ratio) to obtain pure Compound 3-1 as a colorless liquid (4.5684 g, 88% yield). LCMS: m/z = 346.02 [M+H]⁺.

Compound 3-1 (2.072 g, 6 mmol) was added to a reaction flask, and then 21 mL HCl (84 mmol, 4 M solution in 1,4-dioxane) was slowly added to the reaction flask. The resulting mixture was stirred at room temperature for 2 hours. After completion of the reaction, the solvent was removed by concentration under reduced pressure to obtain the crude product Compound 3-2 as a white solid, which was directly used in the next step. LCMS: m/z = 246.30 [M+H]⁺.

6H-pyrazolo[3,4-c]pyridine-3,6-dicarboxylic acid (1.604 g, 6 mmol) and Compound 3-2 (6 mmol) were weighed and dissolved in anhydrous DMF (50 mL), followed by addition of EDCI (1.382 g, 7.2 mmol), HOBt (973 mg, 7.2 mmol), and DIEA (3.1 mL, 18 mmol). The resulting mixture was stirred at room temperature overnight. Then the resulting mixture was quenched with water and extracted with EtOAc. The organic solution was dried over anhydrous Na₂SO₄ and concentrated. The crude product was purified by silica gel column chromatography (DCM/MeOH = 30/1, volume ratio) to obtain Compound 3-3 as a pale yellow solid (2.7094 g, 91% yield). LCMS: m/z = 495.5 [M+H]⁺.

Compound 3-3 (2.67 g, 5.4 mmol) was added to a reaction flask, and then 19 mL HCl (75.6 mmol, 4 M in 1,4-dioxane) was slowly added to the reaction flask. The resulting mixture was stirred at room temperature for 2 hours. After completion of the reaction, the solvent was removed by concentration under reduced pressure to obtain the crude product Compound 3-4 as a pale yellow solid, which was directly used in the next step. LCMS: m/z = 395.3 [M+H]⁺.

Compound 3-4 (3.1 mmol) was weighed and dissolved in anhydrous MeCN (15 mL), and Et₃N (1.29 mL, 9.3 mmol) and 1-bromo-2-methoxyethane (0.29 mL, 3.1 mmol) were added. The mixture was stirred at 80°C overnight. Then the resulting mixture was quenched with water and extracted with EtOAc. The organic solution was dried over anhydrous Na₂SO₄ and concentrated. The crude product was purified by silica gel column chromatography (DCM/MeOH = 20/1, volume ratio) to obtain pure Compound 3 as a white solid (760.2 mg, 54% yield).

### Example 3

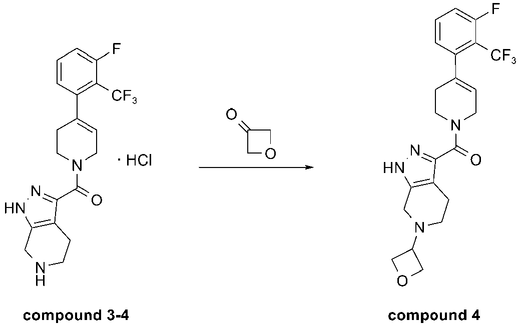

To a solution of Compound 3-4 (137 mg, 0.34 mmol) in DCM (5 mL) were added 3-oxetanone (30 µL, 0.51 mmol) and HOAc (0.12 mL, 2.04 mmol). The resulting mixture was stirred at room temperature overnight. Then sodium triacetoxyborohydride (144 mg, 0.68 mmol) was added to the reaction system, and the resulting mixture was stirred at room temperature for 24 hours. Then the resulting mixture was quenched with saturated sodium bicarbonate and extracted with DCM. The organic solution was dried over anhydrous Na₂SO₄ and concentrated. The crude product was purified by silica gel column chromatography (DCM/MeOH = 20/1, volume ratio) to obtain Compound 4 as a white solid (16 mg, 10.5% yield).

### Example 4

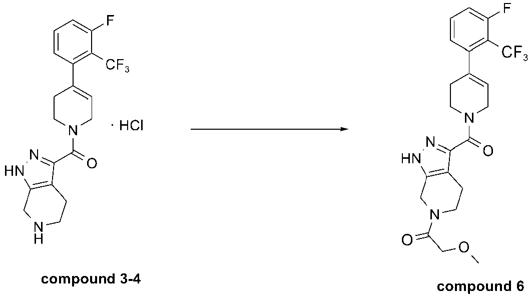

Compound 3-4 (310 mg, 0.721 mmol) was weighed and dissolved in anhydrous DCM (2 mL). Methoxyacetyl chloride (86 mg, 0.793 mmol) and Et₃N (0.12 mL, 0.865 mmol) were added to the system sequentially under ice bath. The reaction was kept at this temperature for 4 hours, quenched with water, and extracted with EtOAc. The organic solution was dried over anhydrous Na₂SO₄ and concentrated. The crude product was purified by silica gel column chromatography (DCM/MeOH = 20/1, volume ratio) to obtain pure product as a white solid (260 mg, 77% yield).

### Example 5

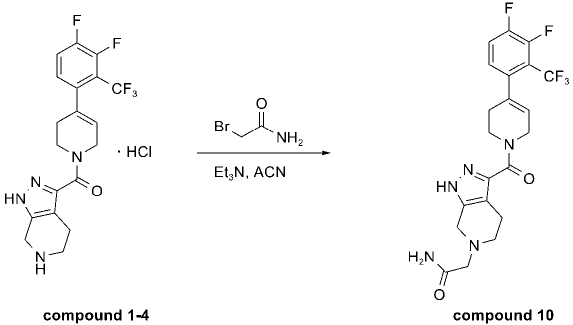

{4-[3,4-difluoro-2-(trifluoromethyl)phenyl]-1,2,3,6-tetrahydropyridin-1-yl}(4,5,6,7-tetrahydro-1H-pyrazo lo[3,4-c]pyridin-3-yl)methanone hydrochloride (Compound 1-4, 135 mg, 0.3 mmol) was weighed and dissolved in anhydrous MeCN (5 mL). Et₃N (0.13 mL, 0.9 mmol) and 2-bromoacetamide (62 mg, 0.45 mmol) were added to the system. The resulting mixture was stirred at 80°C for 2 hours. Then the resulting mixture was quenched with water and extracted with EtOAc. The organic solution was dried over anhydrous Na₂SO₄ and concentrated. The crude product was purified by silica gel column chromatography (DCM/MeOH = 10/1, volume ratio) to obtain pure Compound 10 as a white solid (110.7 mg, 78.5%).

### Example 6

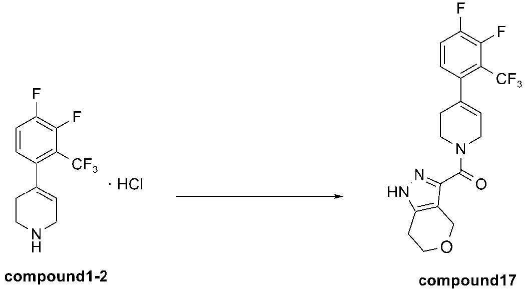

EDCI (68 g, 354.16 mmol), HOBt (47.8 g, 354.16 mmol), and DIEA (94 g, 729.15 mmol) were sequentially added to a solution of Compound 1-2 (54.8 g, 208.33 mmol) and 1,4,6,7-tetrahydropyrano[4,3-C]pyrazole-3-carboxylic acid (35 g, 208.33 mmol) in DMF (600 mL) at room temperature. The reaction system was stirred at room temperature for 12 h, and the reaction was completed as detected by LCMS. The reaction system was added to 4 L of water under ice bath, a large amount of solid precipitated, stirred for 45 minutes and filtered, and the solid was collected. The obtained solid was added to 3 L of methanol solution for pulping, filtered, the solid was collected, and pumped dry with an oil pump for 4 hours to obtain the product as a white solid (56 g, 65% yield).

### Example 7

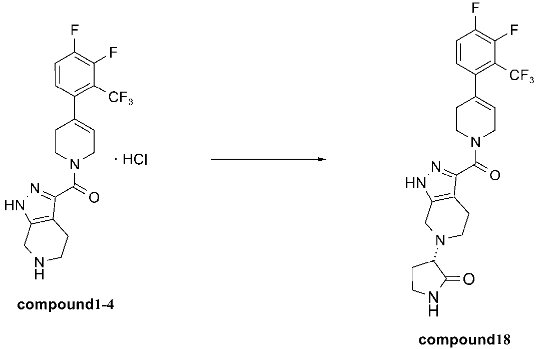

Compound **1-4** (150 mg, 0.36 mmol) and (3R)-3-[(methylsulfonyl)oxy]-2-pyrrolidinone (59.8 mg, 0.36 mmol) were dissolved in 3 mL of acetonitrile solution at room temperature, followed by dropwise addition of DIEA (129.3 mg, 1.08 mmol). The reaction system was heated to 80°C and stirred overnight. The resulting reaction mixture was cooled to room temperature, quenched with water, and extracted with EtOAc. The obtained organic phase was washed with saturated brine, dried over anhydrous sodium sulfate, and concentrated to give a residue. The residue was purified by silica gel column chromatography (eluted with V_{DCM}/V_{MeOH} = 20/1) to obtain Compound 18 as a pale yellow liquid (53 mg, 29.4% yield).

The compounds in Table 1 below were synthesized using the above steps or similar steps with corresponding intermediates or reactants.

**Table 1 Synthesized Compounds**

| **compound** | **structure** | **reactant** | **¹H NMR and MS:(M+H)⁺** |
|---|---|---|---|
| **2** | 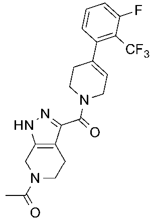 | 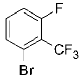 | MS: 437.5 [M+H]⁺; |
| | | | ¹HNMR (400MHz, DMSO-*d₆*, ppm) δ 12.94 (s, 1H), 7.69 (q, J = 7.2 Hz, 1H), 7.43 (dd, J = 8.4, 11.6 Hz, 1H), 7.23-7.14 (m, 1H), 5.71-5.56 (m, 1H), 4.71-4.42 (m, 3H), 4.25-4.02 (m, 2H), 3.88-3.73 (m, 1H), 3.65 (dt, J = 16.4, 5.6 Hz, 2H), 2.75-2.53 (m, 2H), 2.43-2.29 (m, 2H), 2.15-2.05 (m, 3H). |
| **3** | 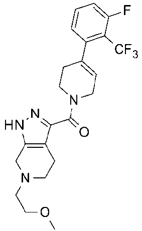 | 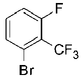 | MS: 453.7[M+H]⁺; |
| | | | ¹HNMR (400MHz, DMSO-*d₆*, ppm) δ 12.78 (s, 1H), 7.69 (q, J = 7.2 Hz, 1H), 7.43 (dd, J = 8.4, 11.6 Hz, 1H), 7.23-7.14 (m, 1H), 5.75-5.54 (m, 1H), 4.54 (s, 1H), 4.23-4.00 (m, 2H), 3.88-3.71 (m, 1H), 3.58 (s, 2H), 3.51 (t, J = 6.0 Hz, 2H), 3.26 (s, 3H), 2.69 (q, J = 5.6 Hz, 4H), 2.63-2.56 (m, 2H), 2.41-2.30 (m, 2H). |
| **4** | 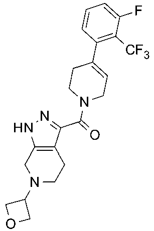 | 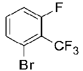 | MS: 437.5 (M+H)⁺; |
| | | | ¹H NMR (400 MHz, DMSO-d6, ppm) δ 13.11-12.94 (m, 1H), 7.68 (q, J = 7.6 Hz, 1H), 7.42 (t, J =9.6Hz, 1H), 7.23-7.15 (m, 1H), 5.71-5.58 (m, 1H), 4.80-4.63 (m, 1H), 4.61-4.49 (m, 2H), 4.25-4.02 (m, 2H), 3.89-3.77 (m, 1H), 3.73 (t, J = 6.0 Hz, 1H), 3.66 (t, J = 6.0 Hz, 1H), 2.78 (t, J = 6.0 Hz, 1H), 2.66 (t, J = 5.6 Hz, 1H), 2.45-2.30 (m, 2H), 2.14-2.03 (m, 3H). |
| **5** | 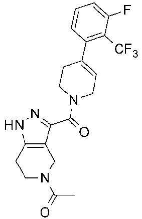 | 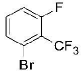 | MS: 451.6 (M+H)⁺; |
| | | | ¹H NMR (400 MHz, CDCl3, ppm) δ 7.46 (dt, J = 5.2, 8.0 Hz, 1H), 7.12 (dd, J = 8.4, 10.8 Hz, 1H), 6.98 (d, J = 7.6 Hz, 1H), 5.66-5.52 (m, 1H), 4.74 (t, J = 6.4 Hz, 2H), 4.70 (t, J = 6.4 Hz, 2H), 4.41-4.29 (m, 2H), 3.99-3.90 (m, 2H), 3.79 (quint, J = 6.4 Hz, 1H), 3.54 (s, 2H), 2.81 (t, J = 5.6 Hz, 2H), 2.62 (t, J = 5.6 Hz, 2H), 2.48-2.40 (m, 2H). |
| **6** | 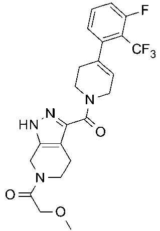 | 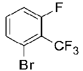 | MS: 467.35 (M+H)⁺. |
| | | | ¹H NMR (400 MHz, DMSO-*d₆*) δ 13.36 - 12.84 (m, 1H), 7.70 (td, J = 8.0, 5.5 Hz, 1H), 7.43 (dd, J = 11.6, 8.4 Hz, 1H), 7.23 - 7.15 (m, 1H), 5.72-5.57 (m, 1H), 4.66 - 4.48 (m, 3H), 4.25 - 4.02 (m, 4H), 3.85 - 3.54 (m, 3H), 3.31 -3.28 (m, 3H), 2.74 - 2.55 (m, 2H), 2.42 - 2.30 (m, 2H). |
| 7 | 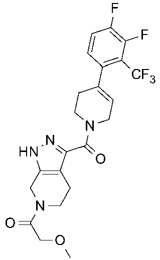 | 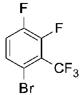 | MS: 485.34 [M+H]⁺ |
| | | | ¹H NMR (400 MHz, DMSO-*d₆*) δ 13.29 - 12.91 (m, 1H), 7.79 (q, J = 8.9 Hz, 1H), 7.28 - 7.19 (m, 1H), 5.77 - 5.59 (m, 1H), 4.66 - 4.51 (m, 3H), 4.23 - 4.02 (m, 4H), 3.84 - 3.55 (m, 3H), 3.31- 3.28 (m, 3H), 2.74 - 2.56 (m, 2H), 2.42 - 2.29 (m, 2H). |
| 8 | 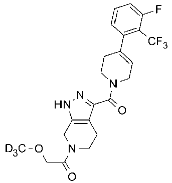 | 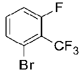 | MS: 470.31 [M+H]⁺ |
| | | | ¹H NMR (400 MHz, DMSO-*d6*, *ppm)* δ 13.31-12.86 (m, 1H), 7.70 (q, J = 7.7 Hz, 1H), 7.43 (dd, J = 11.6, 8.4 Hz, 1H), 7.25 - 7.14 (m, 1H), 5.80 - 5.54 (m, 1H), 4.68-4.48(m, 3H), 4.32 - 4.01 (m, 4H), 3.90 - 3.53 (m, 3H), 2.76-2.57 (m, 2H), 2.46 - 2.28 (m, 2H). |
| **9** | 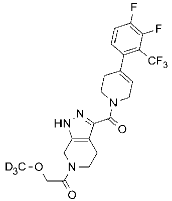 | 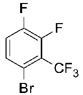 | MS: 488.15 [M+H]⁺ |
| | | | ¹H NMR (400 MHz, DMSO-*d6, ppm*) δ 13.29-12.93 (m, 1H), 7.79 (q, J = 8.9 Hz, 1H), 7.26 (d, J = 10.5 Hz, 1H), 5.74-5.57 (m, 1H), 4.67-4.49 (m, 3H), 4.29 - 4.00 (m, 4H), 3.90 - 3.49 (m, 3H), 2.74-2.56 (m, 2H), 2.44 - 2.26 (m, 2H). |
| **10** | 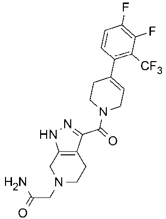 | 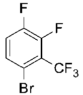 | MS: 470.28 [M+H]⁺ |
| | | | ¹H NMR (400 MHz, DMSO-*d6*, *ppm*) δ 12.81 (s, 1H), 7.80 (q, J = 8.8 Hz, 1H), 7.30-7.19 (m, 2H), 7.16-7.07 (m, 1H), 5.73-5.58 (m, 1H), 4.65-4.43 (m, 1H), 4.23-4.15 (m, 1H), 4.12-4.00 (m, 1H), 3.88-3.71 (m, 1H), 3.63 (s, 2H), 3.10 (s, 2H), 2.77-2.58 (m, 4H), 2.44-2.26 (m, 2H). |
| **11** | 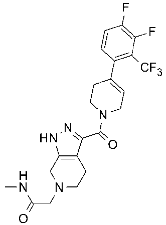 | 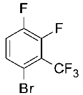 | MS: 484.67 [M+H]⁺ |
| | | | ¹H NMR (400 MHz, DMSO-*d6, ppm*) δ 12.81 (s, 1H), 7.87-7.70 (m, 2H), 7.29-7.18 (m, 1H), 5.72-5.59 (m, 1H), 4.67-4.42 (m, 1H), 4.25-4.01 (m, 2H), 3.88-3.72 (m, 1H), 3.61 (s, 2H), 3.13 (s, 2H), 2.73-2.55 (m, 7H), 2.41-2.30 (m, 2H). |
| **12** | 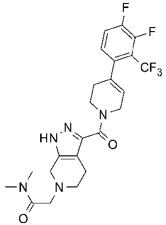 | 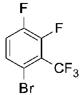 | MS: 498.32 [M+H]⁺ |
| | | | ¹H NMR (400 MHz, DMSO-*d6*, *ppm*) δ 12.79 (s, 1H), 7.78 (q, J = 8.8 Hz, 1H), 7.28-7.18 (m, 1H), 5.71-5.58 (m, 1H), 4.68-4.43 (m, 1H), 4.23-4.02 (m, 2H), 3.87-3.73 (m, 1H), 3.63 (s, 2H), 3.39 (s, 2H), 3.02 (s, 3H), 2.83 (s, 3H), 2.77-2.68 (m, 2H), 2.65-2.56 (m, 2H), 2.42-2.29 (m, 2H). |
| **13** | 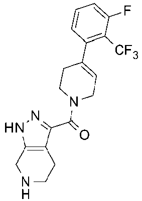 | 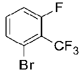 | MS: 395.63 [M+H]⁺ |
| | | | ¹H NMR (400 MHz, DMSO-*d6, ppm*) δ 12.72 (s, 1H), 7.69 (q, J = 8.0 Hz, 1H), 7.42 (dd, J = 8.4, 11.6 Hz, 1H), 7.19 (d, J = 7.6 Hz, 1H), 5.72-5.53 (m, 1H), 4.67-4.29 (m, 1H), 4.24-4.13 (m, 1H), 4.10-3.92 (m, 1H), 3.89-3.67 (m, 3H), 2.83 (t, J = 5.6 Hz, 2H), 2.54-2.50 (m, 2H), 2.40-2.30 (m, 2H). |
| **14** | 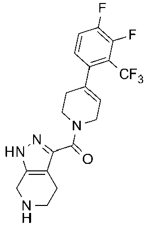 | 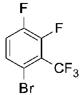 | MS: 413.36 [M+H]⁺ |
| | | | ¹H NMR (400 MHz, DMSO-*d6, ppm*) δ 7.79 (q, J = 8.9 Hz, 1H), 7.24 (dd, J = 9.0, 4.8 Hz, 1H), 5.74-5.58 (m, 1H), 4.57-4.39 (m, 1H), 4.29-4.07 (m, 3H), 4.04 - 3.68 (m, 2H), 3.16 (t, J = 5.2 Hz, 2H), 2.76 (t, J = 5.9 Hz, 2H), 2.41-2.32 (m, 2H). |
| **15** | 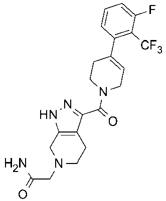 | 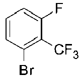 | MS: 452.21 [M+H]⁺ |
| | | | ¹H NMR (400 MHz, DMSO-*d6, ppm*) δ 12.82 (s, 1H), 7.69 (q, J = 8.0 Hz, 1H), 7.42 (dd, J = 11.6, 8.4 Hz, 1H), 7.26 (s, 1H), 7.24-7.16 (m, 1H), 7.13 (s, 1H), 5.72-5.55 (m, 1H), 4.65-4.43 (m, 1H), 4.27-4.01 (m, 2H), 3.86-3.69 (m, 1H), 3.67-3.54 (m, 2H), 3.09 (s, 2H), 2.78-2.56 (m, 4H), 2.44-2.26 (m, 2H). |
| **16** | 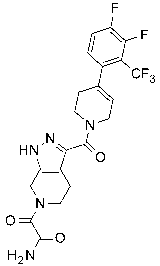 | 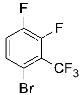 | MS: 484.4 [M+H]⁺ |
| | | | ¹H NMR (400 MHz, DMSO-*d6, ppm*) δ 13.46 - 12.75 (m, 1H), 8.23 - 8.05 (m, 1H), 7.79 (dd, J = 17.4, 9.0 Hz, 2H), 7.31 - 7.17 (m, 1H), 5.74-5.58(m, 1H), 4.87 - 4.39 (m, 3H), 4.23-4.02 (m, 2H), 3.88 - 3.53 (m, 3H), 2.76-2.61 (m, 2H), 2.42-2.29 (m, 2H). |
| **17** | 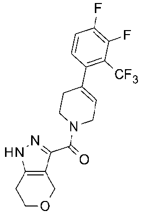 | 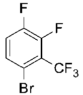 | MS: 414.68 [M+H]⁺ |
| | | | ¹H NMR (400 MHz, DMSO-*d6, ppm*) δ 13.03 (s, 1H), 7.79 (q, J = 8.9 Hz, 1H), 7.24 (d, J = 7.2 Hz, 1H), 5.67 (s, 1H), 4.81-4.61 (m, 3H), 4.33-4.12 (m, 2H), 3.81 (t, J = 5.5 Hz, 3H), 2.72 (t, J = 5.5 Hz, 2H), 2.46 - 2.24 (m, 2H). |
| **18** | 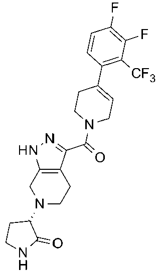 | 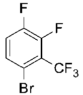 | MS: 496.17 [M+H]⁺ |
| | | | ¹H NMR (400 MHz, DMSO-*d6*, *ppm*) δ 12.79 (s, 1H), 8.51 (s, 1H), 7.79 (d, J = 8.2 Hz, 2H), 7.22 (d, J = 15.4 Hz, 1H), 5.66 (d, J = 19.5 Hz, 1H), 4.63 - 4.43 (m, 1H), 4.27 - 4.02 (m, 2H), 3.94 (d, J = 14.4 Hz, 1H), 3.80 (t, J = 17.9 Hz, 1H), 3.65 (d, J = 15.0 Hz, 2H), 3.48 (t, J = 8.8 Hz, 1H), 3.19 - 3.09 (m, 2H), 3.01 - 2.86 (m, 1H), 2.72 - 2.54 (m, 3H), 2.23 - 2.13 (m, 1H), 2.08 - 1.94 (m, 1H). |
| **19** | 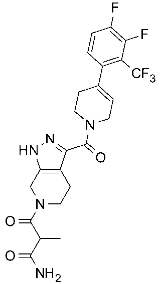 | 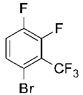 | MS: 512.16 [M+H]⁺ |
| | | | ¹H NMR (400 MHz, DMSO-*d6, ppm*) δ 13.31-12.89 (m, 1H), 7.79 (q, J = 8.9 Hz, 1H), 7.50-7.36 (m, 1H), 7.26 (d, J = 12.0 Hz, 1H), 7.13 - 6.96 (m, 1H), 5.73-5.58 (m, 1H), 4.78 - 4.48 (m, 3H), 4.29 - 3.99 (m, 2H), 3.90 - 3.50 (m, 4H), 2.78 - 2.55 (m, 2H), 2.43 - 2.26 (m, 2H), 1.22 (d, J = 7.0 Hz, 3H). |
| **20** | 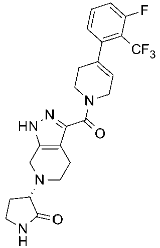 | 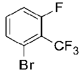 | MS: 478.46 [M+H]⁺ |
| | | | ¹H NMR (400 MHz, DMSO-*d6, ppm*) δ 13.05-12.67 (m, 1H), 7.81 (d, J = 2.2 Hz, 1H), 7.70 (td, J = 8.0, 5.5 Hz, 1H), 7.43 (dd, J = 11.6, 8.4 Hz, 1H), 7.20 (d, J = 7.6 Hz, 1H), 5.70-5.54 (m, 1H), 4.61-4.44 (m, 1H), 4.24-4.01 (m, 2H), 3.98-3.91 (m, 1H), 3.87 - 3.75 (m, 1H), 3.68-3.61 (m, 1H), 3.48 (t, J = 8.8 Hz, 1H), 3.27 - 3.08 (m, 2H), 3.03 - 2.90 (m, 1H), 2.71-2.55 (m, 2H), 2.41-2.29 (m, 2H), 2.22-2.13 (m, 1H), 2.08-1.96 (m, 1H). |
| **21** | 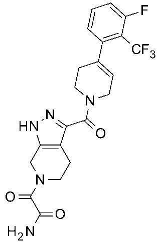 | 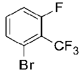 | MS: 466.41 [M+H]⁺ |
| | | | ¹H NMR (400 MHz, DMSO-d6, *ppm)* δ 13.09-12.75 (m, 1H), 7.79 (q, J = 8.9 Hz, 1H), 7.37 - 7.03 (m, 3H), 5.73-5.58 (m, 1H), 4.60-4.49 (m, 1H), 4.24-4.12 (m, 1H), 3.86-3.75 (m, 1H), 3.67-3.59 (m, 2H), 3.13-3.05 (m, 2H), 2.82 - 2.53 (m, 4H), 2.45 - 2.22 (m, 2H). |
| **22** | 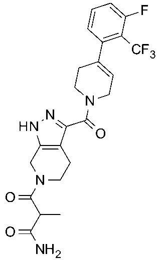 | 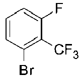 | MS: 494.27 [M+H]⁺ |
| | | | ¹H NMR (400 MHz, DMSO-*d6, ppm*) δ 13.36-12.85 (m, 1H), 7.70 (q, J = 7.3 Hz, 1H), 7.55 - 7.36 (m, 2H), 7.22-7.14 (m, 1H), 7.09-7.01 (m, 1H), 5.72-5.58 (m, 1H), 4.81 - 4.48 (m, 2H), 4.27 - 4.01 (m, 2H), 3.96 - 3.48 (m, 4H), 2.74-2.55 (m, 2H), 2.42-2.31 (m, 2H), 1.31 - 1.13 (m, 3H). |
| **23** | 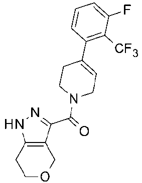 | 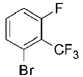 | MS: 396.13 [M+H]⁺ |
| | | | ¹H NMR (400 MHz, DMSO-*d6, ppm*) δ 13.03 (s, 1H), 7.69 (td, J = 8.0, 5.5 Hz, 1H), 7.43 (dd, J = 11.7, 8.4 Hz, 1H), 7.20 (d, J = 7.6 Hz, 1H), 5.71-5.62 (m, 1H), 4.82-4.64 (m, 3H), 4.35-4.13 (m, 2H), 3.81 (t, J = 5.5 Hz, 3H), 2.72 (t, J = 5.5 Hz, 2H), 2.44-2.29 (m, 2H). |

### Reference Compound

The following compound was synthesized with reference to Example 19 of CN111393434B as a reference: 1-(3-(4-(3,4-difluoro-2-(trifluoromethyl)phenyl)piperidine-1-carbonyl)-4,5-dihydro-1H-pyrazolo[3,4-c]pyridin -6(7H)-yl)ethanone.

### 1. Plasma RBP4 Content Detection Test

Test Method: ICR male mice aged 7 to 8 weeks were adaptively fed for 3 to 7 days and randomly divided into groups (4 mice per group), and fasted for 12 hours before the test. Orbital venous blood samples were collected and plasma was separated before administration on the test day (0-hour value as the initial content). Then, test compounds at a dose of 1 mg/kg or 5 mg/kg were orally administered (vehicle: 5% DMSO + 10% cyclodextrin), and vehicle (5% DMSO + 10% cyclodextrin) was orally administered as a control group. Orbital venous blood samples were collected and plasma was separated at 5, 8, 10, 12, and 24 hours after compound administration. The RBP4 content in plasma samples was detected using Mouse RBP4 (Retinol Binding Protein 4, Plasma) ELISA Kit (immunoway, KE1574), the reduction rate was calculated, and the maximum reduction rate within 5 to 24 hours was selected. Reduction rate of RBP4 content in mouse plasma at different sampling time points = (Initial RBP4 content - RBP4 content at sampling point) / Initial RBP4 content. The maximum reduction rates of RBP4 content in mouse plasma by each compound are shown in Table 2 below.

**Table 2 Plasma RBP4 Reduction Levels of Different Compounds**

| Compound | Maximum Plasma RBP4 Reduction Rate (5 mg/kg dose) |
|---|---|
| 1 | 41% |
| 7 | 64% |
| 9 | 82% |
| 10 | 72% |
| 13 | 67% |
| 14 | 53% |
| 15 | 77% |
| 17 | 93% |
| 21 | 14% |
| 22 | 11% |

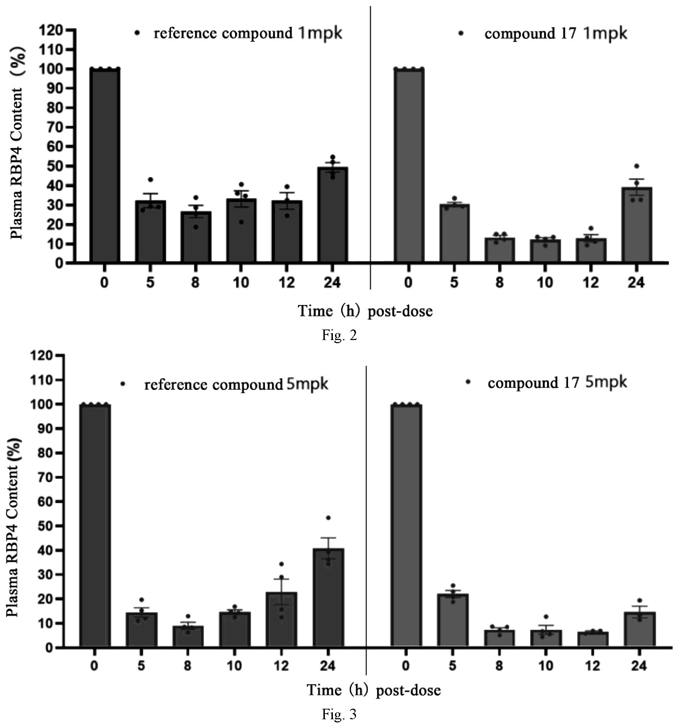

The present application also compared the RBP4 reduction rates of Compound 17 and the reference compound at different time points. The comparison results at a dose of 1 mg/kg are shown in Figure 2, and the results at a dose of 5 mg/kg are shown in Figure 3.

The results show that after a single oral dose of 1 mg/kg or 5 mg/kg, the compounds of the present application can significantly reduce plasma RBP4 content compared with the control group.

### 2. Antagonistic Activity Test

### Test Method:

Test compound solution: Each compound was used at an initial concentration of 100 µM and serially diluted to 10 different concentrations at a 3-fold gradient.

Working solution: Buffer containing 10 mM Tris-HCl pH7.5, 1 mM DTT, 0.05% NP-40, 6% glycerol.

The reaction composition included the following components: 2 µL hFc-RBP4 (MCE, HY-P72031) (62.5 nM, diluted with working solution), 1 µL Retinol (Sigma, A2056-25MG) (retinol, 5 µM, diluted with working solution), 2 µL his-TTR (SinoBiological, 12091-H08H) (10 nM, diluted with working solution), and 5 µL Anti-His-Tb (Cisbio, 61HISTLB) & Anti-IgG-XL665 (Cisbio, 61HFCXLB) working solution (antibodies diluted to 2×mixture with working solution).

Incubation reaction process: 100 nL of test compound solution was transferred to a 384-well reaction plate (two duplicate wells for each concentration), each component in the reaction composition was added sequentially, and then incubated at 25°C for 60 minutes. The HTRF signal 665/620 ratio was read using a BMG microplate reader. 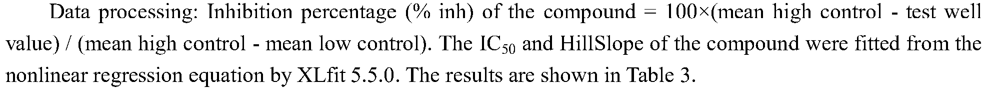 Data processing: Inhibition percentage (% inh) of the compound = 100×(mean high control - test well value) / (mean high control - mean low control). The IC50 and HillSlope of the compound were fitted from the nonlinear regression equation by XLfit 5.5.0. The results are shown in Table 3.

**Table 3 RBP4-TTR Interaction Antagonistic Activity of Different Compounds**

| **compound** | **IC₅₀ (nM)** | **HillSlope** |
|---|---|---|
| 17 | 46.769 | 1.85 |
| reference compound | 58.068 | 1.67 |

The results show that compared with the reference compound, the compounds of the present application can significantly antagonize the RBP4-TTR interaction.

### 3. Pharmacokinetic Test

Test Method: Three ICR mice (male) were used for each compound, with five groups in total. Mice were treated with a single oral dose of 10 mg/kg or 5 mg/kg. For each mouse, blood samples were collected at 0.25, 0.5, 1, 2, 4, and 8 hours after administration. Whole blood samples were placed in test tubes containing EDTA-K2, inverted up and down several times, and then centrifuged at 6000 rpm, 4°C for 15 minutes to obtain plasma. The concentration of the compound in plasma samples was determined using the LC-MS/MS method. The results are shown in Table 4.

**Table 4 Pharmacokinetic Results of Different Compounds**

| compound | Dose (mg/kg) | half-life T_{1/2}(h) | peak time Tₘₐₓ(h) | peak concentration Cₘₐₓ(ng/mL) | AUC₀₋ₜ(h×ng/mL) |
|---|---|---|---|---|---|
| 1 | 10 | 2.78 | 0.5 | 1235 | 2458 |
| 3 | 10 | 7.37 | 0.5 | 159 | 119 |
| 6 | 10 | 3.83 | 0.5 | 285 | 383 |
| 7 | 10 | 1.49 | 0.5 | 1051 | 1529 |
| 8 | 10 | 3.32 | 0.5 | 515 | 556 |
| 9 | 10 | 0.667 | 0.5 | 1091 | 1028 |
| 10 | 10 | 1.88 | 0.5 | 468 | 1200 |
| 13 | 10 | NA | 2.00 | 1203 | 6042 |
| 14 | 5 | 7.54 | 5.3 | 364 | 5996 |
| 15 | 5 | 3.08 | 0.5 | 141 | 319 |
| 16 | 5 | 1.69 | 0.5 | 235 | 490 |
| 17 | 10 | 3.35 | 1.8 | 2857 | 17764 |
| 19 | 10 | 1.98 | 0.833 | 22.3 | 41.9 |
| 20 | 10 | 1.63 | 0.5 | 112 | 208 |
| 21 | 10 | 2.54 | 0.5 | 519 | 1201 |
| 22 | 10 | NA | 0.5 | 15.2 | 13.3 |
| reference compound | 10 | 1.54 | 0.67 | 3230 | 8833 |

### 4. Liver Microsome Stability

### Test Method:

Compound working solution preparation: Prepare 10 mM DMSO stock solutions of the test substance and the reference compound verapamil, dilute to 200 µM working solution with acetonitrile before the test, and the final concentration of the test substance and verapamil is 1 µM.

Phosphate buffer preparation: Weigh 7.098 g of disodium hydrogen phosphate, add 500 mL of pure water and dissolve by ultrasonication to obtain a disodium hydrogen phosphate solution. Weigh 3.400 g of potassium dihydrogen phosphate, add 250 mL of pure water and dissolve by ultrasonication to obtain a potassium dihydrogen phosphate solution. The disodium hydrogen phosphate solution and potassium dihydrogen phosphate solution were mixed and adjusted to pH 7.4+0.2.

10 mM NADPH preparation: Weigh an appropriate amount of NADPH (reduced coenzyme II), freshly prepare a 10 mM working solution with phosphate buffer, and the final concentration is 1 mM.

Incubation system preparation: Prepare the suspension into the incubation plate according to Table 5, and pre-incubate in a 37°C water bath for 10 min.

**Table 5 Incubation System Preparation**

| components | Stock solution concentration | volume | final concentration |
|---|---|---|---|
| Liver Microsome | 20 mg/mL | 10 µL | 0.5 mg/mL |
| Phosphate buffer | 100 mM | 348µL | 100 mM |

2 µL of positive control or test compound working solution was added to 358 µL of the incubation system, mixed uniformly by vortexing, and all samples were prepared in duplicate. 40 µL of 10 mM NADPH was added to the system, mixed uniformly by low-speed vortexing, and the reaction timing was started. 50 µL of the above suspension was taken at 0.5 min, 5 min, 10 min, 15 min, 30 min, and 60 min respectively, added with 400 µL of acetonitrile (containing 100 ng/mL dexamethasone) stop solution, and mixed uniformly by vortexing. Centrifuge at 4700 rpm, 4°C for 15 minutes to precipitate proteins. Transfer 100 µL of the supernatant to the sample plate, add 100 µL of pure water and mix well for UPLC-MS/MS analysis. The results are shown in Table 6.

**Table 6 Liver Microsome Stability Results**

| compound | **pecies** | **half-life t_{1/2} (min)** | clearance rate **(µL/min/mg)** | compound | **pecies** | **half-life t_{1/2} (min)** | clearance rate **(µL/min/mg)** |
|---|---|---|---|---|---|---|---|
| Varapamil | human | 10.5 | 132 | 13 | human | >180 | < 7.44 |
| | mouse | 3.19 | 435 | | mouse | 30.2 | 45.8 |
| 1 | human | >180 | < 7.44 | 14 | human | >180 | < 7.44 |
| | mouse | 45.9 | 30.2 | | mouse | 30.0 | 46.2 |
| 2 | human | 94.4 | 14.7 | 15 | human | >180 | < 7.44 |
| | mouse | 18.8 | 73.6 | | mouse | 17.4 | 79.9 |
| 3 | human | 18.7 | 74.0 | 16 | human | >180 | < 7.44 |
| | mouse | 26.3 | 52.7 | | mouse | >180 | < 7.44 |
| 6 | human | 63.0 | 22.0 | 17 | human | >180 | < 7.44 |
| | mouse | 9.46 | 146 | | mouse | 36.4 | 38.1 |
| 7 | human | 103 | 13.4 | 18 | human | >180 | < 7.44 |
| | mouse | 15.6 | 89.0 | | mouse | 18.8 | 73.5 |
| 8 | human | 70.4 | 19.7 | 19 | human | >180 | < 7.44 |
| | mouse | 6.79 | 204 | | mouse | 74.6 | 18.6 |
| 9 | human | 130 | 10.6 | 20 | human | 99.0 | 14.0 |
| | mouse | 17.2 | 80.8 | | mouse | 13.7 | 101 |
| 10 | human | >180 | < 7.44 | 21 | human | >180 | < 7.44 |
| | mouse | 18.6 | 74.5 | | mouse | 136.5 | 10.2 |
| 11 | human | 140 | 9.90 | 22 | human | >180 | < 7.44 |
| | mouse | 11.4 | 121 | | mouse | 43.0 | 32.2 |
| 12 | human | 126 | 11.0 | 23 | human | 54.3 | 25.5 |
| | mouse | 4.20 | 330 | | mouse | 10.7 | 130 |

The results show that most compounds of the present application have good metabolic stability, especially stable metabolism in human liver microsomes. In addition, some compounds of the present application have a clearance rate of less than 10 µL/min/mg in human liver microsomes, with good druggability.

It should be understood that if any prior art publication is cited in the present application; such citation does not constitute an admission that the publication is part of the common general knowledge in the art in any country.

All publications, patents, patent applications, and published patent applications cited by reference in the present application are incorporated herein by reference in their entirety. Although the foregoing invention has been described in considerable detail by way of illustration and example for purposes of clarity of understanding, it will be obvious to those skilled in the art that certain minor changes and modifications may be practiced. Accordingly, the description and examples should not be construed as limiting the scope of the present application.

## Claims

1. A compound of Formula (I), a stereoisomer thereof, a deuterated derivative thereof, a pharmaceutically acceptable salt thereof, a pharmaceutically acceptable salt of a stereoisomer thereof, or an acceptable salt of a deuterated derivative thereof, 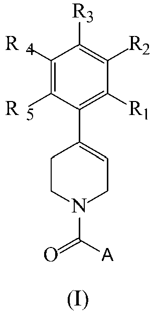
wherein, R₁, R₂, R₃, R₄ and R₅ are each independently -H, -halogen, -CF₃ or -C₁₋₄ alkyl; wherein at least two of R₁, R₂, R₃, R₄ or R₅ are not H;
A is selected from 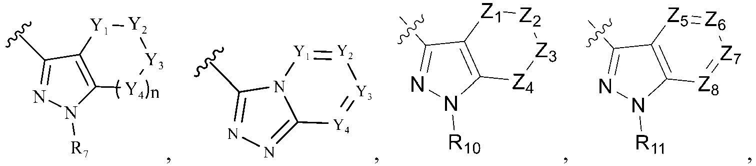 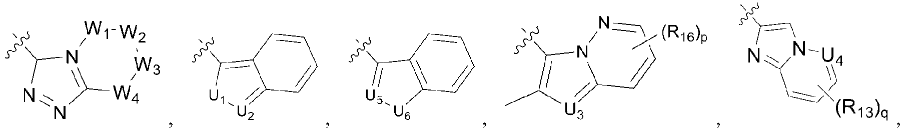 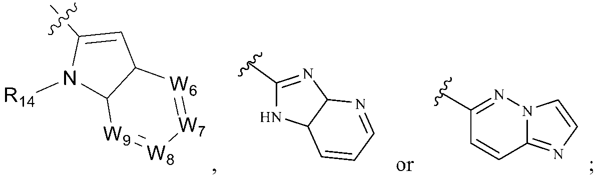
wherein R₇ is selected from -H, -C₁₋₄ alkyl or -(oxetane)yl;
when A is selected from 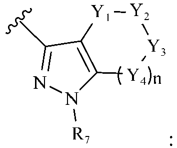
wherein n is 0, 1 or 2;
Y₁, Y₂ and Y₄ are each -CH₂-, and Y₃ is -NR₉-; or Y₁, Y₃ and Y₄ are each -CH₂-, and Y₂ is -NR₉-; or Y₁, Y₂ and Y₄ are each -CH₂-, and Y₃ is -O- or -S(=O)₂-; or Y₁, Y₃ and Y₄ are each -CH₂-, and Y₂ is -O- or -S(=O)₂-;
R₉ is selected from hydrogen, -C₁₋₆ alkyl, -C(=O)(C₁₋₆ alkyl), -S(=O)(C₁₋₆ alkyl), -S(=O)₂(C₁₋₆ alkyl), 3-7 membered carbocyclyl, 3-7 membered heterocyclyl, phenyl, 5-10 membered heteroaryl; said R₉ is optionally substituted with 1, 2, 3, 4, 5 or 6 substituents selected from halogen, -C₁₋₆ alkyl, -C₁₋₆ alkenyl, -C₁₋₆ alkynyl, -C₁₋₆ haloalkyl, -C₁₋₆ alkoxy, -CN, -NH₂, -COOH, -NH(C₁₋₆ alkyl), -N(C₁₋₆ alkyl)₂, -OH, -O(C₁₋₆ alkyl), -O(C₁₋₆ deuteroalkyl), -SH, -S(C₁₋₆ alkyl), =O, -C(=O)(C₁₋₆ alkyl), -C(=O)(C₁₋₆ alkoxy), -S(=O)(C₁₋₆ alkyl), -S(=O)₂(C₁₋₆ alkyl), -C(=O)NH₂, -C(=O)-C(=O)NH₂, -C(=O)NH(C₁₋₆ alkyl), -C(=O)N(C₁₋₆ alkyl)₂, -NHC(=O)(C₁₋₆ alkyl), -C(=O)O(C₁₋₆ alkyl), -OC(=O)(C₁₋₆ alkyl), -S(=O)NH₂, -S(=O)NH(C₁₋₆ alkyl), -S(=O)N(C₁₋₆ alkyl)₂, -NHS(=O)(C₁₋₆ alkyl), -S(=O)₂NH₂, -S(=O)₂NH(C₁₋₆ alkyl), -S(=O)₂N(C₁₋₆ alkyl)₂, -NHS(=O)₂(C₁₋₆ alkyl), -C(=O)-phenyl, -C(=O)-5-10 membered heteroaryl, -C(=O)-3-7 membered heterocyclyl, 3-7 membered carbocyclyl, 3-7 membered heterocyclyl, phenyl or 5-10 membered heteroaryl; said heterocyclyl or heteroaryl each independently includes one or more heteroatoms selected from N, O or S at each occurrence;
when A is selected from 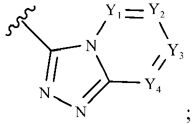
wherein Y₁, Y₂, Y₃ and Y₄ are each independently CR₈ or N, and each R₈ is independently -H, halogen, -C₁₋₆ alkyl, -C(=O)NH₂, -C(=O)NH(C₁₋₆ alkyl), -C(=O)N(C₁₋₆ alkyl)₂, -COOH, -NHC(=O)N(C₁₋₆ alkyl)₂, -S(=O)₂NH(C₁₋₆ alkyl), -O-(C₁₋₆ alkyl), -CN or -C₁₋₆ haloalkyl;
when A is selected from 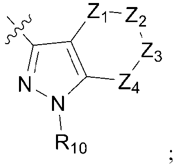
wherein Z₂ is selected from -O-, Z₃ is selected from -C(CH₃)₂-, and Z₁ and Z₄ are selected from -CH₂-; alternatively, Z₂ and Z₃ are each independently selected from -C(O)-, -N(CH₃)- or -NH-, and Z₁ and Z₄ are selected from -CH₂-;
R₁₀ is selected from hydrogen or -CH₃;
when A is selected from 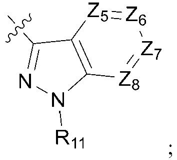
wherein Z₅, Z₆, Z₇ and Z₈ are each independently selected from CR₁₂ or N, and R₁₂ is each independently selected from hydrogen, F, Cl, -CN or -OCH₃;
R₁₁ is selected from H, -CH₃, -CH₂CH₃, 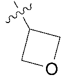 or -CH(CH₃)₂;
when A is selected from 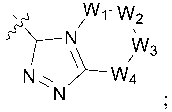
wherein, W₁, W₂, W₃ and W₄ are each independently selected from -CH₂-, -O-, -NH-, 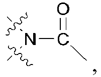 -N(CH₃)- or 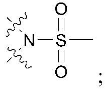
when A is selected from 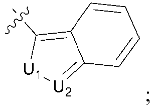
wherein U₁ is selected from O or S, and U₂ is selected from N;
when A is selected from 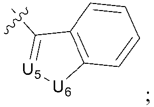
wherein U₅ is selected from N, and U₆ is selected from O or S; alternatively, U₅ is selected from CH, and U₆ is selected from NH;
when A is selected from 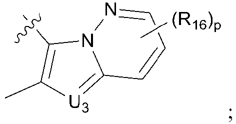
wherein U₃ is selected from N or CH;
R₁₆ is selected from Cl;
p is selected from 0 or 1;
when A is selected from 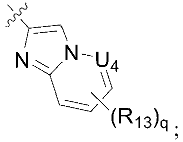
wherein U₄ is selected from N or CH;
R₁₃ is selected from -CH₃, -OCH₃, 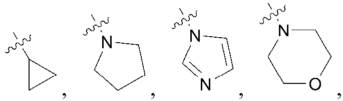 F, Cl or -CF₃;
q is selected from 0 or 1;
when A is selected from 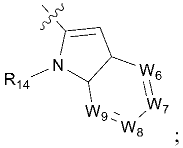
wherein W₆, W₇, W₈ and W₉ are each independently selected from N or CR₁₅;
R₁₄ is selected from hydrogen or -CH₃;
R₁₅ is each independently selected from H, F, Cl, -CH₃, -OCH₃,

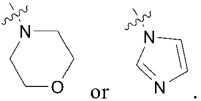

2. A compound of Formula (I), a stereoisomer thereof, a deuterated derivative thereof, a pharmaceutically acceptable salt thereof, a pharmaceutically acceptable salt of a stereoisomer thereof, or an acceptable salt of a deuterated derivative thereof, 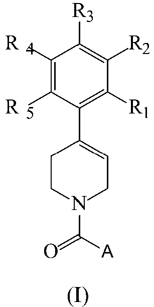
wherein, R₁, R₂, R₃, R₄ and R₅ are each independently -H, -halogen, -CF₃ or -C₁₋₄ alkyl; wherein at least two of R₁, R₂, R₃, R₄ or R₅ are not H;
A is selected from 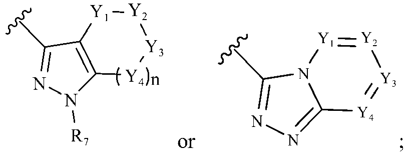
wherein R₇ is selected from -H, -C₁₋₄ alkyl or -(oxetane)yl;
when A is selected from 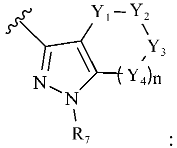
wherein n is 0, 1 or 2;
Y₁, Y₂ and Y₄ are each -CH₂-, and Y₃ is -NR₉-; or Y₁, Y₃ and Y₄ are each -CH₂-, and Y₂ is -NR₉-;
R₉ is selected from hydrogen, -C₁₋₆ alkyl, -C(=O)(C₁₋₆ alkyl), -S(=O)(C₁₋₆ alkyl), -S(=O)₂(C₁₋₆ alkyl), 3-7 membered carbocyclyl, 3-7 membered heterocyclyl, phenyl, 5-10 membered heteroaryl; said R₉ is optionally substituted with 1, 2, 3, 4, 5 or 6 substituents selected from -C₁₋₆ alkyl, -C₁₋₆ alkenyl, -C₁₋₆ alkynyl, -C₁₋₆ haloalkyl, -C₁₋₆ alkoxy, -CN, -NH₂, -COOH, -NH(C₁₋₆ alkyl), -N(C₁₋₆ alkyl)₂, -OH, -O(C₁₋₆ alkyl), -SH, -S(C₁₋₆ alkyl), -C(=O)(C₁₋₆ alkyl), -S(=O)(C₁₋₆ alkyl), -S(=O)₂(C₁₋₆ alkyl), -C(=O)NH₂, -C(=O)NH(C₁₋₆ alkyl), -C(=O)N(C₁₋₆ alkyl)₂, -NHC(=O)(C₁₋₆ alkyl), -C(=O)O(C₁₋₆ alkyl), -OC(=O)(C₁₋₆ alkyl), -S(=O)NH₂, -S(=O)NH(C₁₋₆ alkyl), -S(=O)N(C₁₋₆ alkyl)₂, -NHS(=O)(C₁₋₆ alkyl), -S(=O)₂NH₂, -S(=O)₂NH(C₁₋₆ alkyl), -S(=O)₂N(C₁₋₆ alkyl)₂, -NHS(=O)₂(C₁₋₆ alkyl), 3-7 membered carbocyclyl, 3-7 membered heterocyclyl, phenyl or 5-10 membered heteroaryl; said heterocyclyl or heteroaryl each independently includes one or more heteroatoms selected from N, O or S at each occurrence;
when A is selected from 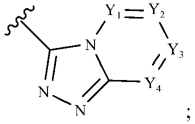
wherein Y₁, Y₂, Y₃ and Y₄ are each independently CR₈ or N, and each R₈ is independently -H, halogen, -O-(C₁₋₆ alkyl), -CN or -C₁₋₆ haloalkyl.

3. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to any one of claims 1-2, wherein, R₁, R₂, R₃, R₄ and R₅ are each independently -H, -F, -Cl, -Br or -CF₃.

4. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to any one of claims 1-3, wherein,
R₁ is -CF₃, R₂ is -F, R₃ is -F, R₄ is -H and R₅ is -H, or
R₁ is -CF₃, R₂ is -F, R₃ is -H, R₄ is -H and R₅ is -H, or
R₁ is -CF₃, R₂ is -F, R₃ is -H, R₄ is -F and R₅ is -H, or
R₁ is -CF₃, R₂ is -H, R₃ is -F, R₄ is -F and R₅ is -H, or
R₁ is -CF₃, R₂ is -H, R₃ is -H, R₄ is -H and R₅ is -F, or
R₁ is -CF₃, R₂ is -H, R₃ is -F, R₄ is -H and R₅ is -H, or
R₁ is -CF₃, R₂ is -H, R₃ is -H, R₄ is -Cl and R₅ is -H, or
R₁ is -CF₃, R₂ is -Cl, R₃ is -H, R₄ is -H and R₅ is -H, or
R₁ is -H, R₂ is -CF₃, R₃ is -H, R₄ is -CF₃ and R₅ is -H, or
R₁ is -Cl, R₂ is -H, R₃ is -H, R₄ is -F and R₅ is -H, or
R₁ is -Cl, R₂ is -F, R₃ is -H, R₄ is -H and R₅ is -H, or
R₁ is -CF₃, R₂ is -H, R₃ is -H, R₄ is -F and R₅ is -H.

5. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to any one of claims 1-4, wherein,
R₁ is -CF₃, R₂ is -F, R₃ is -F, R₄ is -H and R₅ is -H, or
R₁ is -CF₃, R₂ is -F, R₃ is -H, R₄ is -H and R₅ is -H.

6. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to any one of claims 1-4, wherein the compound of Formula (I) is any one of the following formulas:

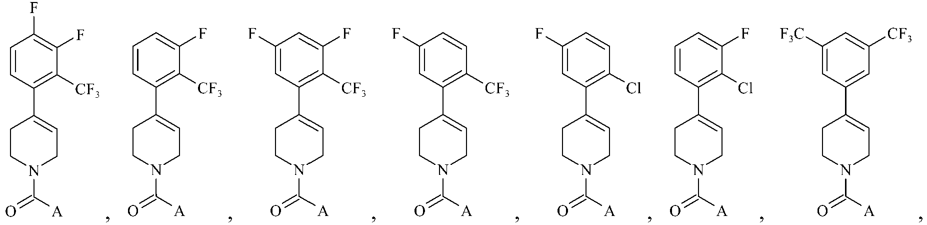

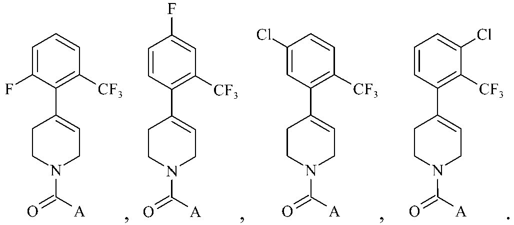

7. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to any one of claims 1-6,
A is 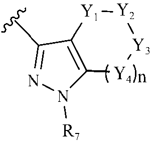
wherein, n is 0 or 1;
when n is 0, Y₁ and Y₃ are each -CH₂-, and Y₂ is -NR₉-;
when n is 1, Y₁, Y₂ and Y₄ are each -CH₂-, and Y₃ is -NR₉-; or Y₁, Y₃ and Y₄ are each -CH₂-, and Y₂ is -NR₉-;
R₇ is -H, -C₁₋₄ alkyl or -(oxetane)yl;
said R₉ is -H, -CN, -C₁₋₆ alkyl, -C₁₋₆ haloalkyl, -C₃₋₆ cycloalkyl, -(C₁₋₄ alkylene)-(C₃₋₆ cycloalkyl), -C₃₋₆ heterocyclyl, -(C₁₋₄ alkylene)-(C₃₋₆ heterocyclyl), -(C₁₋₆ alkylene)-O(C₁₋₃ alkyl), -(C₁₋₆ alkylene)-CF₃, -C(O)-(C₁₋₆ alkyl), -C(O)₂-(C₁₋₆ alkyl), -C(O)-NH₂, -CH₂C(O)-NH₂, -C(O)-C(O)-NH₂, -C(O)-CH(C₁₋₆ alkylene)-C(O)-NH₂, -CH₂C(O)-NH(C₁₋₃ alkyl), -CH₂C(O)-N(C₁₋₃ alkyl)₂, -C(O)-NH(C₁₋₃ alkyl), -C(O)-N(C₁₋₃ alkyl)₂, -C(O)NH-(C₁₋₆ alkyl), -C(O)-(C₁₋₆ haloalkyl), -C(O)-(C₁₋₆ alkoxy), -C(O)-(C₁₋₆ alkylene)-(OC₁₋₆ alkyl), -C(O)-(C6 aryl), -C(O)-(C6 heteroaryl), -C(O)-(C6 heterocyclyl), -(C₁₋₆ alkylene)-CO₂H, -(C₁₋₆ alkylene)-CO₂(C₁₋₆ alkyl), -SO₂-(C₁₋₆ alkyl), oxetanyl or

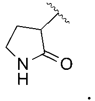

8. A compound of Formula (I), a stereoisomer thereof, a deuterated derivative thereof, a pharmaceutically acceptable salt thereof, a pharmaceutically acceptable salt of a stereoisomer thereof, or an acceptable salt of a deuterated derivative thereof, 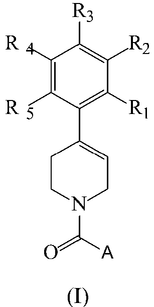
wherein, R₁, R₂, R₃, R₄ and R₅ are each independently -H, -halogen, -CF₃ or -C₁₋₄ alkyl; wherein at least two of R₁, R₂, R₃, R₄ or R₅ are not H; A is selected from 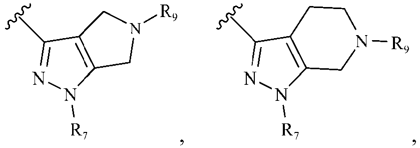 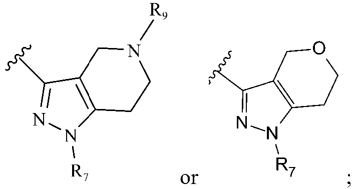
R₇ and R₉ are as defined in claim 1 or 2.

9. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to claim 8, wherein,
R₁, R₂, R₃, R₄ and R₅ are each independently -H, -F, -Cl, -Br or -CF₃.

10. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to claim 8 or 9, wherein,
R₁ is -CF₃, R₂ is -F, R₃ is -F, R₄ is -H and R₅ is -H, or
R₁ is -CF₃, R₂ is -F, R₃ is -H, R₄ is -H and R₅ is -H, or
R₁ is -CF₃, R₂ is -F, R₃ is -H, R₄ is -F and R₅ is -H, or
R₁ is -CF₃, R₂ is -H, R₃ is -F, R₄ is -F and R₅ is -H, or
R₁ is -CF₃, R₂ is -H, R₃ is -H, R₄ is -H and R₅ is -F, or
R₁ is -CF₃, R₂ is -H, R₃ is -F, R₄ is -H and R₅ is -H, or
R₁ is -CF₃, R₂ is -H, R₃ is -H, R₄ is -Cl and R₅ is -H, or
R₁ is -CF₃, R₂ is -Cl, R₃ is -H, R₄ is -H and R₅ is -H, or
R₁ is -H, R₂ is -CF₃, R₃ is -H, R₄ is -CF₃ and R₅ is -H, or
R₁ is -Cl, R₂ is -H, R₃ is -H, R₄ is -F and R₅ is -H, or
R₁ is -Cl, R₂ is -F, R₃ is -H, R₄ is -H and R₅ is -H.

11. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to any one of claims 1-10, wherein,
R₉ is -H, -CN, -CH₃, -CH₂CH₃, -CH₂CH₂CH₃, -CH(CH₃)₂, -CH₂CH(CH₃)₂, -C(CH₃)₃, -CH₂CH(CH₃)₂, -CH₂C(CH₃)₃, -CH₂CF₃, -CH₂CH₂CF₃, -CH₂OCH₃, -CH₂CH₂OCH₃, 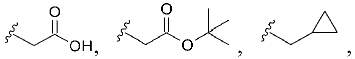 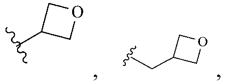 -SO₂-CH₃, -C(O)-CH₃, -C(O)-CH₂CH₃, -C(O)-CH₂CH₂CH₃, -C(O)-CH(CH₃)₂, -C(O)-CH₂CH(CH₃)₂, -C(O)-C(CH₃)₃, -C(O)-OCH₃, -C(O)-NHCH₃, 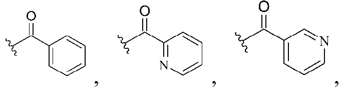 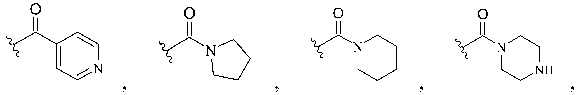 -C(O)-CH₂OCH₃, -C(O)-CH₂OCH₂CH₃, -C(O)-CH₂OCH(CH₃)₂, -C(O)-CH₂CH₂OCH₃, -C(O)-CH₂CH₂OCH₂CH₃, -C(O)-CH₂CH₂OCH(CH₃)₂, -C(O)-CH₂CF₃, -C(O)-CH₂Cl, -C(O)-CH₂F, -C(O)-CH₂CH₂OCH₃ , -C(O)-CH₂CH₂CF₃, -C(O)-CH₂CH₂Cl, -C(O)-CH₂CH₂F,

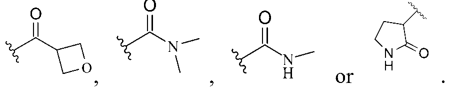

12. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to claim 1 or 2,
A is 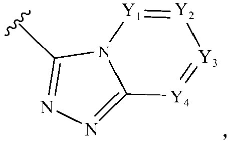
wherein, wherein, Y₁, Y₂, Y₃ and Y₄ are each independently CR₈ or N,
R₈ is each independently -H, halogen, -OCH₃, -CN or -CF₃ at each occurrence.

13. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to claim 12, wherein,
A is

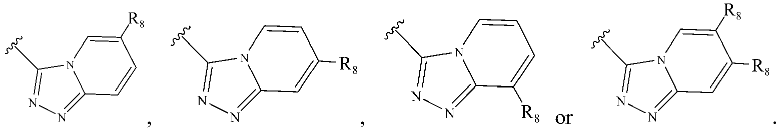

14. The compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof according to claim 1 or 2, wherein,
A is

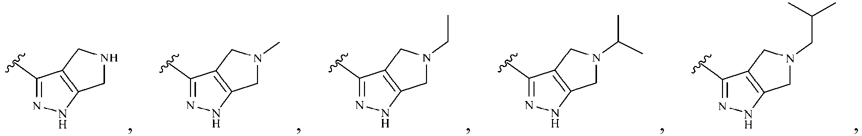

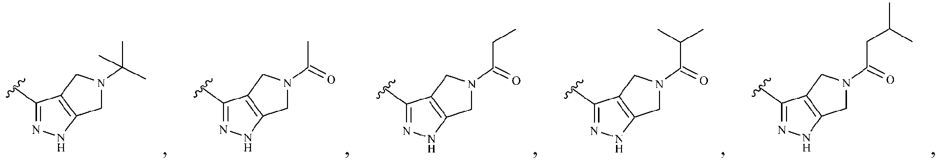

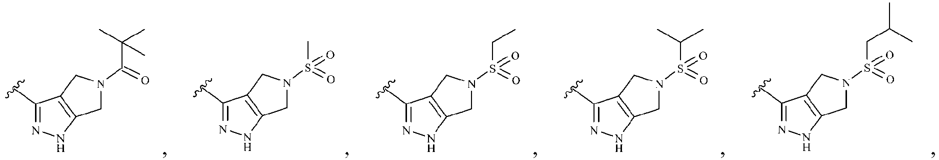

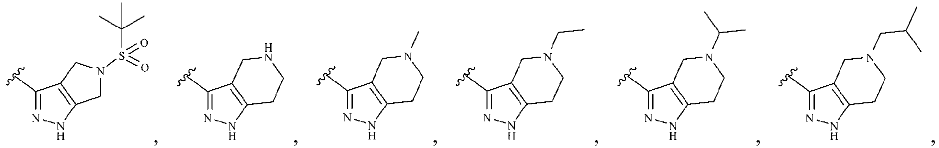

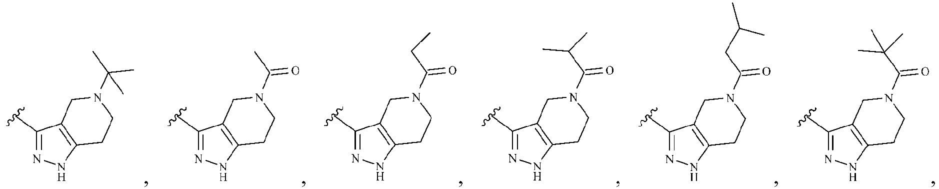

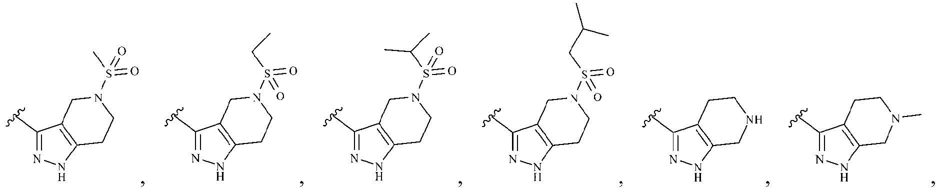

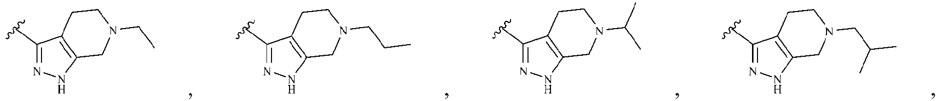

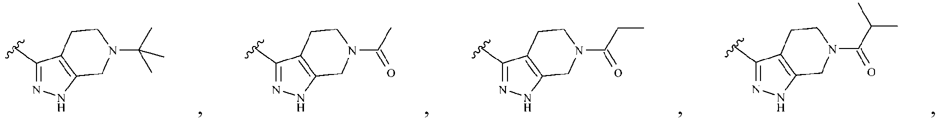

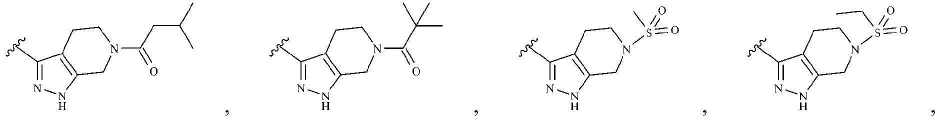

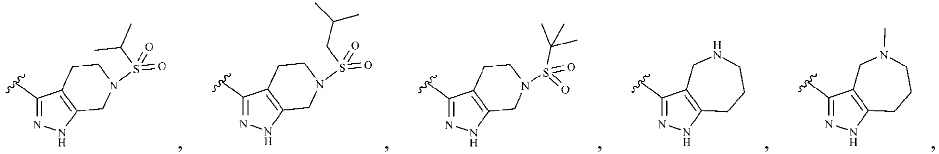

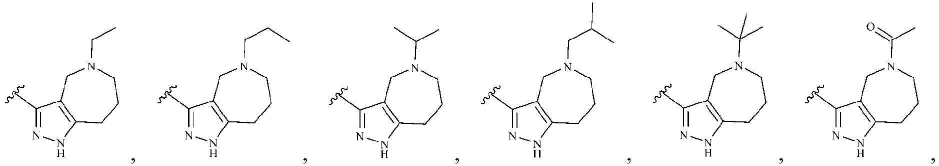

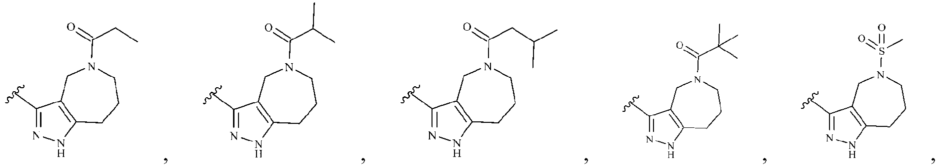

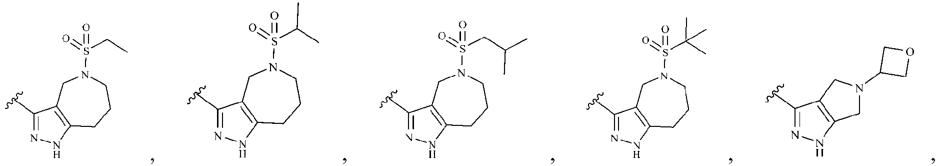

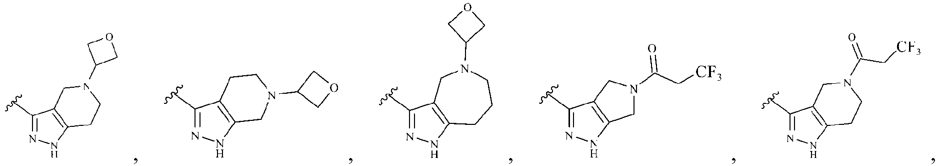

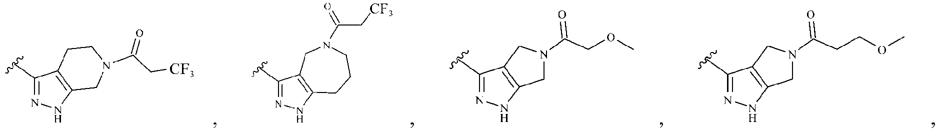

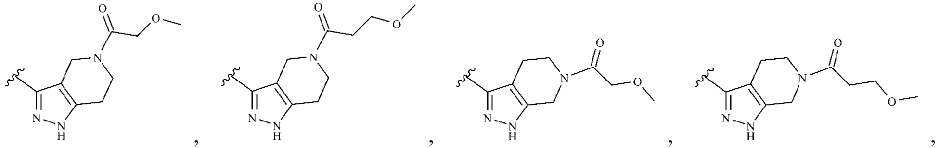

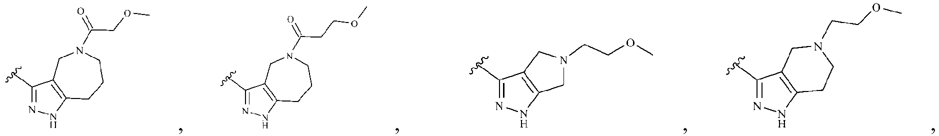

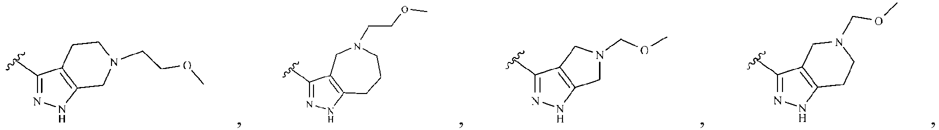

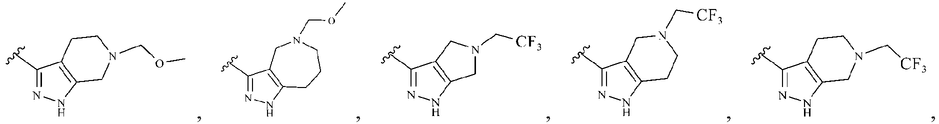

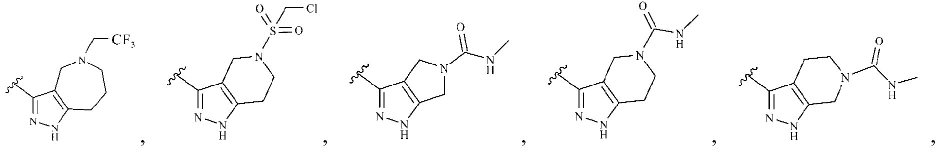

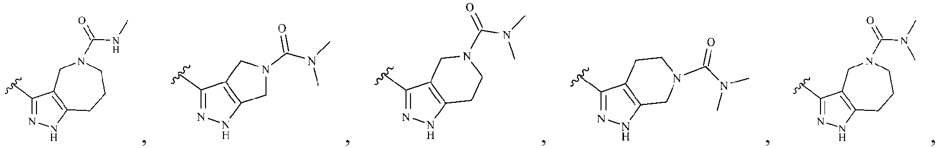

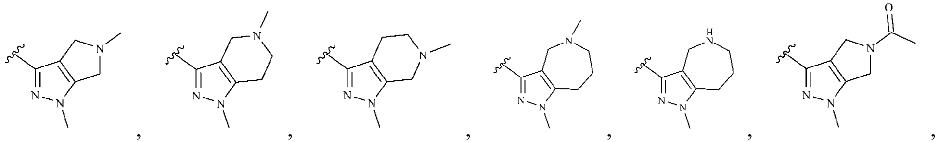

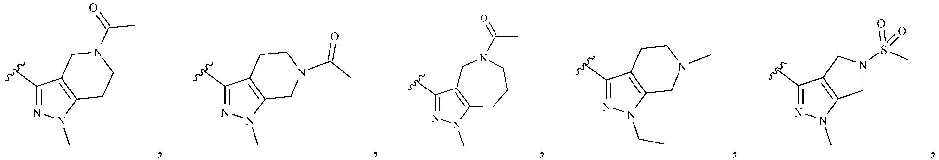

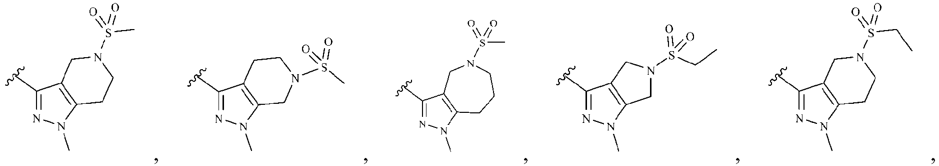

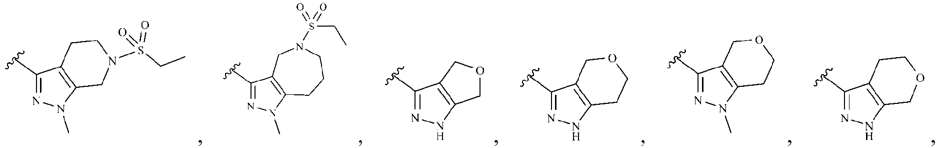

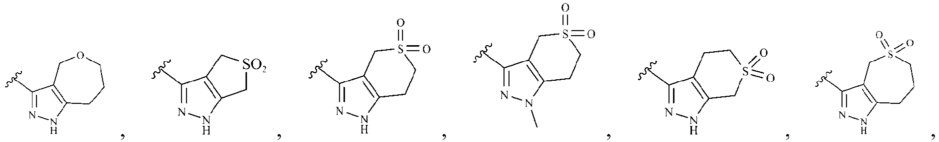

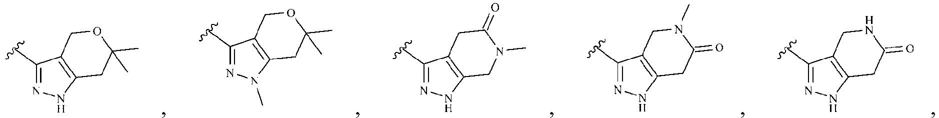

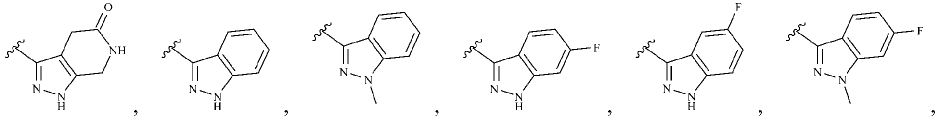

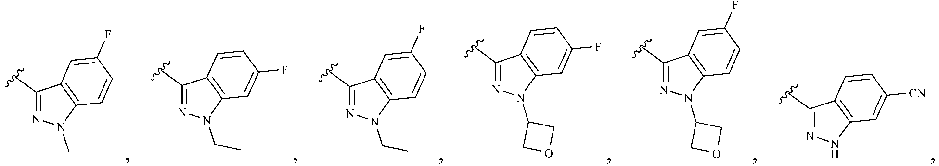

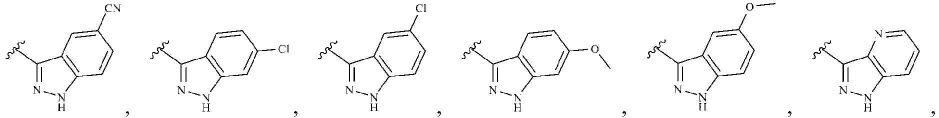

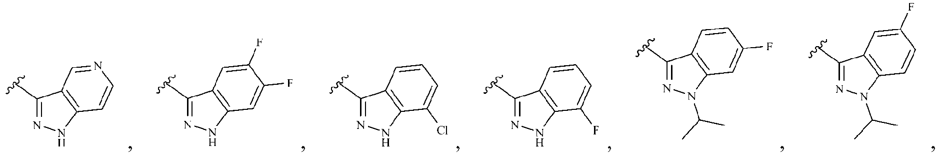

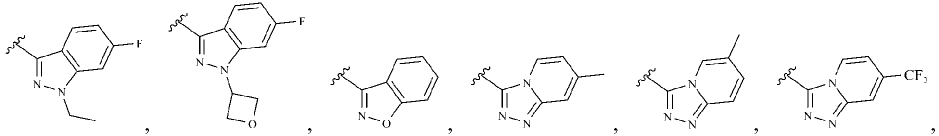

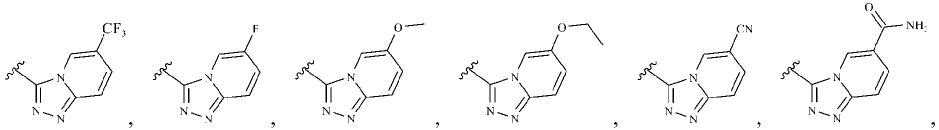

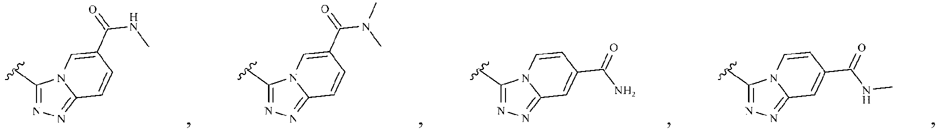

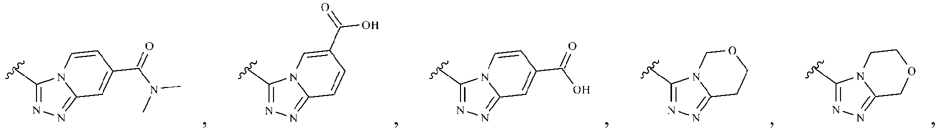

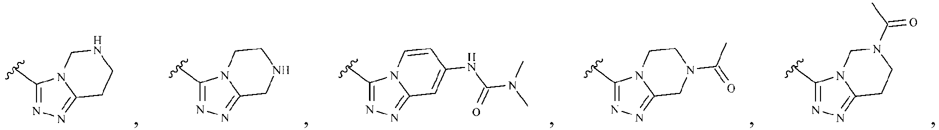

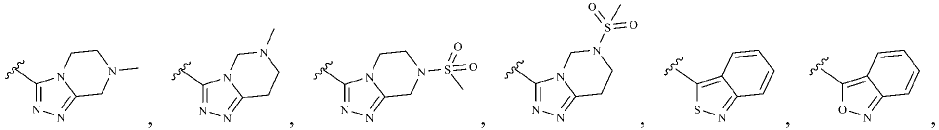

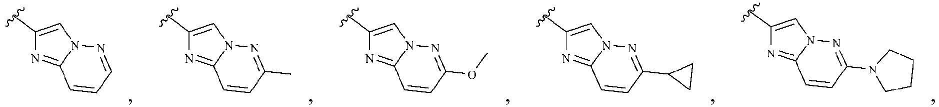

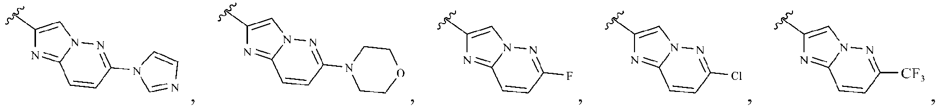

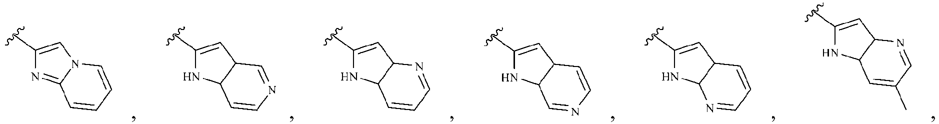

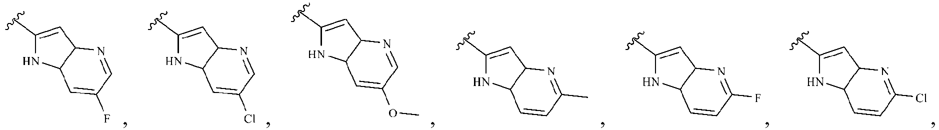

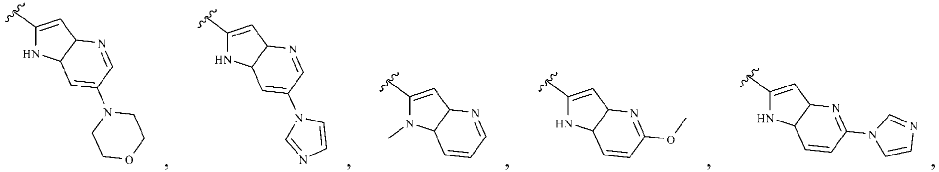

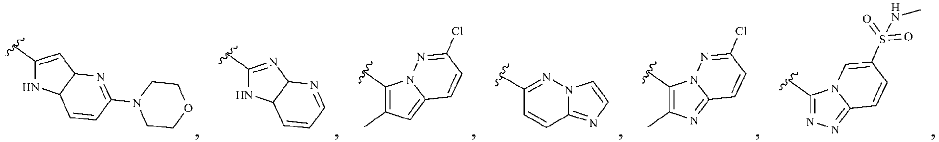

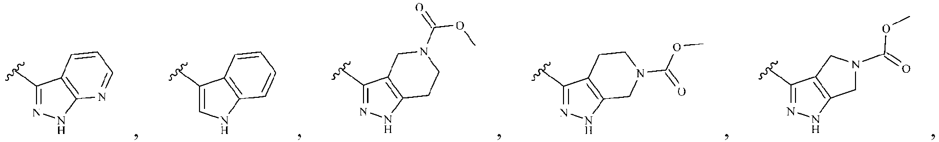

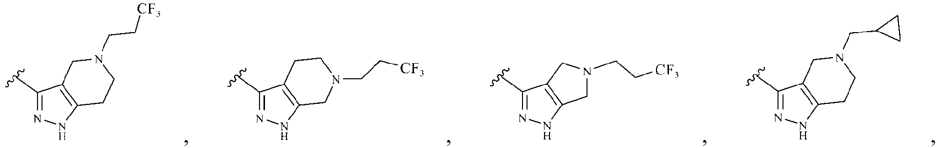

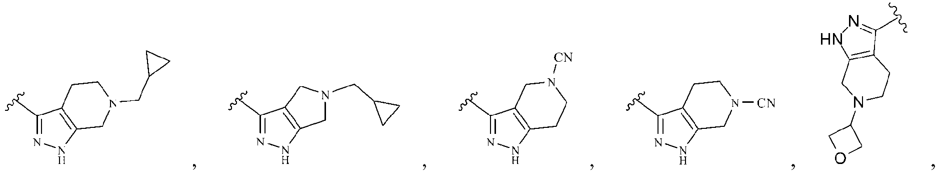

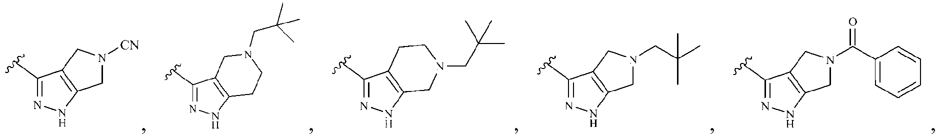

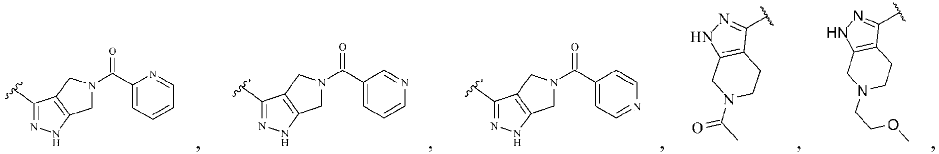

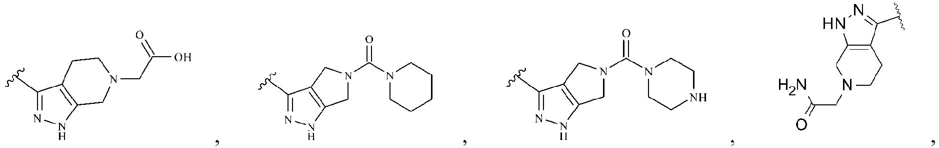

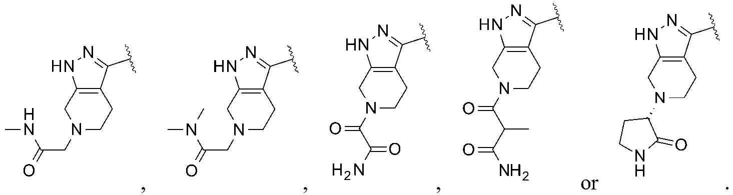

15. A compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof, wherein the compound is any one of the following formulas: 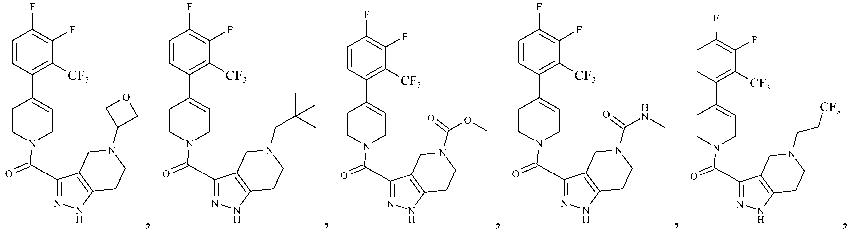 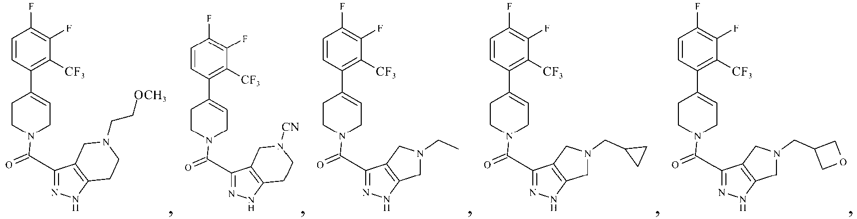 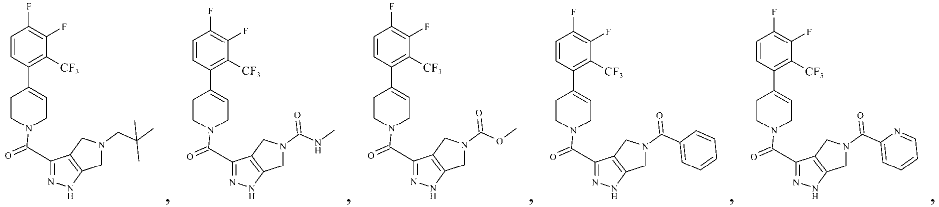 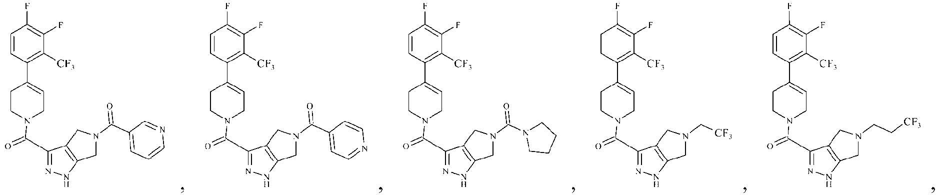 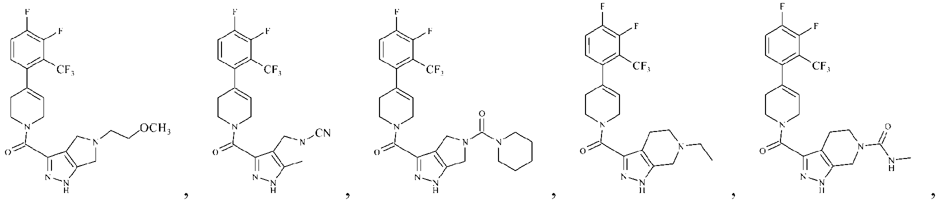 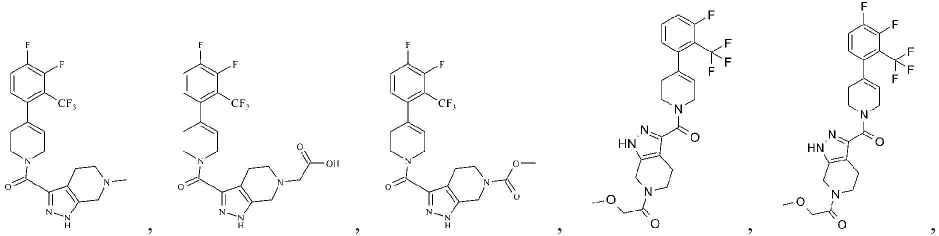 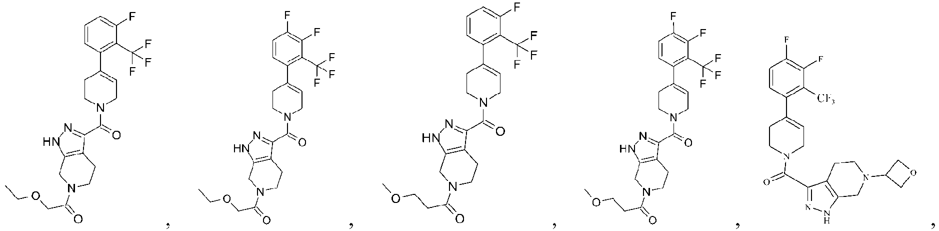 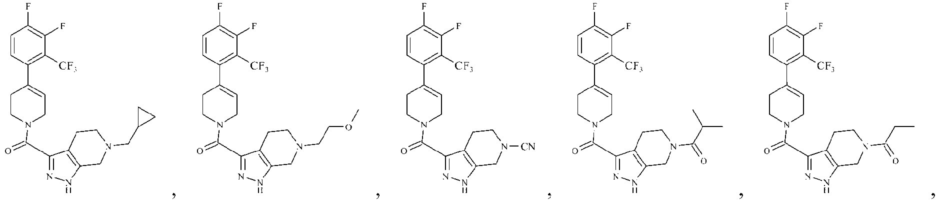 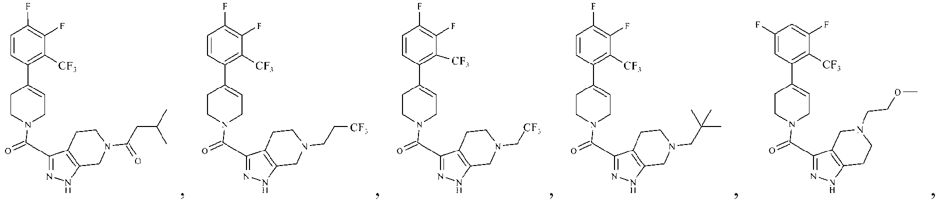 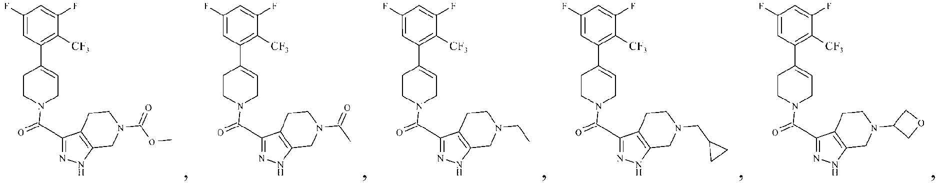 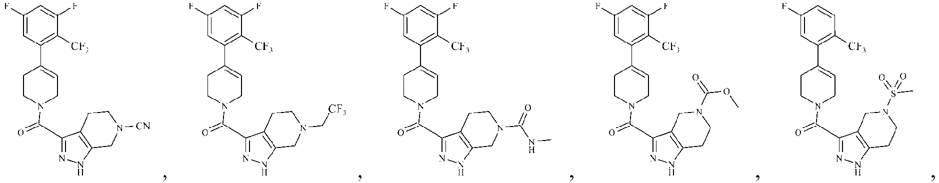 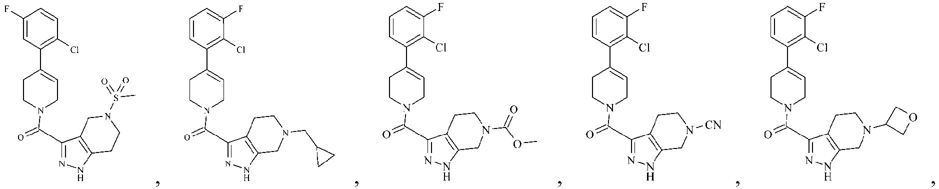 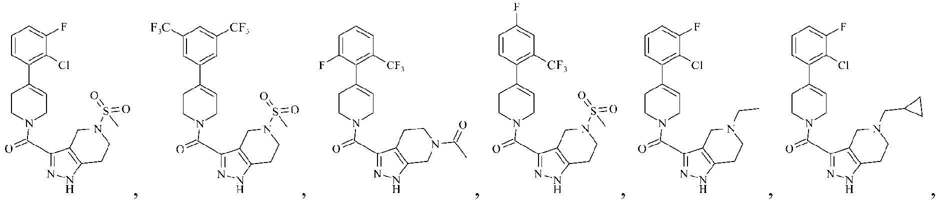 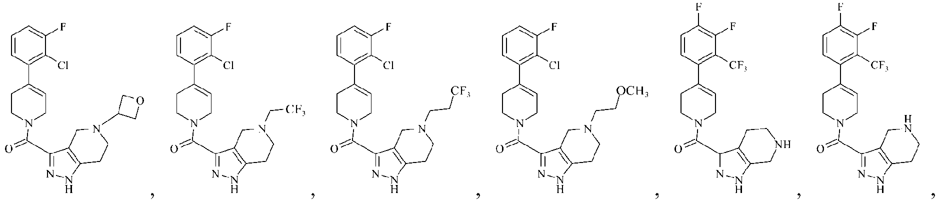 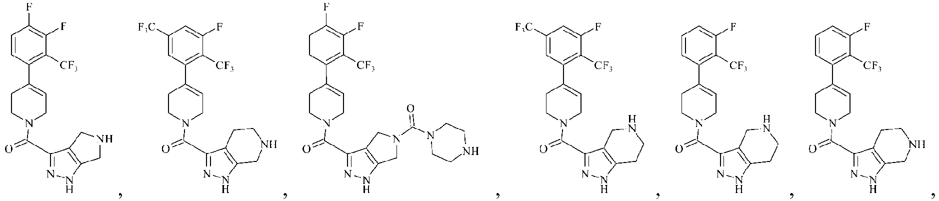 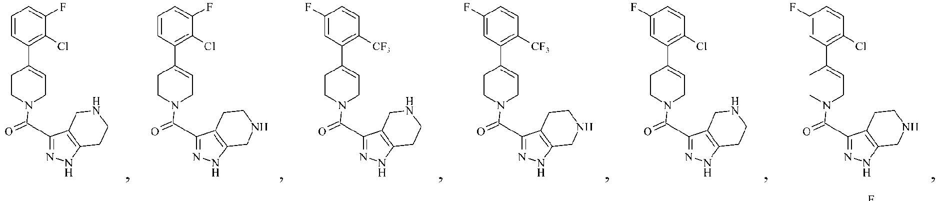 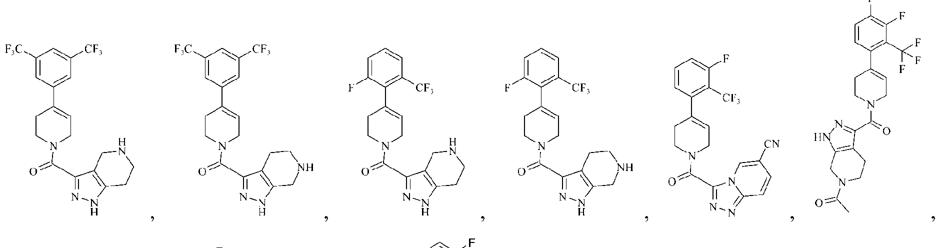 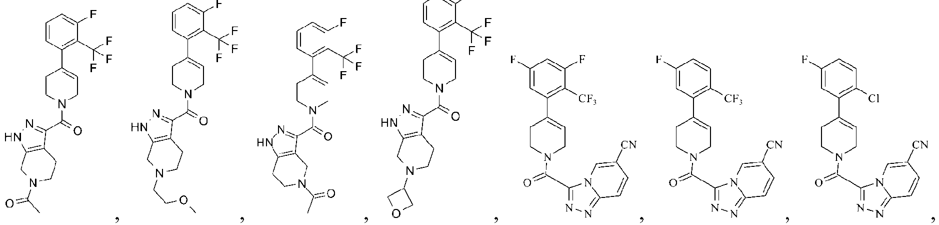 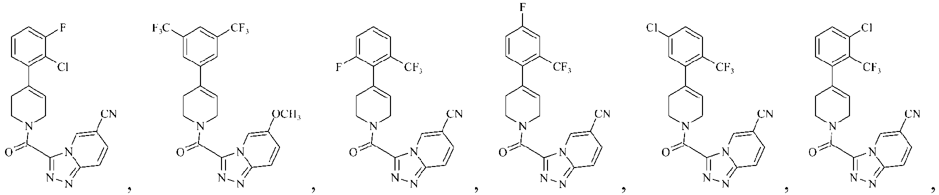 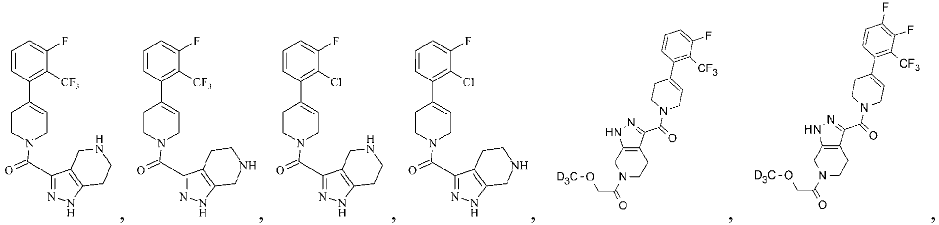 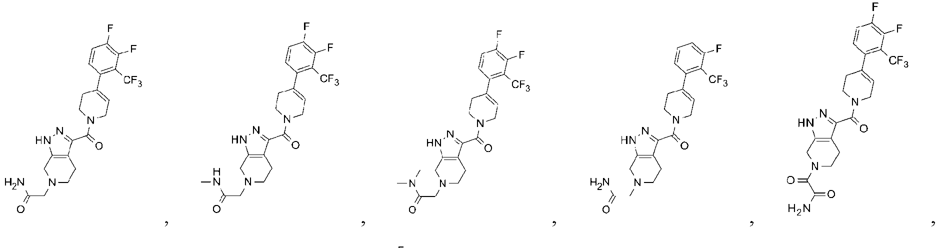 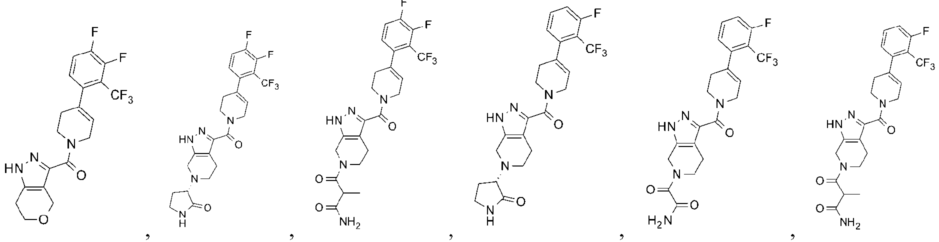 or

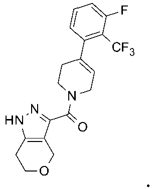

16. A compound, a stereoisomer thereof, a deuterated derivative thereof, a pharmaceutically acceptable salt thereof, a pharmaceutically acceptable salt of a stereoisomer thereof, or an acceptable salt of a deuterated derivative thereof, wherein the compound is any one of the following formulas:

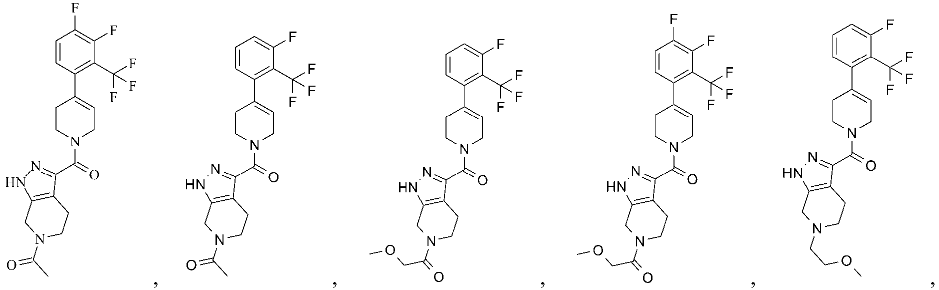

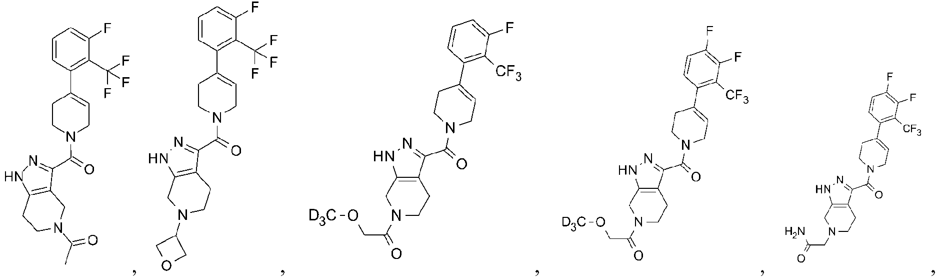

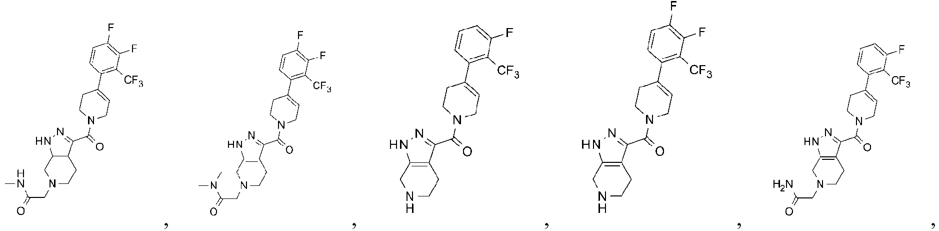

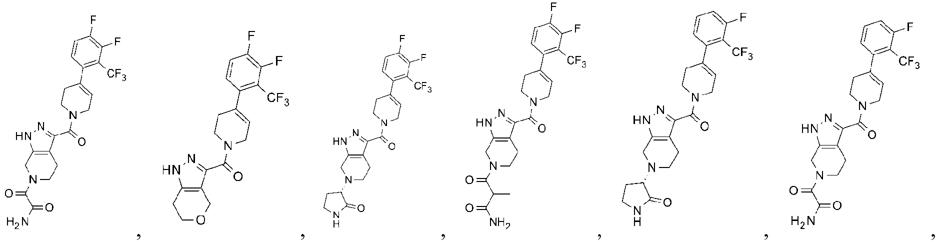

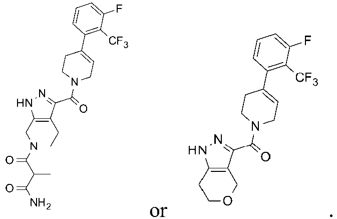

17. A pharmaceutical composition comprising the compound according to any one of claims 1 to 16, a stereoisomer thereof, a deuterated derivative thereof, a pharmaceutically acceptable salt thereof, a pharmaceutically acceptable salt of a stereoisomer thereof, or an acceptable salt of a deuterated derivative thereof; and at least one pharmaceutically acceptable excipient.

18. Use of the compound according to any one of claims 1 to 16, a stereoisomer thereof, a deuterated derivative thereof, a pharmaceutically acceptable salt thereof, a pharmaceutically acceptable salt of a stereoisomer thereof, or an acceptable salt of a deuterated derivative thereof; or the pharmaceutical composition according to claim 17 in the manufacture of a medicament for treating a disease associated with excessive accumulation of lipofuscin in the retina related to serum concentration of RBP4.

19. The use according to claim 18, wherein the disease associated with excessive accumulation of lipofuscin in the retina is di-retinoic acid-mediated macular degeneration, including age-related macular degeneration, dry age-related macular degeneration, Stargardt disease, Best disease, adult vitelliform macular dystrophy, or Stargardt-like macular dystrophy; the di-retinoic acid is A2E, isoA2E, A2-DHP-PE or atRALdi-PE.

20. A method of treating a subject suffering from a disease associated with excessive accumulation of lipofuscin in the retina related to serum concentration of RBP4, the method comprising administering to the subject a therapeutically effective amount of the compound according to any one of claims 1 to 16, a stereoisomer thereof, a deuterated derivative thereof, a pharmaceutically acceptable salt thereof, a pharmaceutically acceptable salt of a stereoisomer thereof, or an acceptable salt of a deuterated derivative thereof; or the pharmaceutical composition according to claim 17.

21. The method according to claim 20, wherein the disease associated with excessive accumulation of lipofuscin in the retina is di-retinoic acid-mediated macular degeneration, including age-related macular degeneration, dry age-related macular degeneration, Stargardt disease, Best disease, adult vitelliform macular dystrophy, or Stargardt-like macular dystrophy; the di-retinoic acid is A2E, isoA2E, A2-DHP-PE or atRALdi-PE.
